(19) Europäisches Patentamt / European Patent Office / Office européen des brevets

(11) **EP 4 628 597 A1**

(12) **EUROPEAN PATENT APPLICATION**
published in accordance with Art. 153(4) EPC

(43) Date of publication:
**08.10.2025 Bulletin 2025/41**

(21) Application number: **23898315.9**

(22) Date of filing: **29.11.2023**

(51) International Patent Classification (IPC):
*C12Q 1/6886* (2018.01)

(52) Cooperative Patent Classification (CPC):
**C12Q 1/6886**

(86) International application number:
**PCT/KR2023/019487**

(87) International publication number:
**WO 2024/117791 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2022 KR 20220162661**

(71) Applicant: **GC Genome Corporation**
**Yongin-si, Gyeonggi-do 16924 (KR)**

(72) Inventors:
• **CHO, Eun-Hae**
  **Yongin-si Gyeonggi-do 16924 (KR)**
• **KIM, Minjung**
  **Yongin-si Gyeonggi-do 16924 (KR)**
• **KIM, Wan**
  **Yongin-si Gyeonggi-do 16924 (KR)**

(74) Representative: **Mewburn Ellis LLP**
**Aurora Building**
**Counterslip**
**Bristol BS1 6BX (GB)**

(54) **DNA METHYLATION MARKERS FOR DIAGNOSING ESOPHAGEAL CANCER AND STOMACH CANCER AND USE THEREOF**

(57) The present invention relates to DNA methylation markers for diagnosing esophageal cancer and stomach cancer and use thereof, and more specifically to: **a** combination of DNA methylation markers, being capable of determining the presence or absence of esophageal cancer and stomach cancer; and use thereof. The DNA methylation markers for diagnosing esophageal cancer and stomach cancer, according to the present invention, are capable of diagnosing esophageal cancer and stomach cancer with high accuracy by using only DNA methylation information of blood samples, without using esophageal cancer and stomach cancer tissue samples, and thus may be usefully employed for early diagnosis of esophageal cancer and stomach cancer.

FIG. 1

EP 4 628 597 A1

FIG. 1

**Description**

[Technical Field]

The present invention relates to DNA methylation markers for diagnosing esophageal cancer and gastric cancer and uses thereof, and more specifically, to a combination of DNA methylation markers for determining whether or not esophageal cancer or gastric cancer is present and use thereof.

[Background Art]

Gastric cancer is the fourth most common cancer diagnosed worldwide and the third most common cause of cancer-related deaths. This gastric cancer is a representative type of cancer diagnosed in East Asian countries. In Korea, the incidence of gastric cancer is high due to heredity, a diet that includes salted foods, smoking, and a high prevalence of Helicobacter pylori. In Korea, most cases of early gastric cancer are asymptomatic (74.2-78.1%) compared to symptomatic cases (25.9-35.7%). Once gastric cancer progresses and causes serious symptoms and complications, the prognosis is poor and the survival rate decreases to less than 20% from about 65% (if detected early). Therefore, early diagnosis is very important.

Esophageal cancer is the eighth most common cancer, occurring in the upper part of the throat between the pharynx and the stomach, from esophageal cells. It is more common in men than in women, and the rate varies considerably across countries. According to the 2019 Korea Central Cancer Registry, 2,870 cases of esophageal cancer occurred in one year, with 2,573 cases (89%) being male. The two most common types of esophageal cancer are esophageal squamous cell carcinoma and esophageal adenocarcinoma. Several rarer subtypes are also known. Squamous cell carcinoma arises in the epithelial cells of the esophagus, while adenocarcinoma arises in glandular cells present in the lower part of the esophagus.

A key factor in the high mortality rate of esophageal cancer is the low diagnosis rate of early esophageal cancer. The cure rate of early esophageal cancer is much higher than that of mid- and late-stage cancer. However, there are no clear and specific symptoms and most subjects are already in the mid- and late-stage when diagnosed. Clinical studies have found that the cancer formation process takes several years on average from the lesion to the appearance of clinical symptoms in the subject, which provides an effective latency period for the detection of early esophageal cancer and the improvement of the early esophageal cancer diagnosis rate. When this latency period is fully utilized, it is expected that the therapeutic effect for esophageal cancer improves and the mortality rate of esophageal cancer decreases.

In order to accurately diagnose cancer, it is important not only to identify the mutant gene, but also to identify the mechanism by which the mutation occurs in the gene. Conventional studies focused on mutations in the coding sequence of the gene, such as point mutations, deletions, and insertions, and macroscopic chromosomal abnormalities. However, it has been recently reported that extragenic changes are just as important, and a representative example is methylation of promoter CpG islands.

In addition to A, C, G, and T, the genomic DNA of mammalian cells contains a fifth base, which is 5-methylcytosine (5-mC), a methyl group attached to the fifth carbon of the cytosine ring. 5-mC always appears only at the C of CG dinucleotides (5'-mCG-3'), and this CG is commonly expressed as CpG. C of CpG is methylated when a methyl group is attached thereto. This methylation of CpG suppresses the expression of repetitive sequences in the genome, such as alu or transposons, and such CpG is the site where extragenic changes most commonly occur in mammalian cells. The 5-mC of CpG is naturally deaminated and is converted to T, and as a result, CpG in the mammalian genome has only a frequency of 1%, which is much lower than the frequency that should normally appear (1/4 x 1/4 = 6.25%).

There are some CpGs that appear exceptionally densely and these are called "CpG islands". CpG islands are 0.2 to 3 kb in length, have a distribution percentage of C and G bases higher than 50%, and are highly concentrated with a distribution percentage of CpGs higher than 3.75%. Approximately 45,000 CpG islands appear in the entire human genome, and CpG islands are particularly concentrated in promoter regions that control gene expression. In fact, CpG islands appear in the promoters of important genes (housekeeping genes), which account for about half of all human genes (Cross, S. et al., Curr. Opin. Gene Develop., 5:309, 1995). Accordingly, attempts have been actively made recently to investigate promoter methylation of tumor-related genes in blood, sputum, saliva, feces, urine, and the like and use the investigate promoter methylation for various cancer treatments.

Currently, in clinical practice, cancer is diagnosed through history taking, physical examination, and clinical pathology tests. Once suspected, X-ray examination and endoscopic examination are performed and presence of cancer is finally confirmed through tissue examination. However, with the current clinical examination method, diagnosis is possible only when the number of cancer cells is 1 billion and the diameter of the cancer is 1 cm or more. In this case, cancer cells already have the ability to metastasize, and in fact, more than half of the cases have already metastasized. Meanwhile, tumor markers that search for substances produced directly or indirectly by cancer in the blood are used for cancer screening, but this has limitations in accuracy. That is, about half of the cases are normal although cancer is present, and they often show positive results when there is no cancer, which causes confusion. In addition, there is a problem that anticancer drugs mainly used for cancer treatment are effective only when the tumor volume is small.

Recently, methods for diagnosing cancer through DNA methylation measurement have been proposed. DNA methylation mainly occurs at cytosine in CpG islands in the promoter region of specific genes, which interferes with the binding of transcription factors and blocks the expression of specific genes (gene silencing). This is the main mechanism by which the function of a gene is lost in the absence of mutations in the coding sequence of the protein in the gene, and is interpreted as the cause of the loss of function of many tumor suppressor genes in human cancers. There is controversy over whether methylation of promoter CpG islands directly causes carcinogenesis or is a secondary change to carcinogenesis, but such abnormal methylation/demethylation in CpG islands has been reported in various cancer cells, including prostate cancer, colon cancer, uterine cancer, and breast cancer. Therefore, it may be utilized in various ways, such as early diagnosis of cancer, prediction of carcinogenesis risk, prediction of cancer prognosis, follow-up investigation after treatment, and prediction of response to chemotherapy. Recently, active attempts have made to use this for cancer diagnosis and screening by testing using methods such as methylation-specific PCR (hereinafter referred to as "MSP"), automated base analysis, or bisulfite pyrosequencing. However, most of these methods are limited to detecting and analyzing methylation of a small number of specific genes or promoter regions (e.g., Korean Patent No. 1557183, Korean Patent No. 1191947). These methods have limitations in the efficiency and accuracy of diagnosis.

Therefore, the present inventors have made great efforts to solve the problems and develop DNA methylation markers for diagnosing esophageal cancer and gastric cancer with high sensitivity and accuracy. As a result, the present inventors selected cancer-specific methylated regions using TCGA methylation data of esophageal cancer and gastric cancer tissue samples, known methylation data related to esophageal cancer and gastric cancer (GEO), and methylated DNA data from tissues and cfDNA of esophageal cancer or gastric cancer patients, and finally selected esophageal cancer and gastric cancer-specific DNA methylation markers in common regions in the data sets, and found that, when the DNA methylation markers are analyzed, esophageal cancer and gastric cancer can be diagnosed early with high accuracy. Based on this finding, the present invention has been completed.

[Disclosure]

Therefore, the present invention has been made in view of the above problems, and it is one object of the present invention to provide a DNA patterning marker combination for diagnosing esophageal cancer and gastric cancer.

It is another object of the present invention to provide a method of providing information for diagnosing esophageal cancer and gastric cancer using the DNA patterning marker combination.

It is another object of the present invention to provide a method of diagnosing esophageal cancer and gastric cancer using the DNA patterning marker combination.

It is another object of the present invention to provide a probe composition and a primer composition capable of detecting the DNA methylation marker combination, and a kit for diagnosing esophageal cancer and gastric cancer containing the composition.

In accordance with one aspect of the present invention, the above and other objects can be accomplished by the provision of a DNA methylation marker combination for diagnosing esophageal cancer and gastric cancer, including DNA methylation markers shown in Table 1.

In accordance with another aspect of the present invention, there is provided a method of providing information for diagnosing esophageal cancer and gastric cancer, including (a) isolating DNA from a biological sample, (b) detecting a methylation level of the DNA methylation marker combination, and (c) determining that esophageal cancer and gastric cancer develop when the detected DNA methylation marker level exceeds a cut-off value.

In accordance with another aspect of the present invention, there is provided a method of diagnosing esophageal cancer and gastric cancer, including (a) isolating DNA from a biological sample, (b) detecting a methylation level of the DNA methylation marker combination, and (c) determining that esophageal cancer and gastric cancer develop when the detected DNA methylation marker level exceeds a cut-off value.

In accordance with another aspect of the present invention, there is provided a primer composition for diagnosing esophageal cancer and gastric cancer, the primer composition capable of amplifying each of the DNA methylation markers of the DNA methylation marker combination.

In accordance with another aspect of the present invention, there is provided a probe composition for diagnosing esophageal cancer and gastric cancer, the probe composition specifically hybridizing with a polynucleotide including 10 or more consecutive bases containing the methylated bases of the DNA methylation marker of the DNA methylation marker combination or a complementary polynucleotide thereof.

In accordance with another aspect of the present invention, there is provided a kit for diagnosing esophageal cancer and gastric cancer containing the composition.

[Description of Drawings]

FIG. 1 is a flowchart illustrating a process of selecting DNA methylation markers for diagnosing esophageal cancer and stomach cancer according to the present invention.

FIG. 2 is a schematic diagram illustrating a split data set for selecting candidate markers according to one embodiment of the present invention.

FIG. 3 is a schematic diagram illustrating a leave one out (LOO) method to select candidate markers according to one embodiment of the present invention.

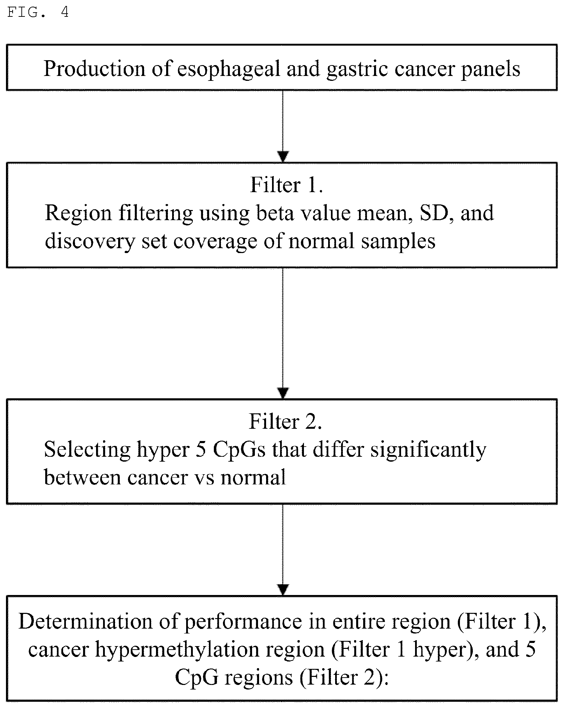

FIG. 4 is a flowchart showing a process of selecting a minimum combination of DNA methylation markers for diagnosing esophageal cancer and stomach cancer according to the present invention.

FIG. 5 is a graph showing the results of determining whether or not esophageal cancer is present in a clinical sample using a combination of 474 esophageal cancer- and stomach cancer-specific DNA methylation markers selected according to one embodiment of the present invention.

FIG. 6 is a graph showing the results of determining whether or not esophageal cancer is present in a clinical sample using a combination of 333 esophageal cancer- and stomach cancer-specific DNA methylation markers selected according to one embodiment of the present invention.

FIG. 7 is a graph showing the results of determining whether or not a clinical sample has esophageal cancer using a combination of 5 esophageal cancer- and stomach cancer-specific DNA methylation markers selected according to one embodiment of the present invention.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

In the present invention, a model capable of diagnosing esophageal cancer and gastric cancer using methylation information of cell-free nucleic acids in blood was developed and the accuracy thereof was verified.

In the present invention, DNA methylation markers capable of determining that esophageal cancer and gastric cancer develop were selected by combining methylation data of esophageal cancer and gastric cancer tissue samples shown in the TCGA database, methylation data of esophageal cancer and gastric cancer tissue samples shown in the known GEO database, and methylation data of cell-free nucleic acids extracted from blood samples of esophageal cancer patients.

That is, in one embodiment of the present invention, esophageal cancer- and gastric cancer-specific methylation regions were selected based on the methylation data of esophageal cancer and gastric cancer tissue samples and normal samples shown in the TCGA database, methylated DNA extracted from the blood of esophageal cancer patients and normal subjects was sequenced (cfMeDIP-seq, EM-seq), esophageal cancer- and gastric cancer tissue-specific methylation regions were selected by comparison, and esophageal cancer- or gastric cancer-specific methylation regions were further selected using known whole genome bisulfite sequencing (WGBS, GEO) data and TCGA data sets, respectively.

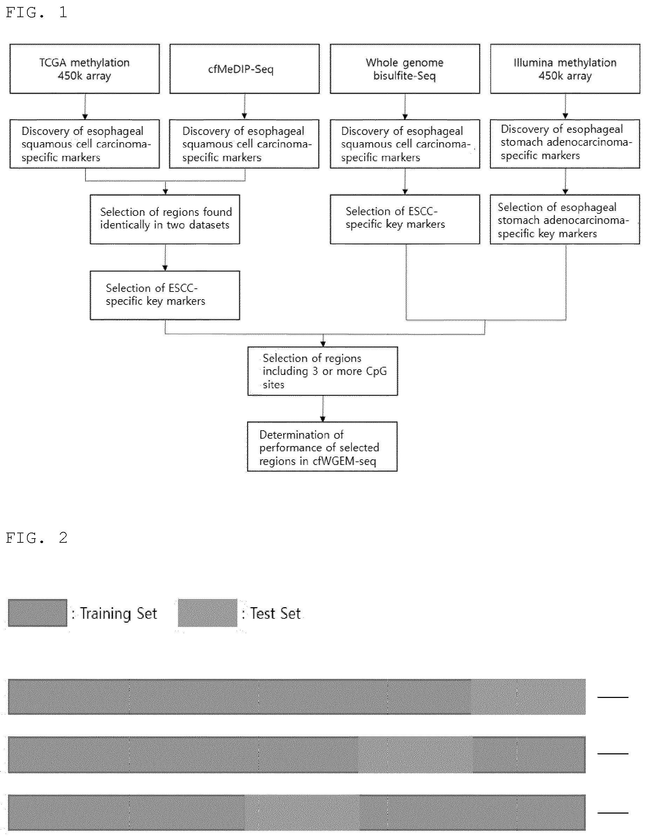

Then, it was found that, among the regions selected at the respective steps, overlapping regions were removed, regions with three or more CpG sites were integrated to select a final marker, and when whether or not esophageal cancer or gastric cancer was present was determined using the marker, it was possible to determine whether or not esophageal cancer or gastric cancer with high accuracy (FIG. 1, FIGs. 5 to 7).

In one aspect, the present invention is directed to a DNA methylation marker combination for diagnosing esophageal cancer and gastric cancer including DNA methylation markers shown in Table 1.

[Table 1]

| Chromosome | Start point | End point |
|---|---|---|
| chr2 | 200327093 | 200327094 |
| chr3 | 157812614 | 157812615 |

(continued)

| Chromosome | Start point | End point |
|---|---|---|
| chr3 | 157812620 | 157812621 |
| chr3 | 157812651 | 157812652 |
| chr14 | 38679763 | 38679764 |

In the present invention, the combination of DNA methylation markers for diagnosing esophageal cancer and gastric cancer may further include DNA markers shown in Table 2 below, but is not limited thereto.

[Table 2]

| Chromosome | Start point | End point | Chromosome | Start point | End point |
|---|---|---|---|---|---|
| chr1 | 1475525 | 1475892 | chr8 | 67090394 | 67090604 |
| chr1 | 32238510 | 32238810 | chr8 | 67344786 | 67345156 |
| chr1 | 33391630 | 33391930 | chr8 | 72470701 | 72471,000 |
| chr1 | 50893501 | 50893800 | chr8 | 72754803 | 72755202 |
| chr1 | 62660474 | 62660774 | chr8 | 72756005 | 72756491 |
| chr1 | 65991026 | 65991326 | chr8 | 80523263 | 80523563 |
| chr1 | 77332988 | 77333288 | chr8 | 99951601 | 99952500 |
| chr1 | 91192201 | 91192500 | chr8 | 132054237 | 132054537 |
| chr1 | 107683625 | 107683925 | chr8 | 143592225 | 143592525 |
| chr1 | 111217044 | 111217865 | chr8 | 145106432 | 145106732 |
| chr1 | 119528280 | 119528580 | chr9 | 841700 | 842000 |
| chr1 | 147790398 | 147790608 | chr9 | 16869582 | 16869882 |
| chr1 | 165323295 | 165323595 | chr9 | 96714448 | 96714748 |
| chr1 | 169396501 | 169397100 | chr9 | 112810252 | 112810552 |
| chr1 | 170630401 | 170630700 | chr9 | 126772285 | 126772585 |
| chr1 | 170634220 | 170634520 | chr9 | 126784789 | 126785089 |
| chr1 | 175568409 | 175568709 | chr10 | 7451672 | 7451972 |
| chr1 | 190447140 | 190447440 | chr10 | 44788662 | 44788962 |
| chr1 | 197890594 | 197890894 | chr10 | 48438574 | 48438874 |
| chr1 | 207843017 | 207843317 | chr10 | 63212357 | 63212657 |
| chr1 | 229569484 | 229569784 | chr10 | 81002068 | 81002630 |
| chr1 | 240255336 | 240255636 | chr10 | 90342588 | 90343049 |
| chr1 | 246952212 | 246952512 | chr10 | 94822758 | 94823058 |
| chr2 | 38301606 | 38301906 | chr10 | 118030820 | 118031120 |
| chr2 | 45155841 | 45156141 | chr10 | 118899257 | 118899557 |
| chr2 | 45160295 | 45160595 | chr10 | 129535743 | 129536043 |
| chr2 | 45172019 | 45172319 | chr10 | 131758181 | 131758481 |
| chr2 | 47797813 | 47798113 | chr10 | 131767376 | 131767676 |
| chr2 | 61371988 | 61372406 | chr10 | 133109717 | 133110017 |
| chr2 | 63285504 | 63286100 | chr10 | 133110094 | 133110394 |
| chr2 | 70995309 | 70995609 | chr11 | 2161742 | 2162333 |

(continued)

| Chromosome | Start point | End point | Chromosome | Start point | End point |
|---|---|---|---|---|---|
| chr2 | 91634481 | 91634781 | chr11 | 7272998 | 7273298 |
| chr2 | 105471810 | 105472110 | chr11 | 31826401 | 31826700 |
| chr2 | 119599501 | 119599800 | chr11 | 31826934 | 31827234 |
| chr2 | 119609442 | 119609742 | chr11 | 35105049 | 35105349 |
| chr2 | 119615841 | 119616141 | chr11 | 43602601 | 43602900 |
| chr2 | 127413681 | 127413981 | chr11 | 57250319 | 57250619 |
| chr2 | 145281792 | 145282092 | chr11 | 70508260 | 70508560 |
| chr2 | 158300325 | 158300635 | chr11 | 105481133 | 105481539 |
| chr2 | 162280501 | 162280800 | chr11 | 120434906 | 120435206 |
| chr2 | 172948817 | 172949117 | chr11 | 123301021 | 123301321 |
| chr2 | 172952136 | 172952436 | chr11 | 128564030 | 128564330 |
| chr2 | 172953242 | 172953542 | chr11 | 128564724 | 128565024 |
| chr2 | 176936286 | 176936586 | chr12 | 4918988 | 4919288 |
| chr2 | 176948093 | 176948393 | chr12 | 22487697 | 22487997 |
| chr2 | 176956556 | 176956991 | chr12 | 43944582 | 43944882 |
| chr2 | 176979454 | 176979754 | chr12 | 54345385 | 54345685 |
| chr2 | 176985539 | 176985839 | chr12 | 54359647 | 54359947 |
| chr2 | 176985923 | 176986223 | chr12 | 54370332 | 54370632 |
| chr2 | 176989208 | 176989508 | chr12 | 54371108 | 54371408 |
| chr2 | 176993401 | 176993700 | chr12 | 54385154 | 54385454 |
| chr2 | 177003960 | 177004260 | chr12 | 65217919 | 65218219 |
| chr2 | 177036438 | 177037106 | chr12 | 79258257 | 79258557 |
| chr2 | 177054423 | 177054723 | chr12 | 85306678 | 85306978 |
| chr2 | 198651001 | 198651497 | chr12 | 103218443 | 103218743 |
| chr2 | 208989098 | 208989398 | chr12 | 114878284 | 114878584 |
| chr2 | 229044901 | 229045200 | chr12 | 114882696 | 114882996 |
| chr3 | 2140549 | 2140849 | chr12 | 115102563 | 115102863 |
| chr3 | 42947540 | 42947840 | chr12 | 115103810 | 115104110 |
| chr3 | 119528806 | 119529106 | chr12 | 115109784 | 115110084 |
| chr3 | 137488469 | 137488769 | chr12 | 115134895 | 115135195 |
| chr3 | 138655677 | 138655977 | chr12 | 130388066 | 130388366 |
| chr3 | 142839841 | 142840141 | chr13 | 28498011 | 28498534 |
| chr3 | 147073801 | 147074100 | chr13 | 28498806 | 28499106 |
| chr3 | 147108362 | 147108662 | chr13 | 28501216 | 28501516 |
| chr3 | 147112080 | 147112200 | chr13 | 28503223 | 28503523 |
| chr3 | 147124023 | 147124567 | chr13 | 28674301 | 28674601 |
| chr3 | 147126068 | 147126278 | chr13 | 37005339 | 37005720 |
| chr3 | 147127429 | 147127729 | chr13 | 37005913 | 37006490 |
| chr3 | 147128306 | 147128606 | chr13 | 46425716 | 46426024 |

(continued)

| Chromosome | Start point | End point | Chromosome | Start point | End point |
|---|---|---|---|---|---|
| chr3 | 147140764 | 147141064 | chr13 | 58204074 | 58204374 |
| chr3 | 157813172 | 157813500 | chr13 | 78493155 | 78493455 |
| chr3 | 157821112 | 157821613 | chr13 | 79176422 | 79176722 |
| chr3 | 157821656 | 157821956 | chr13 | 84454161 | 84454461 |
| chr3 | 181441414 | 181441714 | chr13 | 95355001 | 95355300 |
| chr3 | 184301089 | 184301618 | chr13 | 100607973 | 100608273 |
| chr4 | 1399085 | 1399385 | chr13 | 100624789 | 100625089 |
| chr4 | 3371726 | 3372026 | chr13 | 100625702 | 100626002 |
| chr4 | 4859071 | 4859281 | chr13 | 100640227 | 100640527 |
| chr4 | 5710253 | 5710553 | chr13 | 102068846 | 102069146 |
| chr4 | 13525572 | 13525872 | chr13 | 112720071 | 112720382 |
| chr4 | 13530901 | 13531200 | chr14 | 29229689 | 29229989 |
| chr4 | 81185078 | 81185378 | chr14 | 29236263 | 29236473 |
| chr4 | 111533196 | 111533650 | chr14 | 36982295 | 36982595 |
| chr4 | 111535378 | 111535678 | chr14 | 37128275 | 37128575 |
| chr4 | 111539311 | 111539611 | chr14 | 37129201 | 37129500 |
| chr4 | 111554497 | 111554797 | chr14 | 38067055 | 38067445 |
| chr4 | 111560778 | 111561078 | chr14 | 51561143 | 51561443 |
| chr4 | 111561787 | 111562112 | chr14 | 52735271 | 52735571 |
| chr4 | 111562363 | 111562663 | chr14 | 57276918 | 57277218 |
| chr4 | 174449490 | 174449790 | chr14 | 60951901 | 60952500 |
| chr4 | 174452311 | 174452611 | chr14 | 60973367 | 60973667 |
| chr5 | 1883804 | 1884104 | chr14 | 60976201 | 60976500 |
| chr5 | 5139851 | 5140151 | chr14 | 61109705 | 61110106 |
| chr5 | 42424375 | 42424675 | chr15 | 28351948 | 28352248 |
| chr5 | 42995219 | 42995677 | chr15 | 41793731 | 41794031 |
| chr5 | 87955633 | 87955933 | chr15 | 45409169 | 45409469 |
| chr5 | 118609317 | 118609617 | chr15 | 60287437 | 60287737 |
| chr5 | 122430284 | 122430584 | chr15 | 65689002 | 65689302 |
| chr5 | 136834314 | 136834642 | chr15 | 86233064 | 86233370 |
| chr5 | 140793318 | 140793618 | chr15 | 89922301 | 89922600 |
| chr5 | 140864757 | 140865057 | chr15 | 96887311 | 96887710 |
| chr5 | 153853518 | 153853818 | chr15 | 96887769 | 96888069 |
| chr5 | 153862521 | 153862821 | chr15 | 101514062 | 101514362 |
| chr5 | 178003941 | 178004354 | chr16 | 9107049 | 9107349 |
| chr5 | 178367677 | 178367977 | chr16 | 28074234 | 28074526 |
| chr5 | 178421978 | 178422278 | chr16 | 56696938 | 56697238 |
| chr6 | 391593 | 392086 | chr16 | 67196767 | 67197336 |
| chr6 | 393089 | 393389 | chr16 | 77822269 | 77822569 |

(continued)

| Chromosome | Start point | End point | Chromosome | Start point | End point |
|---|---|---|---|---|---|
| chr6 | 1385345 | 1385645 | chr16 | 86541330 | 86541630 |
| chr6 | 1393425 | 1393725 | chr17 | 1880932 | 1881232 |
| chr6 | 10393848 | 10394148 | chr17 | 12877171 | 12877696 |
| chr6 | 26614501 | 26614800 | chr17 | 36734761 | 36735061 |
| chr6 | 27258856 | 27259156 | chr17 | 43339347 | 43339647 |
| chr6 | 27526069 | 27526369 | chr17 | 46799870 | 46800170 |
| chr6 | 28414801 | 28415100 | chr17 | 46919030 | 46919330 |
| chr6 | 58142486 | 58142786 | chr17 | 59534848 | 59535148 |
| chr6 | 58147218 | 58147750 | chr18 | 500829 | 501129 |
| chr6 | 58148830 | 58149133 | chr18 | 4455187 | 4455487 |
| chr6 | 73330964 | 73331264 | chr18 | 5543398 | 5543698 |
| chr6 | 88876591 | 88876891 | chr18 | 11149285 | 11149620 |
| chr6 | 100901902 | 100902202 | chr18 | 14748135 | 14748435 |
| chr6 | 100911594 | 100911894 | chr18 | 19756552 | 19756852 |
| chr6 | 100915705 | 100916005 | chr18 | 32847416 | 32847716 |
| chr6 | 110679416 | 110679716 | chr18 | 55095027 | 55095327 |
| chr6 | 133562342 | 133562642 | chr18 | 74290778 | 74291078 |
| chr6 | 1611,00092 | 161100392 | chr18 | 74962644 | 74962944 |
| chr6 | 166218369 | 166218669 | chr18 | 76739076 | 76739376 |
| chr7 | 15727291 | 15727591 | chr18 | 76739409 | 76739709 |
| chr7 | 19146001 | 19146300 | chr19 | 9473524 | 9473834 |
| chr7 | 19157043 | 19157413 | chr19 | 13124934 | 13125234 |
| chr7 | 24323790 | 24324089 | chr19 | 15580550 | 15580850 |
| chr7 | 24324301 | 24324600 | chr19 | 17439058 | 17439358 |
| chr7 | 24324826 | 24325126 | chr19 | 17439727 | 17440027 |
| chr7 | 27225169 | 27225469 | chr19 | 21646179 | 21646479 |
| chr7 | 27284490 | 27284923 | chr19 | 22018808 | 22019108 |
| chr7 | 35298241 | 35298541 | chr19 | 22444443 | 22444743 |
| chr7 | 42267948 | 42268259 | chr19 | 22817221 | 22817521 |
| chr7 | 49812883 | 49813261 | chr19 | 31841405 | 31841705 |
| chr7 | 84814533 | 84814833 | chr19 | 31842723 | 31843023 |
| chr7 | 96642301 | 96642600 | chr19 | 36909263 | 36909568 |
| chr7 | 96646967 | 96647438 | chr19 | 37096173 | 37096479 |
| chr7 | 96655294 | 96655594 | chr19 | 37957845 | 37958147 |
| chr7 | 114562697 | 114562997 | chr19 | 37958196 | 37958496 |
| chr7 | 121946273 | 121946573 | chr19 | 48983792 | 48984221 |
| chr7 | 127672308 | 127672714 | chr19 | 51111527 | 51111827 |
| chr7 | 127841686 | 127841986 | chr19 | 53038822 | 53039122 |
| chr7 | 142494835 | 142495135 | chr19 | 56879421 | 56879795 |

(continued)

| Chromosome | Start point | End point | Chromosome | Start point | End point |
|---|---|---|---|---|---|
| chr7 | 149389779 | 149389989 | chr19 | 56904751 | 56905182 |
| chr7 | 149917113 | 149917413 | chr19 | 57018919 | 57019219 |
| chr7 | 151107107 | 151107435 | chr19 | 57049545 | 57049927 |
| chr7 | 158937819 | 158938201 | chr19 | 57050470 | 57050770 |
| chr8 | 495586 | 495967 | chr19 | 57078615 | 57078915 |
| chr8 | 688267 | 688567 | chr19 | 58220101 | 58220700 |
| chr8 | 23562547 | 23562847 | chr19 | 58570269 | 58570569 |
| chr8 | 23562974 | 23563274 | chr20 | 21694558 | 21694858 |
| chr8 | 38964864 | 38965402 | chr20 | 25062104 | 25062597 |
| chr8 | 41166957 | 41167257 | chr20 | 34894498 | 34894798 |
| chr8 | 41753910 | 41754210 | chr20 | 37434079 | 37434379 |
| chr8 | 53851557 | 53851857 | chr20 | 41818206 | 41818508 |
| chr8 | 53851880 | 53852811 | chr20 | 61808738 | 61809038 |
| chr8 | 57069863 | 57070163 | chr21 | 34442210 | 34442527 |
| chr8 | 59058506 | 59058806 | chr21 | 38075449 | 38075749 |
| chr8 | 65281346 | 65281646 | chr21 | 38079541 | 38079841 |
| chr8 | 65294493 | 65294793 | chr21 | 45336523 | 45336823 |

In the present invention, the DNA methylation marker combination for diagnosing esophageal cancer and gastric cancer may further include two or more DNA methylation markers selected from the group consisting of DNA markers shown in Table 3 below, but is not limited thereto.

[Table 3]

| Chromosome | Start point | End point | Chromosome | Start point | End point |
|---|---|---|---|---|---|
| chr1 | 2425710 | 2426010 | chr8 | 144347047 | 144347257 |
| chr1 | 3139807 | 3140017 | chr9 | 79625119 | 79625419 |
| chr1 | 3210267 | 3210567 | chr9 | 137097130 | 137097430 |
| chr1 | 5661736 | 5662036 | chr9 | 139634101 | 139634400 |
| chr1 | 19599366 | 19599666 | chr10 | 696171 | 696482 |
| chr1 | 21877426 | 21877726 | chr10 | 778559 | 778859 |
| chr1 | 157401687 | 157401987 | chr10 | 3329816 | 3330116 |
| chr1 | 162319425 | 162319725 | chr10 | 31388486 | 31388786 |
| chr1 | 199326770 | 199327070 | chr10 | 44285701 | 44286000 |
| chr1 | 248366249 | 248366549 | chr10 | 77021717 | 77022017 |
| chr2 | 1879801 | 1880100 | chr10 | 95256767 | 95256977 |
| chr2 | 30496175 | 30496475 | chr10 | 130185301 | 130185600 |
| chr2 | 100327219 | 100327519 | chr10 | 134587890 | 134588190 |
| chr2 | 169658731 | 169659031 | chr10 | 134732701 | 134733000 |
| chr2 | 189064407 | 189064707 | chr10 | 134734501 | 134734800 |
| chr2 | 201612794 | 201613094 | chr10 | 134834701 | 134835000 |
| chr2 | 238188557 | 238188857 | chr10 | 135030404 | 135030704 |

(continued)

| Chromosome | Start point | End point | Chromosome | Start point | End point |
|---|---|---|---|---|---|
| chr2 | 238578341 | 238578641 | chr11 | 655429 | 655729 |
| chr2 | 238972874 | 238973174 | chr11 | 1422301 | 1422600 |
| chr2 | 240169011 | 240169311 | chr11 | 5009161 | 5009461 |
| chr3 | 11178443 | 11178948 | chr11 | 12188846 | 12189146 |
| chr3 | 19987378 | 19987965 | chr11 | 17562975 | 17563275 |
| chr3 | 61623149 | 61623449 | chr11 | 64658576 | 64658876 |
| chr3 | 159386842 | 159387142 | chr11 | 69218483 | 69218783 |
| chr3 | 160409874 | 160410095 | chr11 | 111154676 | 111154976 |
| chr3 | 169374960 | 169375260 | chr12 | 53319067 | 53319367 |
| chr3 | 180003041 | 180003341 | chr13 | 113426100 | 113426420 |
| chr3 | 180076871 | 180077171 | chr13 | 113648732 | 113649032 |
| chr3 | 180366205 | 180366505 | chr13 | 114064858 | 114065158 |
| chr3 | 180977784 | 180978084 | chr13 | 114065701 | 114066000 |
| chr3 | 193840031 | 193840331 | chr13 | 114769924 | 114770224 |
| chr3 | 197247601 | 197247901 | chr13 | 114917377 | 114917790 |
| chr4 | 7483501 | 7483800 | chr14 | 58599632 | 58599842 |
| chr4 | 54374099 | 54374399 | chr14 | 65405781 | 65406081 |
| chr5 | 15521632 | 15521932 | chr14 | 104600401 | 104600700 |
| chr6 | 44698584 | 44698884 | chr14 | 105195666 | 105195966 |
| chr6 | 166259862 | 166260162 | chr15 | 22798836 | 22799136 |
| chr7 | 544375 | 544675 | chr15 | 90456774 | 90457074 |
| chr7 | 1303201 | 1303501 | chr16 | 552604 | 552904 |
| chr7 | 3221078 | 3221523 | chr16 | 7640302 | 7640512 |
| chr7 | 3234547 | 3234847 | chr16 | 21245001 | 21245301 |
| chr7 | 23749383 | 23749683 | chr16 | 30456175 | 30456475 |
| chr7 | 27154502 | 27154712 | chr16 | 33572254 | 33572563 |
| chr7 | 38726444 | 38726744 | chr16 | 73266529 | 73266829 |
| chr7 | 40566371 | 40566671 | chr16 | 86653065 | 86653365 |
| chr7 | 41026047 | 41026347 | chr16 | 87823801 | 87824100 |
| chr7 | 55145577 | 55145877 | chr17 | 30488733 | 30489033 |
| chr7 | 73247463 | 73247763 | chr17 | 34328442 | 34328742 |
| chr7 | 73392547 | 73392847 | chr17 | 39059248 | 39059548 |
| chr7 | 97568604 | 97568814 | chr17 | 74911370 | 74911670 |
| chr7 | 99136949 | 99137159 | chr17 | 77174101 | 77174400 |
| chr7 | 140227123 | 140227423 | chr19 | 2278468 | 2278768 |
| chr7 | 157467901 | 157468200 | chr19 | 42408308 | 42408608 |
| chr7 | 157568101 | 157568400 | chr19 | 51128490 | 51128790 |
| chr8 | 2024099 | 2024399 | chr20 | 21588943 | 21589243 |
| chr8 | 11839694 | 11839994 | chr20 | 29960832 | 29961132 |

(continued)

| Chromosome | Start point | End point | Chromosome | Start point | End point |
|---|---|---|---|---|---|
| chr8 | 13294962 | 13295262 | chr20 | 31591997 | 31592297 |
| chr8 | 23083321 | 23083680 | chr20 | 43029842 | 43030144 |
| chr8 | 37217918 | 37218218 | chr20 | 44876196 | 44876496 |
| chr8 | 53517050 | 53517350 | chr20 | 44881299 | 44881599 |
| chr8 | 64588078 | 64588378 | chr20 | 56134498 | 56134798 |
| chr8 | 105988133 | 105988433 | chr20 | 56272712 | 56273012 |
| chr8 | 110984688 | 110984988 | chr20 | 56273109 | 56273409 |
| chr8 | 117586928 | 117587228 | chr20 | 57616041 | 57616341 |
| chr8 | 118628850 | 118629150 | chr20 | 59047338 | 59047638 |
| chr8 | 120686835 | 120687135 | chr20 | 59832774 | 59833074 |
| chr8 | 124729780 | 124730080 | chr20 | 60445848 | 60446058 |
| chr8 | 124730147 | 124730447 | chr20 | 60470101 | 60470400 |
| chr8 | 139344592 | 139344892 | chr20 | 62048701 | 62049000 |
| chr8 | 140630030 | 140630330 | chr20 | 62121768 | 62122068 |
| chr8 | 144346612 | 144346912 |  |  |  |

As used herein, the term "DNA methylation" means a state in which a methyl group is covalently bonded to the position C5 of a cytosine base in genomic DNA. The methylation level means, for example, the quantity of methylation present in a DNA base sequence within all genomic regions and some non-genomic regions, and in the present invention, means the degree of methylation of the DNA methylation marker. In the DNA methylation marker, methylation may occur over the entire sequence or part of the sequence.

In the present invention, the esophageal cancer means a malignant tumor that occurs in the esophagus, and more specifically, the esophageal cancer means cervical esophageal cancer, thoracic esophageal cancer, gastroesophageal junction cancer, and metastatic esophageal cancer that occurs in other organs and metastasizes to the esophagus, but is not limited thereto.

In the present invention, the gastric cancer means a malignant tumor occurring in the stomach, and more specifically, the gastric cancer means gastric adenocarcinoma, lymphoma, gastric submucosal tumor, leiomyoma, and metastatic gastric cancer that occurs in other organs and metastasized to the stomach, but is not limited thereto.

In another aspect, the present invention is directed to a method of providing information for diagnosing esophageal cancer and gastric cancer, including:

(a) isolating DNA from a biological sample;

(b) detecting a methylation level of the DNA methylation marker combination; and

(c) determining that esophageal cancer and gastric cancer develop when the detected DNA methylation marker level exceeds a cut-off value.

In another aspect, the present invention is directed to a method of diagnosing esophageal cancer and gastric cancer, including:

(a) isolating DNA from a biological sample;

(b) detecting a methylation level of the DNA methylation marker combination; and

(c) determining that esophageal cancer and gastric cancer develop when the detected DNA methylation marker level exceeds a cut-off value.

In the present invention, the DNA may be used without limitation as long as it is DNA extracted from a biological sample, but is preferably a fragment of cell-free nucleic acid or intracellular nucleic acid, but is not limited thereto.

In the present invention, the biological sample refers to any substance, biological fluid, tissue or cell obtained from or derived from a subject, and examples thereof include, but are not limited to, whole blood, leukocytes, peripheral blood mononuclear peripheral cells, leukocyte buffy coat, blood including plasma and serum, sputum, tears, mucus, nasal washes, nasal aspirates, breath, urine, semen, saliva, peritoneal washings, pelvic fluids, cyst fluids, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, semen, hair, saliva, urine, oral cells, placenta cells, cerebrospinal fluid, and mixtures thereof.

In the present invention, the detection of the methylation level in step (b) may be performed by various known methods, and preferably, by bisulfite conversion or methylated DNA immunoprecipitation (MeDIP), but is not limited thereto.

In the present invention, another method for detecting DNA methylation is restriction enzyme-based detection, which is a method including cutting unmethylated nucleic acids using a methylation restriction enzyme (MRE), or cutting a specific sequence (recognition site) regardless of methylation and combining the sequence with hybridization or PCR for analysis.

In the present invention, methods based on bisulfite substitution include whole-genome bisulfite sequencing (WGBS), reduced-representation bisulfite sequencing (RRBS), methylated CpG tandems amplification and sequencing (MCTA-seq), targeted bisulfite sequencing, methylation array, and methylation-specific PCR (MSP).

In the present invention, methods for enriching and analyzing methylated DNA include methylated DNA immunoprecipitation sequencing (MeDIP-seq), methyl-CpG binding domain protein capture sequencing (MBD-seq), and the like.

Another method for analyzing methylated DNA in the present invention is 5-hydroxymethylation profiling and examples thereof include 5hmC-Seal (hMe-Seal), hmC-CATCH, hydroxymethylated DNA immunoprecipitation sequencing (hMeDIP-seq), and oxidative bisulfite conversion.

In the present invention, the detection of the methylation level in step (b) may be performed using any one method selected from the group consisting of PCR, methylation specific PCR, real time methylation specific PCR, PCR using methylated DNA-specific binding protein, quantitative PCR, PCR using methylation-specific PNA, melting curve analysis, DNA chip, pyrosequencing, bisulfite sequencing, and methylation next-generation base sequence sequencing, but is not limited thereto.

In the present invention, the next-generation sequencer may be used for sequencing known in the art. Sequencing of the nucleic acid separated by the selection method is typically performed using next-generation sequencing (NGS). Next generation sequencing includes any sequencing method that determines the nucleotide sequence of each nucleic acid molecule or of a clonally expanded proxy for each nucleic acid molecule in a highly similar manner (e.g., more than 105 molecules are sequenced simultaneously). In one embodiment, the relative abundance of a nucleic acid species in a library may be estimated by counting the relative occurrences of the cognate sequences thereof in data generated by a sequencing experiment. Next generation sequencing methods are known in the art and are described, for example, in Metzker, M. (2010) Nature Biotechnology Reviews 11:31-46, which is incorporated herein by reference.

In one embodiment, next-generation sequencing is used to determine the nucleotide sequence of respective nucleic acid molecules (e.g., the HeliScope Gene Sequencing system from Helicos BioSciences and the PacBio RS system from Pacific BioSciences). In another embodiment, massively parallel short-read sequencing (e.g., the Solexa sequencer, Illumina Inc., San Diego, Calif.), which produces more bases of sequence per unit of sequencing than sequencing, for example, other sequencing methods that produce fewer but longer reads, determines the nucleotide sequence of clonally expanded proxies for individual nucleic acid molecules (e.g., the Solexa sequencer, Illumina Inc., San Diego, Calif.; 454 Life Sciences, Branford, Conn.; and Ion Torrent). Other methods or machines for next generation sequencing include, but are not limited to, 454 Life Sciences (Branford, CT), Applied Biosystems (Foster City, CA; SOLiD sequencer), Helicos Biosciences Corporation (Cambridge, MA), and emulsion and microfluidic sequencing techniques nanodroplets (e.g., GnuBio drops).

Platforms for next-generation sequencing include, but are not limited to, the genome sequencer (GS) FLX system from Roche/454, genome analyzer (GA) from Illumina/Solexa, support oligonucleotide ligation detection (SOLiD) system from Life/APG, G.007 system from Polonator, HeliScope gene sequencing system from Helicos BioSciences, PromethION, GriION, and MinION systems from Oxford Nanopore Technologies, and PacBio RS system from Pacific Biosciences.

In the present invention, the methylation level of step (b) may be expressed as an alpha value or a beta value, but is not limited thereto.

In the present invention, the alpha value may be defined as a methylation ratio of a CpG site present in a nucleic acid fragment including a lead. For example, when there are 10 CpG sites present in a nucleic acid fragment, and 9 of these sites are methylated, the alpha value of the nucleic acid fragment is 90%.

In the present invention, step (c) may be performed by a method including the following steps:

(c-i) calculating a methylation score using the following Equation 1; and

Equation 1:

Methylation score =

$$\frac{\text{Number of nucleic acid fragments with alpha value of hypermethylation marker of 80\% or more } + \text{Number of nucleic acid fragments with alpha value of hypomethylation marker of 20\% or less}}{\text{Total number of nucleic acid fragments}}$$

(c-i) determining that esophageal cancer and stomach cancer develop when the calculated methylation score exceeds the cut-off value.

In the present invention, the expression "alpha value of the hypermethylation marker is 80% or more" means that the nucleic acid fragment includes a read aligned to a region including the hypermethylation marker, and the methylation marker ratio of the CpG site it contains is 80% or more, and the expression "alpha value of the hypomethylation marker is 20% or less" means that the nucleic acid fragment includes a read aligned to a region including the hypomethylation marker, and the methylation marker ratio of the CpG site it contains is 20% or less.

In the present invention, the cut-off value of step (c) may be used without limitation as long as it is a value that can determine whether or not esophageal cancer or stomach cancer is present, and preferably, the cut-off value of step (c) ranges between 0.001 and 0.1, more preferably between 0.003 and 0.08, and most preferably between 0.005 and 0.05, but is not limited thereto.

In another aspect,
the present invention is directed to a primer composition for diagnosing esophageal cancer and gastric cancer containing a primer combination capable of amplifying each DNA methylation marker of the DNA methylation marker combination.

In the present invention, the appropriate length of the primer may vary depending on the intended use, but may generally include 15 to 30 bases. The primer sequence does not need to be completely complementary to the template, but should be sufficiently complementary to hybridize with the template. The primer may hybridize to a DNA sequence including a methylation marker to amplify a DNA fragment including the methylation marker. The primer of the present invention may be used in a diagnostic kit or prediction method for determining whether or not esophageal cancer or gastric cancer develops by detecting the level of DNA methylation.

In the present invention, the primer capable of amplifying the DNA methylation marker may be used without limitation as long as it is a base sequence of the same chromosome that does not directly include the marker region, and specifically, the primer may be 1 to 1,000 bp 5' upstream of the marker region and 1 to 1,000 bp 3' downstream of the marker region, and more specifically, 1 to 200 bp 5' upstream of the marker region and 1 to 200 bp 3' downstream of the marker region, but is not limited thereto.

In another aspect,
the present invention is directed to a probe composition for diagnosing esophageal cancer and gastric cancer containing a probe combination capable of specifically hybridizing with a polynucleotide including 10 or more consecutive bases containing methylated bases of the DNA methylohar marker of the DNA methylation marker combination or a complementary polynucleotide thereof.

In the present invention, the probe may be methylation-specific, which means that the probe hybridizes specifically only to methylated nucleic acids in the methylation marker region. Here, hybridization is usually performed under strict conditions, for example, a salt concentration of 1 M or less and a temperature of 25°C or higher. For example, conditions of 5XSSPE (750 mM NaCl, 50 mM Na Phosphate, 5 mM EDTA, pH 7.4) and 25 to 30°C may be suitable for methylation-specific probe hybridization.

In the present invention, the probe refers to a hybridization probe and means an oligonucleotide capable of binding sequence-specifically to a complementary strand of a nucleic acid. The methylation-specific probe of the present invention may hybridize to a DNA fragment derived from one subject but not hybridize to a fragment derived from another subject, because methylation exists among nucleic acid fragments derived from two subjects of the same species. In this case, the hybridization conditions have significant difference in hybridization intensity and thus should be sufficiently stringent to hybridize depending on whether or not methylation is present. It is preferable that the central portion of the probe of the present invention is aligned to the region of the methylation marker. The probe of the present invention may be used in a diagnostic kit or prediction method for detecting esophageal cancer and gastric cancer by detecting the level of DNA methylation.

In another aspect,
the present invention is directed to a kit for diagnosing esophageal cancer and gastric cancer containing any one of the compositions described above.

In the present invention, the kit may include not only the polynucleotide of the present invention, but also one or more other component compositions, solutions or devices suitable for the analysis method. In one embodiment, the kit of the present invention may be a kit including essential elements required for performing PCR, and may further include a test tube or other appropriate container, a reaction buffer (with various pH and magnesium concentrations), deoxynucleotides (dNTPs), enzymes such as Taq polymerase and reverse transcriptase, DNase, RNAse inhibitors, DEPC-water, and sterile water. In another embodiment, the kit of the present invention may be a kit for predicting a blood statin concentration including essential elements required for performing a DNA chip, and the DNA chip kit may include a substrate to which a specific polynucleotide, primer or probe for the methylation is attached, and the substrate may contain a nucleic acid corresponding to a quantitative control gene or a fragment thereof.

### Example

Hereinafter, the present invention will be described in more detail with reference to examples. However, it will be obvious to those skilled in the art that these examples are provided only for illustration of the present invention, and should not be construed as limiting the scope of the present invention.

Example 1. Selection of esophageal squamous cell carcinoma (ESCC)-specific methylation regions from TCGA methylation 450K array data

The degree of methylation was determined using the infinium human methylation 450K beadchip array data (UCSC Xena, http:/xena.ucsc.edu) from the cancer genome atlas (TCGA). DNA extracted from tissues may be converted through bisulfite treatment and DNA methylation may be determined through modification of cytosine bases. The degree of methylation may be determined for each region, and differentially methylated regions between ESCC tissues and surrounding normal tissues were selected using the beta value, which represents the degree of methylation.

TCGA methylation 450k array data was divided into Train and Test groups as shown in Table 4, marker selection was performed using the Train group, and it was determined whether or not the discovered markers exhibited the same pattern in the Test set group.

[Table 4]

|  | Solid Tissue Normal | Primary Solid Tumor | Blood | Total |
|---|---|---|---|---|
| **Train** | 16 | 129 | 459 | 604 |
| **Test** | - | 56 | 197 | 253 |
| **Total** | 16 | 185 | 656 | 857 |

First, missing values were excluded from approximately 480K regions, and then regions having FDR values less than 0.01 and absolute delta beta greater than 0.25, were selected using Limma (linear models for microarray data) software, and 1,020 hypomethylated regions and 3,137 hypermethylated regions specific to ESCC were selected, excluding sex chromosomes, to select ESCC-specific methylation regions.

Example 2. Extraction of methylated cfDNA from blood and next-generation base sequence analysis (cfMeDIP-Seq)

Blood was collected from 68 patients with esophageal cancer and 283 normal subjects (Table 3), only the plasma portion was primarily centrifuged under the conditions of 3,000 rpm and 25°C for 10 minutes, and then the plasma separated from the first centrifugation was secondarily centrifuged under the conditions of 16,000 g and 25°C for 10 minutes to isolate the plasma supernatant excluding the precipitate. Cell-free DNA was extracted from the separated plasma using the Chemagen DNA kit, the adaptor ligation process was performed using the Truseq Nano DNA HT library prep kit (Illumina) and 5 mC immunoprecipitation was performed using the antibody of the cfMeDIP kit (diagnode) at 10 rpm and 4°C for 17 hours. After purification, PCR enrichment was performed again using the Truseq Nano DNA HT library prep kit (Illumina) to produce a final library. The produced library was sequenced using Novaseq 6000 (Illumina) in 150 paired-end mode and about 30 million reads were produced from each sample.

[Table 5]

|  | Count |
| --- | --- |
| **Esophageal cancer** | 68 |
| **Normal** | 283 |
| **Total** | 351 |

Example 3. Selection of ESCC-specific methylation regions through cfMeDIP-Seq data analysis

Since methylated cell-free nucleic acids were sequenced in Example 2, the obtained nucleic acid fragment data were methylated. By aligning the nucleic acid fragment data to the human reference genome, methylated regions in the entire human genome region can be identified. MeDIP-Seq data represents methylated regions and differentially methylated regions between the esophageal cancer and gastric cancer groups and the normal group were selected using the normalized value per 300 bp bin.

cfMeDIP-Seq data was classified into train and test groups as shown in Table 6 below, data set splitting was performed five times (FIG. 2), marker selection was performed using the train group, and then markers found in two or more data sets were used.

That is, test set splitting into different combinations was repeated five times to produce a dataset of five different combinations and identify DMRs (FIG. 2). Additional marker selection was performed later using markers found in two or more data sets among the corresponding DMRs.

[Table 6]

|  | Esophageal cancer | Normal | Total |
| --- | --- | --- | --- |
| **Train** | 54 | 226 | 280 |
| **Test** | 14 | 57 | 71 |
| **Total** | 68 | 283 | 351 |

First, adapter trimming and quality trimming were performed on the fastq file using Trim Galore (version 0.6.6), the nucleic acid fragment data were aligned to the reference genome (hg19) using the bwa (version 0.7.17-r1188) alignment tool, PCR duplicate nucleic acid fragments were removed using the samtools rmdup (version 1.11) tool, and nucleic acid fragments with a mapping quality of less than 10 were removed using the samtools view (version 1.11) tool. Then, only chr1 to 22, X and Y were left, the sex chromosomes were excluded, 300 bp bins were subjected to binning without overlapping, and the read count per 300 bp bin was obtained.

Blacklist regions (Low_mappability_island, centromeric_repeat, etc.) and bins with a total read count of 10 or less in the entire sample per bin were excluded.

Normalized values (TMM normalized values) were generated per 300 bp bin using edgeR (empirical analysis of digital gene expression data in R) software.

Finally, 544,553 hypermethylated regions and 443,967 hypomethylated regions specific to ESCC having FDR values less than 0.05 were selected using edgeR software.

Then, 1,497 CpG sites that were found both in the regions selected in Example 1 and in the regions selected in cfMeDIP-seq and overlapped in at least two of the five cfMeDIP data sets were finally selected as ESCC-specific markers. When regions were defined in 300 bp bin units, a total of 1,133 differentially methylated regions (DMRs) were selected.

For selection of main markers, an additional selection process was conducted using the AUC and median difference of absolute delta beta value between groups that divide tumor and normal samples, and the hypermethylated or hypomethylated regions in normal samples. Regions satisfying the criteria of an AUC of 0.9 or higher, a median beta value difference between tumor and normal samples of 0.2 or higher, and a median beta value in normal samples of 0.2 or lower or 0.8 or higher were selected. Based on the criteria, 28 hypomethylated regions and 145 hypermethylated regions were selected.

For regions overlapping with previously known methylation regions (WO 2019/195268 A2, WO 2019/199696 WO 2019/195268 A2, WO 2019/199696A1), only significant hypermethylation regions were added. The significance was based on the AUC and the group median difference of the absolute delta beta values used to divide tumor and normal samples, and the criterion for the AUC was 0.9 or higher and the criterion for the median beta value difference between tumor and normal samples was 0.3 or higher. When only major regions among the known regions were added using the criteria, 19 hypomethylation regions and 29 hypermethylation regions were selected (regions remaining after excluding the known regions: 12 hypermethylation regions, 19 hypomethylation regions, number of major regions among the known regions: 17 hypermethylation regions).

Finally, only the regions with three or more CpG sites in the corresponding DMR were left, and 18 hypomethylated regions and 29 hypermethylated regions were selected. In terms of CpG site units, there were 21 hypomethylated regions and 53 hypermethylated regions were selected.

Example 4. Selection of ESCC-specific methylation regions from whole genome bisulfite sequencing (WGBS) data

The methylation level was determined using whole genome bisulfite sequencing (WGBS) data (GSE149608, GSE186458, source: https:/www.ncbi.nlm.nih.gov/geo/). DNA extracted from tissues was converted through bisulfite treatment and DNA methylation was determined by modification of cytosine bases. The methylation level was determined for each region and differentially methylated regions between ESCC tissues, surrounding normal tissues, and normal blood samples were selected using the beta value, which represents the methylation level.

The data is shown in Table 7.

[Table 7]

|  | Count |
|---|---|
| **ESCC** | 10 |
| **Normal tissue** | 9 |
| **Blood** | 14 |

Differentially methylated cytosine analysis was performed using 10,405,333 CpG sites with median beta values less than 0.2 or greater than 0.8 in normal esophageal tissue samples and normal blood samples.

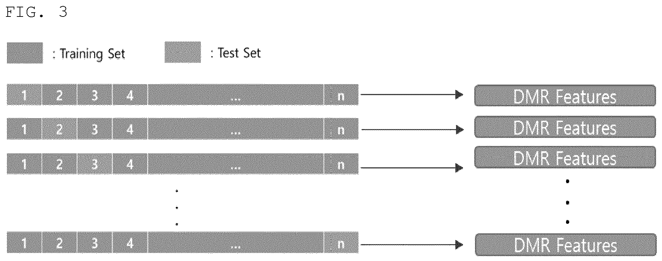

The markers were performed using leave one out (LOO), which is a method of obtaining differentially methylated cytosine (DMC) using a T-test for each fold while excluding all samples once (FIG. 3). For each fold, regions with p-values of 0.01 or less and median beta value differences of 0.05 or more were selected.

As a result, 8,989 hypomethylated sites and 499 hypermethylated sites that differed between ESCC and normal blood samples were selected, and 10,003 hypomethylated sites and 378 hypermethylated sites that differed between ESCC and normal tissue were also selected as candidate markers.

Then, among the regions that differed between normal tissue and normal blood sample, and tumor tissue, the regions that were found to be identical in both cases were selected, and 3,593 hypomethylated regions and 341 hypermethylated regions specific to ESCC were selected. Then, CpG sites that were repeated more than 10 times in the entire fold were selected, 573 hypomethylated regions and 213 hypermethylated regions were selected, and then the selected regions were extended to ±150 bp based on the CpG site to determine the DMR.

For regions overlapping with previously known methylation regions (WO 2019/195268 A2, WO 2019/199696 WO 2019/195268 A2, WO 2019/199696A1), only significant hypermethylation regions were added. The significance was based on the AUC and the group median difference values of the absolute delta beta values used to divide tumor and normal samples, and the criterion for the AUC was 0.9 or higher and the criterion for the median beta value difference between tumor and normal was 0.3 or higher. When only major regions among the known regions were added using the criteria, 36 hypomethylation regions and 119 hypermethylation regions were selected (regions remaining after excluding the known regions: 100 hypermethylation regions, 36 hypomethylation regions, number of major regions among the known regions: 19 hypermethylation regions).

Finally, only the regions with three or more CpG sites in the corresponding DMR were left, and 17 hypomethylated regions and 118 hypermethylated regions were selected. In terms of CpG site units, 19 hypomethylated regions and 134 hypermethylated regions were selected.

Example 5. Selection of esophageal adenocarcinoma (EAC)-specific methylation regions from Infinium Human Methylation 450K BeadChip array data

The methylation level was determined using the infinium human methylation 450K beadChip array data (GSE72872, source: https:/www.ncbi.nlm.nih.gov/geo/). DNA extracted from tissues was converted through bisulfite treatment and DNA methylation was determined by modification of cytosine bases. The methylation level was determined for each region and differentially methylated regions between surrounding normal tissue, EAC tissue, benign disease (gastroesophageal reflux disease) and normal blood sample were selected using the beta value, which represents the methylation level.

The data is shown in Table 8.

[Table 8]

| | Count |
|---|---|
| **EAC** | 125 |
| **Normal tissue** | 75 |
| **GERD** | 10 |
| **Blood** | 656 |

Differentially methylated cytosine (DMC) analysis was performed using 225,287 CpG sites having median beta values less than 0.2 or greater than 0.8 in normal esophageal tissue samples and normal blood samples.

Regions with FDR values less than 0.01 and absolute delta beta greater than 0.25 were selected using Limma (linear models for microarray data) software, and regions that were found to be identical in both regions among regions that differ between normal tissue and normal blood samples, and tumor tissue. As a result, 41,701 hypomethylated regions and 35,723 hypermethylated regions specific for EAC were selected, excluding sex chromosomes.

Then, among the regions having an adjust P value less than 0.005, the top 500 hypermethylated regions and the top 100 hypomethylated regions were selected based on absolute delta beta. The regions were extended to ±150 bp based on the CpG site to obtain DMR.

For regions overlapping with previously known methylation regions (WO 2019/195268 A2, WO 2019/199696 WO 2019/195268 A2, WO 2019/199696A1), only significant hypermethylation regions were added. The significance was based on the AUC and the group median difference values of the absolute delta beta values used to divide tumor and normal samples, and the criterion for the AUC was 0.95 or higher and the criterion for the median beta value difference between tumor and normal was 0.47 or higher. When only major regions among the known regions were added using the criteria, 79 hypomethylation regions and 122 hypermethylation regions were selected (regions remaining after excluding the known regions: 102 hypermethylation regions, 79 hypomethylation regions, number of major regions among the known regions: 20 hypermethylation regions).

Finally, only the regions with three or more CpG sites in the corresponding DMR were left, and 68 hypomethylated regions and 121 hypermethylated regions were selected. In terms of CpG site units, 72 hypomethylated regions and 175 hypermethylated regions were selected.

Example 6. Selection of stomach adenocarcinoma-specific methylation regions from known data

The methylation level was determined using infinium human methylation 450K BeadChip array data from the Cancer Genome Atlas (TCGA) (UCSC Xena, http:/xena.ucsc.edu) and infinium human methylation 450K beadChip array data from a known data set (GSE72872). DNA extracted from tissues was converted through bisulfite treatment and DNA methylation was determined by modification of cytosine bases. The methylation level was determined for each region and differentially methylated regions between stomach cancer tissue, normal tissue and normal blood samples were selected using the beta value, which represents the methylation level. The dataset is shown in Table 9.

[Table 9]

| | Count |
|---|---|
| **Stomach cancer** | 395 |
| **Normal tissue** | 23 |
| **Blood** | 656 |

Differentially methylated cytosine (DMC) analysis was performed using 230,897 CpG sites with median beta values less than 0.2 or greater than 0.8 in normal stomach tissue samples and normal blood samples.

Regions with FDR values less than 0.01 and absolute delta beta greater than 0.25 were selected using Limma (linear models for microarray data) software, and regions that were found to be identical in both regions among regions that differ between normal tissue and normal blood samples, and tumor tissue. As a result, 50,324 hypomethylated regions and 42,214 hypermethylated regions specific for EAC were primarily selected, excluding sex chromosomes.

Then, among the regions having an adjust P value less than 0.005, the top 500 hypermethylated regions and the top 100 hypomethylated regions were selected based on absolute delta beta. The regions were extended to ±150 bp based on the CpG site to obtain DMR.

For regions overlapping with previously known methylation regions (WO 2019/195268 A2, WO 2019/199696 WO 2019/195268 A2, WO 2019/199696A1), only significant hypermethylation regions were added. The significance was based on the AUC and the group median difference values of the absolute delta beta values used to divide tumor and normal samples, and the criterion for the AUC was 95 or higher and the criterion for the median beta value difference between tumor and normal was 0.4 or higher. When only major regions among the known regions were added using the criteria, 76 hypomethylation regions and 132 hypermethylation regions were selected (regions remaining after excluding the known regions: 102 hypermethylation regions, 79 hypomethylation regions, number of major regions among the known regions: 25 hypermethylation regions).

Finally, only the regions with three or more CpG sites in the corresponding DMR were left, and 60 hypomethylated regions and 132 hypermethylated regions were selected. In terms of CpG site units, 70 hypomethylated regions and 179 hypermethylated regions were selected.

Example 7. Selection of final marker candidates

The 18 hypomethylated regions and 29 hypermethylated regions selected in Example 3, the 17 hypomethylated regions and 118 hypermethylated regions selected in Example 4, the 68 hypomethylated regions and 121 hypermethylated regions selected in Example 5, and the 60 hypomethylated regions and 132 hypermethylated regions selected in Example 6 were all integrated and then a total of 646 CpG sites excluding overlapping regions were selected as the final esophageal cancer and gastric cancer diagnostic marker candidates.

The list of the 646 selected methylation markers is shown in Table 10 below.

[Table 10]

| chrom osome | start | end | Cancer type | illumina ProbID | gene | gain /los s |
|---|---|---|---|---|---|---|
| chr1 | 1475675 | 1475676 | EAC | cg16306898 | TMEM240 | gain |
| chr1 | 1475737 | 1475738 | EAC | cg16601494 | TMEM240 | gain |
| chr1 | 1475742 | 1475743 | EAC | cg15487867 | TMEM240 | gain |
| chr1 | 32238660 | 32238661 | ESCC | cg10505580 | - | gain |
| chr1 | 33391780 | 33391781 | ESCC | . | - | gain |
| chr1 | 50893658 | 50893659 | ESCC | cg25738714 | DMRTA2 | gain |
| chr1 | 62660624 | 62660625 | EAC,stom ach | cg23049458 | L1TD1 | gain |
| chr1 | 65991176 | 65991177 | EAC,stom ach | cg08234308 | LEPR | gain |
| chr1 | 77333138 | 77333139 | stomach | cg13823136 | ST6GALNACS | gain |
| chr1 | 91192451 | 91192452 | ESCC | cg06936155 | - | gain |
| chr1 | 91192466 | 91192467 | ESCC | cg22167515 | - | gain |
| chr1 | 107683775 | 107683776 | EAC,stom ach | cg07155336 | NTNG1 | gain |
| chr1 | 111217194 | 111217195 | stomach | cg20302133 | KCNA3 | gain |
| chr1 | 111217406 | 111217407 | EAC,stom ach | cg26013553 | KCNA3 | gain |
| chr1 | 111217497 | 111217498 | EAC,stom ach | cg11595545 | KCNA3 | gain |
| chr1 | 111217575 | 111217576 | EAC,stom ach | cg01423964 | KCNA3 | gain |
| chr1 | 111217691 | 111217692 | EAC,stom ach | cg06750832 | KCNA3 | gain |
| chr1 | 111217712 | 111217713 | EAC,stom ach | cg07808555 | KCNA3 | gain |
| chr1 | 111217715 | 111217716 | EAC | cg09410234 | KCNA3 | gain |

(continued)

| chrom osome | start | end | Cancer type | illumina ProbID | gene | gain /los s |
|---|---|---|---|---|---|---|
| chr1 | 119528430 | 119528431 | ESCC | . | TBX15 | gain |
| chr1 | 147790458 | 147790459 | stomach | cg25200152 | NBPF8 | gain |
| chr1 | 165323445 | 165323446 | ESCC | . | LMX1A | gain |
| chr1 | 169396637 | 169396638 | ESCC | cg13958426 | CCDC181 | gain |
| chr1 | 169396706 | 169396707 | ESCC | cg00002719 | CCDC181 | gain |
| chr1 | 169396712 | 169396713 | ESCC | cg08104202 | CCDC181 | gain |
| chr1 | 169396785 | 169396786 | ESCC | cg23818870 | CCDC181 | gain |
| chr1 | 169396834 | 169396835 | ESCC | cg16998150 | CCDC181 | gain |
| chr1 | 169396858 | 169396859 | ESCC | cg08047907 | CCDC181 | gain |
| chr1 | 169396868 | 169396869 | ESCC | cg24808280 | CCDC181 | gain |
| chr1 | 170630457 | 170630458 | ESCC | cg15711902 | PRRX1 | gain |
| chr1 | 170630558 | 170630559 | ESCC | cg23089825 | PRRX1 | gain |
| chr1 | 170634370 | 170634371 | ESCC | . | PRRX1 | gain |
| chr1 | 175568559 | 175568560 | stomach | cg24932457 | TNR | gain |
| chr1 | 190447290 | 190447291 | stomach | cg23010538 | BRINP3 | gain |
| chr1 | 197890744 | 197890745 | ESCC | . | LHX9 | gain |
| chr1 | 207843167 | 207843168 | ESCC | . | CR1L | gain |
| chr1 | 209393693 | 209393694 | ESCC | . | - | gain |
| chr1 | 229569634 | 229569635 | ESCC | . | ACTA1 | gain |
| chr1 | 240255486 | 240255487 | EAC | cg25208017 | FMN2 | gain |
| chr1 | 246952362 | 246952363 | stomach | cg03596016 | LINC01341 | gain |
| chr2 | 27988785 | 27988786 | ESCC | cg20518572 | - | gain |
| chr2 | 38301756 | 38301757 | stomach | cg09799983 | CYP1B1 | gain |
| chr2 | 45155991 | 45155992 | EAC | cg14168530 | - | gain |
| chr2 | 45160445 | 45160446 | stomach | cg03714619 | - | gain |
| chr2 | 45172169 | 45172170 | stomach | cg08696165 | SIX3 | gain |
| chr2 | 47797963 | 47797964 | stomach | cg13913015 | KCNK12,MSH2 | gain |
| chr2 | 61372138 | 61372139 | stomach | cg16328106 | C2orf74, LOC3 39803 | gain |
| chr2 | 61372256 | 61372257 | stomach | cg18158151 | LOC339803,C2 orf74 | gain |
| chr2 | 63285654 | 63285655 | ESCC | . | - | gain |
| chr2 | 63285950 | 63285951 | ESCC | cg27315333 | - | gain |
| chr2 | 70995459 | 70995460 | EAC | cg15170605 | ADD2 | gain |
| chr2 | 91634631 | 91634632 | ESCC | . | - | gain |
| chr2 | 105471960 | 105471961 | stomach | cg01878345 | POU3F3,PANT R1 | gain |
| chr2 | 119599744 | 119599745 | ESCC | cg20627174 | - | gain |
| chr2 | 119599748 | 119599749 | ESCC | cg21811143 | EN1 | gain |
| chr2 | 119609592 | 119609593 | stomach | cg11236727 | EN1 | gain |
| chr2 | 119615991 | 119615992 | ESCC | . | - | gain |
| chr2 | 127413831 | 127413832 | stomach | cg15975865 | GYPC | gain |

(continued)

| chrom osome | start | end | Cancer type | illumina ProbID | gene | gain /los s |
|---|---|---|---|---|---|---|
| chr2 | 145281942 | 145281943 | EAC | cg16405026 | ZEB2 | gain |
| chr2 | 158300475 | 158300476 | stomach | cg12177677 | CYTIP | gain |
| chr2 | 158300485 | 158300486 | stomach | cg10559416 | CYTIP | gain |
| chr2 | 162280519 | 162280520 | ESCC | cg06382344 | TBR1 | gain |
| chr2 | 172948967 | 172948968 | ESCC | . | DLX1 | gain |
| chr2 | 172952286 | 172952287 | ESCC | . | DLX1 | gain |
| chr2 | 172953392 | 172953393 | ESCC | cg12762816 | DLX1 | gain |
| chr2 | 176936436 | 176936437 | EAC,stom ach | cg09323727 | - | gain |
| chr2 | 176948243 | 176948244 | ESCC | . | EVX2 | gain |
| chr2 | 176956706 | 176956707 | ESCC | . | HOXD13 | gain |
| chr2 | 176956841 | 176956842 | ESCC | cg05256269 | HOXD13 | gain |
| chr2 | 176979604 | 176979605 | ESCC | . | HOXD10 | gain |
| chr2 | 176981654 | 176981655 | ESCC | cg03918304 | HOXD10 | gain |
| chr2 | 176985689 | 176985690 | ESCC | . | HOXD9 | gain |
| chr2 | 176986073 | 176986074 | ESCC | . | HOXD9 | gain |
| chr2 | 176989358 | 176989359 | ESCC | . | HOXD9 | gain |
| chr2 | 176993632 | 176993633 | ESCC | cg09238180 | HOXD8 | gain |
| chr2 | 177004110 | 177004111 | ESCC | . | HOXD-AS2 | gain |
| chr2 | 177036588 | 177036589 | ESCC | . | HOXD3 | gain |
| chr2 | 177036685 | 177036686 | ESCC | . | HOXD3 | gain |
| chr2 | 177036956 | 177036957 | ESCC | . | HOXD3 | gain |
| chr2 | 177054573 | 177054574 | EAC,stom ach | cg02466815 | HAGLR,HOXD 1 | gain |
| chr2 | 198651076 | 198651077 | ESCC | cg07495363 | BOLL | gain |
| chr2 | 198651347 | 198651348 | EAC | cg24589459 | BOLL | gain |
| chr2 | 200327236 | 200327237 | ESCC | . | SATB2 | gain |
| chr2 | 208989248 | 208989249 | stomach | cg22399133 | CRYGD,LOC10 0507443 | gain |
| chr2 | 229045020 | 229045021 | ESCC | cg12001304 | SPHKAP | gain |
| chr3 | 2140699 | 2140700 | EAC | cg06598836 | CNTN4 | gain |
| chr3 | 42947690 | 42947691 | stomach | cg24384244 | ZNF662 | gain |
| chr3 | 119528956 | 119528957 | EAC,stom ach | cg00836482 | NR112 | gain |
| chr3 | 137488619 | 137488620 | ESCC | . | - | gain |
| chr3 | 138655827 | 138655828 | ESCC | cg02744524 | - | gain |
| chr3 | 142839991 | 142839992 | stomach | cg17373442 | CHST2 | gain |
| chr3 | 147074073 | 147074074 | ESCC | cg07906632 | - | gain |
| chr3 | 147108512 | 147108513 | stomach | cg26790247 | ZIC4 | gain |
| chr3 | 147112096 | 147112097 | ESCC | cg26014036 | ZIC4 | gain |

(continued)

| chrom osome | start | end | Cancer type | illumina ProbID | gene | gain /los s |
|---|---|---|---|---|---|---|
| chr3 | 147124173 | 147124174 | ESCC | . | ZIC4,ZIC1 | gain |
| chr3 | 147124417 | 147124418 | ESCC | cg21127068 | ZIC1,ZIC4 | gain |
| chr3 | 147126218 | 147126219 | ESCC | . | ZIC1,ZIC4 | gain |
| chr3 | 147127579 | 147127580 | ESCC | cg01610632 | ZIC4,ZIC1 | gain |
| chr3 | 147128456 | 147128457 | ESCC | . | ZIC4,ZIC1 | gain |
| chr3 | 147140914 | 147140915 | ESCC | . | - | gain |
| chr3 | 157812763 | 157812764 | ESCC | cg18143296 | - | gain |
| chr3 | 157813322 | 157813323 | ESCC | . | - | gain |
| chr3 | 157813363 | 157813364 | ESCC | cg10526277 | - | gain |
| chr3 | 157813417 | 157813418 | ESCC | cg08654915 | - | gain |
| chr3 | 157821262 | 157821263 | ESCC | cg04521 004 | SHOX2 | gain |
| chr3 | 157821407 | 157821408 | ESCC | cg12993163 | SHOX2 | gain |
| chr3 | 157821463 | 157821464 | ESCC | . | SHOX2 | gain |
| chr3 | 157821536 | 157821537 | ESCC | cg09542210 | SHOX2 | gain |
| chr3 | 157821806 | 157821807 | ESCC | . | SHOX2 | gain |
| chr3 | 181441564 | 181441565 | ESCC | . | SOX2-OT | gain |
| chr3 | 184301239 | 184301240 | EAC | cg17342973 | - | gain |
| chr3 | 184301468 | 184301469 | EAC | cg09591072 | - | gain |
| chr3 | 194018158 | 194018159 | EAC,stom ach | cg16924010 | LINC00887 | gain |
| chr4 | 1399235 | 1399236 | stomach | cg24980653 | - | gain |
| chr4 | 3371876 | 3371877 | EAC | cg19974227 | RGS12 | gain |
| chr4 | 4859221 | 4859222 | ESCC | . | MSX1 | gain |
| chr4 | 5710403 | 5710404 | EAC | cg06426416 | EVC2,EVC | gain |
| chr4 | 13525722 | 13525723 | ESCC | . | - | gain |
| chr4 | 13530923 | 13530924 | ESCC | cg17922359 | LINC01097 | gain |
| chr4 | 13531076 | 13531077 | ESCC | cg18120446 | LINC01097 | gain |
| chr4 | 13531163 | 13531164 | ESCC | cg00339682 | LINC01097 | gain |
| chr4 | 81185228 | 81185229 | ESCC | cg12337525 | FGF5 | gain |
| chr4 | 111533346 | 111533347 | ESCC | . | - | gain |
| chr4 | 111533500 | 111533501 | ESCC | . | - | gain |
| chr4 | 111535528 | 111535529 | stomach | cg23806894 | - | gain |
| chr4 | 111539461 | 111539462 | ESCC | . | PITX2 | gain |
| chr4 | 111554647 | 111554648 | ESCC | . | PITX2 | gain |
| chr4 | 111560928 | 111560929 | ESCC | . | PITX2 | gain |
| chr4 | 111561937 | 111561938 | ESCC | . | PITX2 | gain |
| chr4 | 111561962 | 111561963 | ESCC | . | PITX2 | gain |
| chr4 | 111562513 | 111562514 | ESCC | cg03148184 | PITX2 | gain |
| chr4 | 174449640 | 174449641 | ESCC | . | HAND2-AS1,HAND2 | gain |

(continued)

| chrom osome | start | end | Cancer type | illumina ProbID | gene | gain /los s |
|---|---|---|---|---|---|---|
| chr4 | 174452461 | 174452462 | ESCC | cg07681935 | HAND2,HAND 2-AS1 | gain |
| chr5 | 1883954 | 1883955 | EAC | cg23121993 | IRX4 | gain |
| chr5 | 2744422 | 2744423 | ESCC | cg19049964 | - | gain |
| chr5 | 5139853 | 5139854 | EAC,stom ach | cg25973534 | ADAMTS16 | gain |
| chr5 | 5139866 | 5139867 | EAC,stom ach | cg15048991 | ADAMTS16 | gain |
| chr5 | 5139878 | 5139879 | EAC,stom ach | cg04136610 | ADAMTS16 | gain |
| chr5 | 5140001 | 5140002 | EAC,stom ach | cg22954449 | ADAMTS16 | gain |
| chr5 | 42424525 | 42424526 | EAC | cg24773720 | GHR | gain |
| chr5 | 42995369 | 42995370 | stomach | cg24676817 | - | gain |
| chr5 | 42995527 | 42995528 | stomach | cg23052568 | - | gain |
| chr5 | 87955783 | 87955784 | ESCC | . | LINC00461 | gain |
| chr5 | 118609467 | 118609468 | stomach | cg04855216 | TNFAIP8 | gain |
| chr5 | 122430434 | 122430435 | stomach | cg02081006 | PRDM6 | gain |
| chr5 | 136834464 | 136834465 | EAC | cg24847829 | SPOCK1 | gain |
| chr5 | 136834492 | 136834493 | EAC | cg14650610 | SPOCK1 | gain |
| chr5 | 140793468 | 140793469 | ESCC | . | PCDHGB7,PCD HGA10,PCDH GA1,PCDHGA 2,PCDHGA3,P CDHGB1,PCD HGA4,PCDHG B2,PCDHGA5, PCDHGB3,PCD HGA6,PCDHG A7,PCDHGB4, PCDHGA8,PC DHGB5,PCDH GA9,PCDHGB6 | gain |
| chr5 | 140864907 | 140864908 | ESCC | . | PCDHGC5,PCD HGC4,PCDHG A1,PCDHGA2, PCDHGA3,PC DHGB1,PCDH GA4,PCDHGB2 ,PCDHGA5,PC DHGB3,PCDH GA6,PCDHGA 7,PCDHGB4,P CDHGA8,PCD HGB5,PCDHG A9,PCDHGB6, PCDHGA10,PC DHGB7,PCDH GA11,PCDHG A12,PCDHGC3 | gain |
| chr5 | 153853668 | 153853669 | ESCC | . | - | gain |
| chr5 | 153862671 | 153862672 | ESCC | . | HAND1 | gain |
| chr5 | 178004012 | 178004013 | EAC | cg20764887 | COL23A1 | gain |
| chr5 | 178004091 | 178004092 | EAC | cg08475953 | COL23A1 | gain |
| chr5 | 178004204 | 178004205 | stomach | cg06183338 | COL23A1 | gain |
| chr5 | 178367827 | 178367828 | stomach | cg24843380 | ZNF454 | gain |
| chr5 | 178422128 | 178422129 | EAC,stom ach | cg00582971 | GRM6 | gain |
| chr6 | 391743 | 391744 | stomach | cg05766140 | IRF4 | gain |

(continued)

| chromosome | start | end | Cancer type | illumina ProbID | gene | gain/loss s |
|---|---|---|---|---|---|---|
| chr6 | 391764 | 391765 | stomach | cg17228900 | IRF4 | gain |
| chr6 | 391936 | 391937 | stomach | cg06392169 | IRF4 | gain |
| chr6 | 393239 | 393240 | EAC,stomach | cg21277995 | IRF4 | gain |
| chr6 | 1385495 | 1385496 | ESCC | . | FOXF2 | gain |
| chr6 | 1393575 | 1393576 | ESCC | . | FOXF2 | gain |
| chr6 | 10393998 | 10393999 | ESCC | . | - | gain |
| chr6 | 26614649 | 26614650 | ESCC | cg16519587 | - | gain |
| chr6 | 27259006 | 27259007 | ESCC | . | - | gain |
| chr6 | 27526219 | 27526220 | ESCC | . | - | gain |
| chr6 | 28415023 | 28415024 | ESCC | cg09971314 | ZSCAN23 | gain |
| chr6 | 28510306 | 28510307 | stomach | cg11205072 | - | gain |
| chr6 | 29521152 | 29521153 | stomach | cg27546977 | - | gain |
| chr6 | 29521598 | 29521599 | stomach | cg18488157 | - | gain |
| chr6 | 29521602 | 29521603 | stomach | cg00370229 | - | gain |
| chr6 | 29521624 | 29521625 | stomach | cg03409187 | - | gain |
| chr6 | 29521631 | 29521632 | stomach | cg19690984 | - | gain |
| chr6 | 29521751 | 29521752 | stomach | cg22617773 | - | gain |
| chr6 | 29521781 | 29521782 | stomach | cg20034792 | - | gain |
| chr6 | 30139832 | 30139833 | stomach | cg12904880 | TRIM15 | gain |
| chr6 | 30139903 | 30139904 | EAC,stomach | cg08858649 | TRIM15 | gain |
| chr6 | 30139979 | 30139980 | stomach | cg22711111 | TRIM15 | gain |
| chr6 | 30140018 | 30140019 | stomach | cg13567542 | TRIM15 | gain |
| chr6 | 32116653 | 32116654 | EAC,stomach | cg14644001 | PPT2,PRRT1 | gain |
| chr6 | 32116780 | 32116781 | EAC,stomach | cg18243760 | PPT2,PPT2-EGFL8,PRRT1 | gain |
| chr6 | 32117079 | 32117080 | stomach | cg22897615 | PPT2,PPT2-EGFL8,PRRT1 | gain |
| chr6 | 58142636 | 58142637 | ESCC | . | - | gain |
| chr6 | 58147368 | 58147369 | ESCC | . | - | gain |
| chr6 | 58147600 | 58147601 | ESCC | . | - | gain |
| chr6 | 58148980 | 58148981 | ESCC | . | - | gain |
| chr6 | 58148983 | 58148984 | ESCC | . | - | gain |
| chr6 | 73331114 | 73331115 | stomach | cg05447008 | KCNQ5 | gain |
| chr6 | 88876741 | 88876742 | EAC,stomach | cg14186641 | CNR1 | gain |
| chr6 | 100902052 | 100902053 | ESCC | . | SIM1 | gain |
| chr6 | 100911744 | 100911745 | ESCC | cg22478310 | SIM1 | gain |
| chr6 | 100915855 | 100915856 | ESCC | . | SIM1 | gain |

(continued)

| chrom osome | start | end | Cancer type | illumina ProbID | gene | gain /los s |
|---|---|---|---|---|---|---|
| chr6 | 110679566 | 110679567 | EAC | cg20737185 | METTL24 | gain |
| chr6 | 117869524 | 117869525 | ESCC | . | DCBLD1,GOPC | gain |
| chr6 | 133562492 | 133562493 | stomach | cg22871668 | EYA4 | gain |
| chr6 | 161100242 | 161100243 | stomach | cg06500337 | - | gain |
| chr6 | 166218519 | 166218520 | stomach | cg07126525 | - | gain |
| chr7 | 15727441 | 15727442 | stomach | cg00839579 | MEOX2 | gain |
| chr7 | 19146032 | 19146033 | ESCC | cg23244488 | - | gain |
| chr7 | 19157193 | 19157194 | stomach | cg17839237 | TWIST1 | gain |
| chr7 | 19157263 | 19157264 | stomach | cg24446548 | TWIST1 | gain |
| chr7 | 24323799 | 24323800 | stomach | cg16964348 | NPY | gain |
| chr7 | 24323840 | 24323841 | stomach | cg25884711 | NPY | gain |
| chr7 | 24323939 | 24323940 | stomach | cg21097881 | NPY | gain |
| chr7 | 24324435 | 24324436 | ESCC | cg12614105 | NPY | gain |
| chr7 | 24324570 | 24324571 | ESCC | cg11475550 | NPY | gain |
| chr7 | 24324976 | 24324977 | EAC,stom ach | cg24245418 | NPY | gain |
| chr7 | 27225319 | 27225320 | ESCC | cg12810084 | HOXA11,HOX A11-AS | gain |
| chr7 | 27284640 | 27284641 | ESCC | . | EVX1 | gain |
| chr7 | 27284773 | 27284774 | ESCC | . | EVX1 | gain |
| chr7 | 35298391 | 35298392 | stomach | cg08732352 | TBX20 | gain |
| chr7 | 42268098 | 42268099 | ESCC | . | GLI3 | gain |
| chr7 | 42268109 | 42268110 | ESCC | cg10199556 | GLI3 | gain |
| chr7 | 49813033 | 49813034 | EAC | cg04904331 | VWC2 | gain |
| chr7 | 49813088 | 49813089 | EAC | cg01893212 | VWC2 | gain |
| chr7 | 49813111 | 49813112 | EAC | cg09493505 | VWC2 | gain |
| chr7 | 84814683 | 84814684 | EAC,stom ach | cg01578017 | SEMA3D | gain |
| chr7 | 96642510 | 96642511 | ESCC | cg15199678 | DLX6-AS1 | gain |
| chr7 | 96647117 | 96647118 | stomach | cg01680010 | DLX6-AS1 | gain |
| chr7 | 96647288 | 96647289 | ESCC | . | DLX6-AS1 | gain |
| chr7 | 96655444 | 96655445 | stomach | cg19962750 | DLX5 | gain |
| chr7 | 114562847 | 114562848 | stomach | cg11256364 | MDFIC | gain |
| chr7 | 121946423 | 121946424 | ESCC | . | FEZF1,FEZF1-AS1 | gain |
| chr7 | 127672458 | 127672459 | EAC | cg12628196 | LRRC4,SND1 | gain |
| chr7 | 127672564 | 127672565 | EAC | cg09296001 | LRRC4,SND1 | gain |
| chr7 | 127841752 | 127841753 | ESCC | . | - | gain |
| chr7 | 127841836 | 127841837 | ESCC | . | - | gain |
| chr7 | 142494985 | 142494986 | EAC,stom ach | cg09493063 | - | gain |

(continued)

| chrom osome | start | end | Cancer type | illumina ProbID | gene | gain /los s |
|---|---|---|---|---|---|---|
| chr7 | 149389929 | 149389930 | stomach | cg18177414 | - | gain |
| chr7 | 149917263 | 149917264 | EAC | cg02864844 | - | gain |
| chr7 | 151107257 | 151107258 | stomach | cg17362861 | WDR86,WDR8 6-AS1 | gain |
| chr7 | 151107285 | 151107286 | EAC | cg10770742 | WDR86,WDR8 6-AS1 | gain |
| chr7 | 158937969 | 158937970 | stomach | cg23572908 | VIPR2 | gain |
| chr7 | 158938051 | 158938052 | EAC,stom ach | cg25189564 | VIPR2 | gain |
| chr8 | 495736 | 495737 | EAC,stom ach | cg05142982 | TDRP | gain |
| chr8 | 495817 | 495818 | EAC,stom ach | cg19019537 | TDRP | gain |
| chr8 | 688417 | 688418 | EAC | cg26777883 | - | gain |
| chr8 | 23562697 | 23562698 | ESCC | . | NKX2-6 | gain |
| chr8 | 23563124 | 23563125 | ESCC | . | NKX2-6 | gain |
| chr8 | 38965014 | 38965015 | EAC | cg08827307 | ADAM32 | gain |
| chr8 | 38965026 | 38965027 | EAC | cg22848598 | ADAM32 | gain |
| chr8 | 38965252 | 38965253 | EAC | cg02637318 | ADAM32 | gain |
| chr8 | 41167107 | 41167108 | EAC | cg21517947 | SFRP1 | gain |
| chr8 | 41754060 | 41754061 | EAC,stom ach | cg26530758 | ANK1 | gain |
| chr8 | 41754181 | 41754182 | EAC,stom ach | cg17331296 | ANK1 | gain |
| chr8 | 53851707 | 53851708 | EAC | cg24857620 | NPBWR1 | gain |
| chr8 | 53852030 | 53852031 | EAC | cg16330450 | NPBWR1 | gain |
| chr8 | 53852184 | 53852185 | stomach | cg15531403 | NPBWR1 | gain |
| chr8 | 53852422 | 53852423 | EAC,stom ach | cg07770968 | NPBWR1 | gain |
| chr8 | 53852661 | 53852662 | EAC | cg06528306 | NPBWR1 | gain |
| chr8 | 57070013 | 57070014 | EAC | cg16504626 | - | gain |
| chr8 | 59058656 | 59058657 | EAC | cg23881926 | FAM110B | gain |
| chr8 | 65281496 | 65281497 | stomach | cg18249580 | MIR124-2HG | gain |
| chr8 | 65294643 | 65294644 | ESCC | . | MIR124-2HG | gain |
| chr8 | 67090454 | 67090455 | ESCC | . | CRH | gain |
| chr8 | 67344936 | 67344937 | EAC,stom ach | cg01988129 | RRS1-AS1,ADHFE1 | gain |
| chr8 | 67345006 | 67345007 | EAC | cg25046651 | RRS1-AS1,ADHFE1 | gain |
| chr8 | 72470982 | 72470983 | ESCC | cg20386316 | - | gain |
| chr8 | 72754953 | 72754954 | EAC | cg26799209 | MSC-AS1,MSC | gain |
| chr8 | 72755052 | 72755053 | EAC,stom ach | cg13620034 | MSC-AS1,MSC | gain |
| chr8 | 72756155 | 72756156 | stomach | cg09734791 | MSC-AS1,MSC | gain |

(continued)

| chrom osome | start | end | Cancer type | illumina ProbID | gene | gain /los s |
|---|---|---|---|---|---|---|
| chr8 | 72756341 | 72756342 | stomach | cg14409559 | MSC-AS1,MSC | gain |
| chr8 | 80523413 | 80523414 | ESCC | cg09071889 | STMN2 | gain |
| chr8 | 99951730 | 99951731 | ESCC | cg03020554 | OSR2,STK3 | gain |
| chr8 | 99952047 | 99952048 | ESCC | cg06992285 | OSR2,STK3 | gain |
| chr8 | 99952217 | 99952218 | ESCC | cg05236677 | OSR2,STK3 | gain |
| chr8 | 132054387 | 132054388 | ESCC | . | ADCY8 | gain |
| chr8 | 143592375 | 143592376 | EAC,stom ach | cg14669274 | ADGRB1 | gain |
| chr8 | 145106582 | 145106583 | EAC | cg26256223 | OPLAH | gain |
| chr9 | 841850 | 841851 | stomach | cg03168582 | DMRT1 | gain |
| chr9 | 16869732 | 16869733 | ESCC | . | BNC2 | gain |
| chr9 | 96714598 | 96714599 | ESCC | . | BARX1 | gain |
| chr9 | 112810402 | 112810403 | stomach | cg15083233 | PALM2AKAP2 | gain |
| chr9 | 126772435 | 126772436 | ESCC | . | LHX2 | gain |
| chr9 | 126784939 | 126784940 | ESCC | . | LHX2 | gain |
| chr10 | 7451822 | 7451823 | EAC,stom ach | cg25291138 | SFMBT2 | gain |
| chr10 | 44788812 | 44788813 | EAC | cg21163429 | LINC02881 | gain |
| chr10 | 48438724 | 48438725 | EAC | cg04110601 | GDF10 | gain |
| chr10 | 63212507 | 63212508 | ESCC | . | TMEM26 | gain |
| chr10 | 81002218 | 81002219 | EAC | cg26654807 | ZMIZ1 | gain |
| chr10 | 81002480 | 81002481 | EAC | cg17853216 | ZMIZ1 | gain |
| chr10 | 90342738 | 90342739 | EAC | cg04812509 | LIPJ,RNLS | gain |
| chr10 | 90342899 | 90342900 | EAC | cg19235339 | LIPJ,RNLS | gain |
| chr10 | 94822908 | 94822909 | EAC,stom ach | cg05127821 | CYP26C1 | gain |
| chr10 | 118030970 | 118030971 | stomach | cg13320291 | GFRA1 | gain |
| chr10 | 118899407 | 118899408 | EAC | cg25496678 | VAX1 | gain |
| chr10 | 129535893 | 129535894 | EAC,stom ach | cg26115633 | FOXI2 | gain |
| chr10 | 131758331 | 131758332 | stomach | cg25746778 | EBF3 | gain |
| chr10 | 131767526 | 131767527 | stomach | cg16827475 | - | gain |
| chr10 | 133109867 | 133109868 | EAC,stom ach | cg06304097 | TCERG1L | gain |
| chr10 | 133110244 | 133110245 | EAC,stom ach | cg23632875 | TCERG1L | gain |
| chr10 | 133110349 | 133110350 | EAC | cg03109827 | TCERG1L | gain |
| chr11 | 2161892 | 2161893 | EAC | cg13791131 | INS-IGF2,IGF2,IGF -AS | gain |
| chr11 | 2161894 | 2161895 | EAC | cg25574024 | INS-IGF2,IGF2,IGF -AS | gain |
| chr11 | 2162183 | 2162184 | EAC,stom ach | cg23030069 | INS-IGF2,IGF2,IGF -AS | gain |

(continued)

| chrom osome | start | end | Cancer type | illumina ProbID | gene | gain /los s |
|---|---|---|---|---|---|---|
| chr11 | 7273148 | 7273149 | EAC,stom ach | cg18560328 | SYT9 | gain |
| chr11 | 31826421 | 31826422 | ESCC | cg11469061 | PAX6 | gain |
| chr11 | 31826574 | 31826575 | ESCC | cg09537620 | PAX6 | gain |
| chr11 | 31827084 | 31827085 | ESCC | cg18372607 | PAX6 | gain |
| chr11 | 35105199 | 35105200 | EAC | cg18437209 | - | gain |
| chr11 | 43602845 | 43602846 | ESCC | cg15556502 | MIR129-2 | gain |
| chr11 | 43602847 | 43602848 | ESCC | cg14416371 | MIR129-2 | gain |
| chr11 | 43602857 | 43602858 | ESCC | cg14944647 | MIR129-2 | gain |
| chr11 | 57250469 | 57250470 | EAC | cg00264441 | - | gain |
| chr11 | 70508410 | 70508411 | EAC | cg17627654 | SHANK2 | gain |
| chr11 | 105481283 | 105481284 | stomach | cg15603568 | GRIA4 | gain |
| chr11 | 105481292 | 105481293 | stomach | cg23559689 | GRIA4 | gain |
| chr11 | 105481306 | 105481307 | stomach | cg00343633 | GRIA4 | gain |
| chr11 | 105481319 | 105481320 | stomach | cg04747226 | GRIA4 | gain |
| chr11 | 105481509 | 105481510 | stomach | cg19343464 | GRIA4 | gain |
| chr11 | 120435056 | 120435057 | stomach | cg07976064 | GRIK4 | gain |
| chr11 | 123301171 | 123301172 | EAC,stom ach | cg03401096 | - | gain |
| chr11 | 128564180 | 128564181 | stomach | cg17872757 | FLI1,SENCR | gain |
| chr11 | 128564874 | 128564875 | stomach | cg11017065 | FLI1,SENCR | gain |
| chr12 | 4919138 | 4919139 | stomach | cg11224582 | KCNA6 | gain |
| chr12 | 22487847 | 22487848 | EAC | cg17079833 | ST8SIA1 | gain |
| chr12 | 43944732 | 43944733 | ESCC | . | ADAMTS20 | gain |
| chr12 | 54345535 | 54345536 | ESCC | . | HOXC12 | gain |
| chr12 | 54359797 | 54359798 | EAC | cg10641615 | HOTAIR | gain |
| chr12 | 54370482 | 54370483 | ESCC | . | HOTAIR | gain |
| chr12 | 54371258 | 54371259 | ESCC | . | HOTAIR | gain |
| chr12 | 54385304 | 54385305 | ESCC | . | MIR196A2 | gain |
| chr12 | 65218069 | 65218070 | stomach | cg03030717 | TBC1D30 | gain |
| chr12 | 79258407 | 79258408 | stomach | cg03205584 | SYT1 | gain |
| chr12 | 85306828 | 85306829 | EAC | cg18023283 | SLC6A15 | gain |
| chr12 | 103218593 | 103218594 | ESCC | . | LINC00485 | gain |
| chr12 | 114878434 | 114878435 | ESCC | . | - | gain |
| chr12 | 114882846 | 114882847 | ESCC | . | - | gain |
| chr12 | 115102713 | 115102714 | stomach | cg14738806 | - | gain |
| chr12 | 115103960 | 115103961 | stomach | cg16945186 | - | gain |
| chr12 | 115109934 | 115109935 | stomach | cg22635491 | TBX3 | gain |
| chr12 | 115135045 | 115135046 | ESCC | cg05398903 | - | gain |

(continued)

| chrom osome | start | end | Cancer type | illumina ProbID | gene | gain /los s |
|---|---|---|---|---|---|---|
| chr12 | 130388216 | 130388217 | EAC | cg12122146 | TMEM132D | gain |
| chr13 | 28498161 | 28498162 | EAC | cg21900495 | PDX1 | gain |
| chr13 | 28498384 | 28498385 | EAC | cg09975620 | PDX1 | gain |
| chr13 | 28498956 | 28498957 | EAC | cg26299169 | PDX1 | gain |
| chr13 | 28501366 | 28501367 | EAC | cg16300300 | - | gain |
| chr13 | 28503373 | 28503374 | EAC | cg17200768 | - | gain |
| chr13 | 28674451 | 28674452 | stomach | cg07017374 | FLT3 | gain |
| chr13 | 37005489 | 37005490 | EAC,stom ach | cg12417071 | CCNA1 | gain |
| chr13 | 37005501 | 37005502 | EAC,stom ach | cg11513637 | CCNA1 | gain |
| chr13 | 37005570 | 37005571 | EAC,stom ach | cg05089090 | CCNA1 | gain |
| chr13 | 37005582 | 37005583 | EAC | cg08676975 | CCNA1 | gain |
| chr13 | 37006063 | 37006064 | stomach | cg02478448 | CCNA1 | gain |
| chr13 | 37006107 | 37006108 | EAC,stom ach | cg18348647 | CCNA1 | gain |
| chr13 | 37006127 | 37006128 | EAC | cg05137358 | CCNA1 | gain |
| chr13 | 37006340 | 37006341 | EAC,stom ach | cg17495912 | CCNA1 | gain |
| chr13 | 46425866 | 46425867 | stomach | cg08018585 | SIAH3 | gain |
| chr13 | 46425874 | 46425875 | stomach | cg26667946 | SIAH3 | gain |
| chr13 | 58204224 | 58204225 | EAC,stom ach | cg20634967 | PCDH17 | gain |
| chr13 | 78493203 | 78493204 | EAC | cg19916212 | OBI1-AS1,EDNRB | gain |
| chr13 | 78493305 | 78493306 | EAC,stom ach | cg13434989 | OBI1-AS1,EDNRB | gain |
| chr13 | 79176572 | 79176573 | stomach | cg14123923 | POU4F1,OBI1-AS1 | gain |
| chr13 | 84454311 | 84454312 | ESCC | . | SLITRK1 | gain |
| chr13 | 95355117 | 95355118 | ESCC | cg02671880 | - | gain |
| chr13 | 100608123 | 100608124 | ESCC | cg00998451 | - | gain |
| chr13 | 100624939 | 100624940 | ESCC | . | ZIC5 | gain |
| chr13 | 100625852 | 100625853 | ESCC | . | ZIC5 | gain |
| chr13 | 100640377 | 100640378 | ESCC | . | - | gain |
| chr13 | 102068996 | 102068997 | stomach | cg15202954 | NALCN | gain |
| chr13 | 112720221 | 112720222 | ESCC | . | SOX1 | gain |
| chr13 | 112720232 | 112720233 | ESCC | . | SOX1 | gain |
| chr14 | 29229839 | 29229840 | ESCC | . | - | gain |
| chr14 | 29236323 | 29236324 | EAC | cg10300684 | FOXG1 | gain |
| chr14 | 36982445 | 36982446 | ESCC | . | SFTA3 | gain |
| chr14 | 37128425 | 37128426 | ESCC | . | PAX9 | gain |

(continued)

| chrom osome | start | end | Cancer type | illumina ProbID | gene | gain /los s |
|---|---|---|---|---|---|---|
| chr14 | 37129342 | 37129343 | ESCC | cg21207665 | PAX9 | gain |
| chr14 | 38067205 | 38067206 | ESCC | . | FOXA1 | gain |
| chr14 | 38067295 | 38067296 | ESCC | . | FOXA1 | gain |
| chr14 | 38679632 | 38679633 | ESCC | . | SSTR1 | gain |
| chr14 | 51561293 | 51561294 | stomach | cg18683604 | TRIM9 | gain |
| chr14 | 52735421 | 52735422 | EAC | cg18693395 | PTGDR | gain |
| chr14 | 57277068 | 57277069 | ESCC | . | OTX2-AS1,OTX2 | gain |
| chr14 | 60952097 | 60952098 | ESCC | cg05255275 | C14orf39 | gain |
| chr14 | 60952196 | 60952197 | ESCC | cg18921771 | C14orf39 | gain |
| chr14 | 60952405 | 60952406 | ESCC | cg23989821 | C14orf39 | gain |
| chr14 | 60973517 | 60973518 | stomach | cg21026830 | SIX6 | gain |
| chr14 | 60976285 | 60976286 | ESCC | cg19456540 | SIX6 | gain |
| chr14 | 61109855 | 61109856 | ESCC | . | - | gain |
| chr14 | 61109956 | 61109957 | ESCC | . | - | gain |
| chr15 | 28352098 | 28352099 | EAC,stom ach | cg03061682 | - | gain |
| chr15 | 41793881 | 41793882 | EAC | cg08680048 | ITPKA | gain |
| chr15 | 45409319 | 45409320 | stomach | cg19968840 | DUOX2,DUOX A2 | gain |
| chr15 | 60287587 | 60287588 | ESCC | . | - | gain |
| chr15 | 65689152 | 65689153 | EAC | cg18650670 | IGDCC4 | gain |
| chr15 | 65689298 | 65689299 | EAC | cg06899985 | IGDCC4 | gain |
| chr15 | 86233214 | 86233215 | stomach | cg05341539 | AKAP13 | gain |
| chr15 | 86233220 | 86233221 | stomach | cg25947619 | AKAP13 | gain |
| chr15 | 89922328 | 89922329 | ESCC | cg09969277 | MIR9-3HG | gain |
| chr15 | 89922345 | 89922346 | ESCC | cg09086835 | MIR9-3HG | gain |
| chr15 | 96887461 | 96887462 | stomach | cg02326806 | - | gain |
| chr15 | 96887560 | 96887561 | stomach | cg10186131 | - | gain |
| chr15 | 96887919 | 96887920 | stomach | cg22840361 | - | gain |
| chr15 | 101514212 | 101514213 | ESCC | . | LRRK1 | gain |
| chr16 | 9107199 | 9107200 | stomach | cg00970396 | - | gain |
| chr16 | 28074384 | 28074385 | EAC | cg03394150 | GSG1L | gain |
| chr16 | 28074462 | 28074463 | EAC | cg10471437 | GSG1L | gain |
| chr16 | 56697088 | 56697089 | EAC | cg05486035 | - | gain |
| chr16 | 67196917 | 67196918 | ESCC | . | HSF4,TRADD,F BXL8 | gain |
| chr16 | 67197186 | 67197187 | EAC | cg07816687 | HSF4,TRADD,F BXL8 | gain |
| chr16 | 77822419 | 77822420 | stomach | cg07821427 | VAT1L | gain |
| chr16 | 86541480 | 86541481 | stomach | cg16801887 | FOXF1,FENDR R | gain |
| chr17 | 1881082 | 1881083 | ESCC | . | RTN4RL1 | gain |

(continued)

| chromosome | start | end | Cancer type | illumina ProbID | gene | gain/los s |
|---|---|---|---|---|---|---|
| chr17 | 12877321 | 12877322 | EAC,stomach | cg17278864 | ARHGAP44 | gain |
| chr17 | 12877546 | 12877547 | EAC,stomach | cg06766427 | ARHGAP44 | gain |
| chr17 | 36734911 | 36734912 | EAC | cg10184983 | SRCIN1 | gain |
| chr17 | 43339497 | 43339498 | stomach | cg03048083 | MAP3K14-AS1,SPATA32 | gain |
| chr17 | 46800020 | 46800021 | EAC,stomach | cg20945566 | PRAC2,PRAC1 | gain |
| chr17 | 46919180 | 46919181 | ESCC | cg02753187 | CALCOCO2 | gain |
| chr17 | 59534998 | 59534999 | EAC | cg17975443 | TBX4 | gain |
| chr18 | 500979 | 500980 | EAC | cg19461621 | COLEC12 | gain |
| chr18 | 4455337 | 4455338 | EAC | cg25627226 | DLGAP1 | gain |
| chr18 | 5543548 | 5543549 | EAC,stomach | cg07352438 | EPB41L3 | gain |
| chr18 | 11149435 | 11149436 | EAC,stomach | cg03117976 | PIEZO2 | gain |
| chr18 | 11149470 | 11149471 | stomach | cg03686593 | PIEZO2 | gain |
| chr18 | 14748285 | 14748286 | stomach | cg03014326 | ANKRD30B | gain |
| chr18 | 19756702 | 19756703 | ESCC | . | GATA6 | gain |
| chr18 | 32847566 | 32847567 | EAC | cg16657538 | ZSCAN30 | gain |
| chr18 | 32847642 | 32847643 | EAC | cg00487232 | ZSCAN30 | gain |
| chr18 | 55095177 | 55095178 | stomach | cg05735180 | - | gain |
| chr18 | 74290928 | 74290929 | ESCC | . | - | gain |
| chr18 | 74962794 | 74962795 | EAC | cg10390058 | GALR1 | gain |
| chr18 | 76739226 | 76739227 | EAC,stomach | cg13634602 | SALL3 | gain |
| chr18 | 76739559 | 76739560 | stomach | cg16580499 | SALL3 | gain |
| chr19 | 9473674 | 9473675 | stomach | cg13703871 | ZNF177,ZNF55 9-ZNF177 | gain |
| chr19 | 9473684 | 9473685 | stomach | cg08065231 | ZNF177,ZNF55 9-ZNF177 | gain |
| chr19 | 13125084 | 13125085 | EAC | cg20454073 | NFIX | gain |
| chr19 | 15580700 | 15580701 | stomach | cg16206460 | PGLYRP2 | gain |
| chr19 | 17439208 | 17439209 | EAC | cg12526923 | ANO8 | gain |
| chr19 | 17439877 | 17439878 | EAC | cg24192660 | ANO8 | gain |
| chr19 | 21646329 | 21646330 | EAC | cg23004160 | - | gain |
| chr19 | 22018958 | 22018959 | EAC | cg09360501 | ZNF43 | gain |
| chr19 | 22444593 | 22444594 | stomach | cg03692651 | - | gain |
| chr19 | 22817371 | 22817372 | EAC | cg01485075 | ZNF492 | gain |
| chr19 | 31841555 | 31841556 | EAC | cg15208375 | TSHZ3 | gain |
| chr19 | 31842873 | 31842874 | stomach | cg08607018 | TSHZ3 | gain |
| chr19 | 36909413 | 36909414 | EAC,stomach | cg24724633 | LOC644189,ZF P82 | gain |

(continued)

| chrom osome | start | end | Cancer type | illumina ProbID | gene | gain /los s |
|---|---|---|---|---|---|---|
| chr19 | 36909418 | 36909419 | EAC | cg25886284 | LOC644189,ZF P82 | gain |
| chr19 | 37096323 | 37096324 | stomach | cg18630667 | ZNF529,ZNF38 2 | gain |
| chr19 | 37096329 | 37096330 | stomach | cg05020604 | ZNF529,ZNF38 2 | gain |
| chr19 | 37957995 | 37957996 | EAC | cg14142713 | ZNF570,ZNF56 9 | gain |
| chr19 | 37957997 | 37957998 | EAC | cg03884783 | ZNF570,ZNF56 9 | gain |
| chr19 | 37958346 | 37958347 | EAC | cg08630279 | ZNF570,ZNF56 9 | gain |
| chr19 | 48983942 | 48983943 | EAC,stom ach | cg01532436 | CYTH2 | gain |
| chr19 | 48984071 | 48984072 | EAC | cg02590401 | CYTH2 | gain |
| chr19 | 51111677 | 51111678 | stomach | cg10152131 | - | gain |
| chr19 | 53038972 | 53038973 | EAC | cg22455450 | ZNF808 | gain |
| chr19 | 56879571 | 56879572 | EAC | cg26309134 | ZNF542P,ZSC AN5A | gain |
| chr19 | 56879645 | 56879646 | EAC | cg27477373 | ZNF542P,ZSC AN5A | gain |
| chr19 | 56904901 | 56904902 | EAC | cg09568464 | ZNF582-AS1,ZNF582 | gain |
| chr19 | 56904945 | 56904946 | EAC | cg02763101 | ZNF582-AS1,ZNF582 | gain |
| chr19 | 56904965 | 56904966 | EAC | cg22647407 | ZNF582-AS1,ZNF582 | gain |
| chr19 | 56904997 | 56904998 | EAC | cg13916740 | ZNF582-AS1,ZNF582 | gain |
| chr19 | 56905032 | 56905033 | EAC | cg20984085 | ZNF582-AS1,ZNF582 | gain |
| chr19 | 57019069 | 57019070 | EAC,stom ach | cg00674365 | ZNF471 | gain |
| chr19 | 57049695 | 57049696 | EAC | cg12973930 | ZFP28 | gain |
| chr19 | 57049777 | 57049778 | EAC,stom ach | cg25963041 | ZFP28 | gain |
| chr19 | 57050620 | 57050621 | EAC | cg05292954 | ZFP28 | gain |
| chr19 | 57078765 | 57078766 | EAC | cg09265529 | ZNF470 | gain |
| chr19 | 58220295 | 58220296 | ESCC | cg11294513 | ZNF776,ZNF15 4 | gain |
| chr19 | 58220370 | 58220371 | ESCC | cg05661282 | ZNF154,ZNF77 6 | gain |
| chr19 | 58220494 | 58220495 | ESCC | cg21790626 | ZNF154,ZNF77 6 | gain |
| chr19 | 58570419 | 58570420 | EAC,stom ach | cg08701621 | ZNF135 | gain |
| chr19 | 58570466 | 58570467 | EAC,stom ach | cg09907936 | ZNF135 | gain |
| chr20 | 21694708 | 21694709 | ESCC | . | PAX1 | gain |
| chr20 | 25062254 | 25062255 | EAC | cg06151165 | VSX1 | gain |
| chr20 | 25062447 | 25062448 | EAC | cg14763548 | VSX1 | gain |
| chr20 | 34894648 | 34894649 | EAC,stom ach | cg03414318 | DLGAP4 | gain |
| chr20 | 37434229 | 37434230 | stomach | cg11172693 | PPP1R16B | gain |
| chr20 | 41818356 | 41818357 | EAC | cg13168820 | PTPRT | gain |
| chr20 | 41818358 | 41818359 | EAC | cg03403065 | PTPRT | gain |

(continued)

| chrom osome | start | end | Cancer type | illumina ProbID | gene | gain /los s |
|---|---|---|---|---|---|---|
| chr20 | 61808888 | 61808889 | EAC | cg02650317 | MIR124-3 | gain |
| chr21 | 34442360 | 34442361 | EAC | cg17016394 | OLIG1 | gain |
| chr21 | 34442377 | 34442378 | EAC | cg20340508 | OLIG1 | gain |
| chr21 | 38075599 | 38075600 | ESCC | cg22711869 | SIM2 | gain |
| chr21 | 38079691 | 38079692 | ESCC | . | SIM2 | gain |
| chr21 | 38083333 | 38083334 | ESCC | . | SIM2 | gain |
| chr21 | 45336673 | 45336674 | stomach | cg09777775 | AGPAT3 | gain |
| chr1 | 2425860 | 2425861 | stomach | cg17820022 | PLCH2 | loss |
| chr1 | 3139867 | 3139868 | EAC | cg15727188 | PRDM16 | loss |
| chr1 | 3210417 | 3210418 | stomach | cg11157172 | PRDM16 | loss |
| chr1 | 5661886 | 5661887 | EAC | cg18770052 | - | loss |
| chr1 | 19599516 | 19599517 | EAC | cg17431922 | AKR7L | loss |
| chr1 | 21877576 | 21877577 | EAC | cg12047425 | ALPL | loss |
| chr1 | 157401837 | 157401838 | ESCC | . | - | loss |
| chr1 | 162319575 | 162319576 | stomach | cg20299802 | NOS1AP | loss |
| chr1 | 199326920 | 199326921 | ESCC | . | - | loss |
| chr1 | 248366399 | 248366400 | stomach | cg05353872 | OR2M3 | loss |
| chr2 | 1880012 | 1880013 | ESCC | cg22946562 | MYT1L | loss |
| chr2 | 30496325 | 30496326 | stomach | cg21335012 | - | loss |
| chr2 | 100327369 | 100327370 | stomach | cg14552010 | AFF3 | loss |
| chr2 | 100371023 | 100371024 | stomach | cg22092126 | AFF3 | loss |
| chr2 | 169658881 | 169658882 | EAC | cg18556822 | NOSTRIN | loss |
| chr2 | 189064557 | 189064558 | stomach | cg04810745 | - | loss |
| chr2 | 201612944 | 201612945 | EAC | cg20268352 | AOX2P | loss |
| chr2 | 238188707 | 238188708 | EAC | cg16974832 | - | loss |
| chr2 | 238578491 | 238578492 | EAC | cg22734058 | LRRFIP1 | loss |
| chr2 | 238973024 | 238973025 | stomach | cg01439631 | UBE2F-SCLY,SCLY | loss |
| chr2 | 240169161 | 240169162 | EAC | cg19324997 | HDAC4 | loss |
| chr3 | 11178593 | 11178594 | stomach | cg16929739 | HRH1 | loss |
| chr3 | 11178758 | 11178759 | stomach | cg25677394 | HRH1 | loss |
| chr3 | 11178798 | 11178799 | stomach | cg27092248 | HRH1 | loss |
| chr3 | 19987528 | 19987529 | EAC,stomach | cg15227848 | RAB5A,EFHB | loss |
| chr3 | 19987815 | 19987816 | stomach | cg03827118 | RAB5A,EFHB | loss |
| chr3 | 61623299 | 61623300 | EAC | cg11934688 | PTPRG | loss |
| chr3 | 159386992 | 159386993 | EAC | cg25726549 | IQCJ-SCHIP1,SCHIP 1 | loss |
| chr3 | 160410024 | 160410025 | ESCC | . | - | loss |
| chr3 | 160410046 | 160410047 | ESCC | . | - | loss |
| chr3 | 169375110 | 169375111 | EAC | cg13021015 | MECOM | loss |

(continued)

| chrom osome | start | end | Cancer type | illumina ProbID | gene | gain /los s |
|---|---|---|---|---|---|---|
| chr3 | 180003191 | 180003192 | ESCC | . | - | loss |
| chr3 | 180077021 | 180077022 | ESCC | . | - | loss |
| chr3 | 180366355 | 180366356 | ESCC | . | CCDC39 | loss |
| chr3 | 180977934 | 180977935 | ESCC | . | SOX2-OT | loss |
| chr3 | 193840181 | 193840182 | EAC | cg11419262 | - | loss |
| chr3 | 197247751 | 197247752 | EAC | cg19798702 | BDH1 | loss |
| chr4 | 7483577 | 7483578 | ESCC | cg24689061 | SORCS2 | loss |
| chr4 | 54374249 | 54374250 | EAC | cg07080653 | LNX1,PDGFRA, LNX1-AS1 | loss |
| chr5 | 15521782 | 15521783 | stomach | cg18631301 | FBXL7 | loss |
| chr5 | 34687565 | 34687566 | EAC | cg19405883 | RAI14 | loss |
| chr6 | 30131283 | 30131284 | EAC | cg00879790 | TRIM10,TRIM1 5 | loss |
| chr6 | 30131719 | 30131720 | EAC,stom ach | cg09769113 | TRIM10,TRIM1 5 | loss |
| chr6 | 30131749 | 30131750 | EAC,stom ach | cg23778358 | TRIM10,TRIM1 5 | loss |
| chr6 | 30131755 | 30131756 | EAC,stom ach | cg05664039 | TRIM10,TRIM1 5 | loss |
| chr6 | 44698734 | 44698735 | EAC | cg21572599 | - | loss |
| chr6 | 166260012 | 166260013 | stomach | cg20418529 | - | loss |
| chr7 | 544525 | 544526 | EAC | cg14496282 | PDGFA | loss |
| chr7 | 1303351 | 1303352 | EAC | cg02442572 | - | loss |
| chr7 | 3221228 | 3221229 | stomach | cg25403488 | - | loss |
| chr7 | 3221373 | 3221374 | stomach | cg18002126 | - | loss |
| chr7 | 3234697 | 3234698 | EAC | cg16391242 | - | loss |
| chr7 | 23749533 | 23749534 | EAC | cg13802883 | STK31 | loss |
| chr7 | 27154562 | 27154563 | stomach | cg03650946 | HOXA3 | loss |
| chr7 | 38726594 | 38726595 | ESCC | . | FAM183BP | loss |
| chr7 | 40566521 | 40566522 | stomach | cg04099158 | SUGCT | loss |
| chr7 | 41026197 | 41026198 | EAC | cg18355562 | - | loss |
| chr7 | 44083518 | 44083519 | EAC | cg12979234 | DBNL,RASA4C P,LINC00957 | loss |
| chr7 | 55145727 | 55145728 | EAC | cg27598340 | EGFR | loss |
| chr7 | 69434196 | 69434197 | EAC | cg27158793 | AUTS2 | loss |
| chr7 | 73247613 | 73247614 | EAC,stom ach | cg12620035 | - | loss |
| chr7 | 73392697 | 73392698 | stomach | cg00305740 | - | loss |
| chr7 | 97568664 | 97568665 | stomach | cg05950713 | CCZ1P-OR7E38P | loss |
| chr7 | 99137009 | 99137010 | stomach | cg21783672 | - | loss |
| chr7 | 107735684 | 107735685 | EAC | cg19471856 | LAMB4 | loss |
| chr7 | 140227273 | 140227274 | EAC | cg13675721 | DENND2A | loss |
| chr7 | 157468082 | 157468083 | ESCC | cg02478172 | PTPRN2 | loss |

(continued)

| chrom osome | start | end | Cancer type | illumina ProbID | gene | gain /los s |
|---|---|---|---|---|---|---|
| chr7 | 157568273 | 157568274 | ESCC | cg06094238 | PTPRN2 | loss |
| chr7 | 157568342 | 157568343 | ESCC | cg27143007 | PTPRN2 | loss |
| chr8 | 2024249 | 2024250 | EAC | cg17665357 | MYOM2 | loss |
| chr8 | 11839844 | 11839845 | stomach | cg22743884 | DEFB135 | loss |
| chr8 | 13295112 | 13295113 | stomach | cg26125690 | DLC1 | loss |
| chr8 | 23083353 | 23083354 | ESCC,sto mach | cg26530341 | TNFRSF10A,L OC389641 | loss |
| chr8 | 23083551 | 23083552 | ESCC,sto mach | cg24278165 | TNFRSF10A,L OC389641 | loss |
| chr8 | 23083578 | 23083579 | ESCC | cg23303108 | TNFRSF10A,L OC389641 | loss |
| chr8 | 37218068 | 37218069 | EAC | cg00576689 | - | loss |
| chr8 | 53517200 | 53517201 | ESCC | . | - | loss |
| chr8 | 64588228 | 64588229 | ESCC | . | - | loss |
| chr8 | 90624127 | 90624128 | stomach | cg12176793 | - | loss |
| chr8 | 105988283 | 105988284 | stomach | cg11185753 | - | loss |
| chr8 | 110984838 | 110984839 | ESCC | . | KCNV1 | loss |
| chr8 | 117587078 | 117587079 | stomach | cg02185007 | - | loss |
| chr8 | 118629000 | 118629001 | EAC | cg16319142 | - | loss |
| chr8 | 120686985 | 120686986 | stomach | cg19564648 | - | loss |
| chr8 | 124729930 | 124729931 | ESCC | . | ANXA13 | loss |
| chr8 | 124730297 | 124730298 | ESCC | . | ANXA13 | loss |
| chr8 | 139344742 | 139344743 | ESCC | . | FAM135B | loss |
| chr8 | 140630180 | 140630181 | stomach | cg24020826 | KCNK9 | loss |
| chr8 | 144346762 | 144346763 | stomach | cg12888521 | GLI4,ZFP41 | loss |
| chr8 | 144347197 | 144347198 | stomach | cg26039482 | GLI4,ZFP41 | loss |
| chr9 | 79625269 | 79625270 | stomach | cg13692426 | - | loss |
| chr9 | 137097280 | 137097281 | stomach | cg13727473 | - | loss |
| chr9 | 139634196 | 139634197 | ESCC | cg14052065 | LCN10 | loss |
| chr10 | 695844 | 695845 | EAC | cg05585149 | DIP2C-AS1,DIP2C | loss |
| chr10 | 696321 | 696322 | EAC | cg12753366 | DIP2C-AS1,DIP2C | loss |
| chr10 | 696332 | 696333 | EAC | cg14121234 | DIP2C-AS1,DIP2C | loss |
| chr10 | 778709 | 778710 | EAC,stom ach | cg06264868 | - | loss |
| chr10 | 3329966 | 3329967 | EAC,stom ach | cg14534279 | - | loss |
| chr10 | 31388636 | 31388637 | stomach | cg19735151 | - | loss |
| chr10 | 44285864 | 44285865 | ESCC | cg04625975 | HNRNPA3P1 | loss |
| chr10 | 77021867 | 77021868 | EAC | cg03013917 | - | loss |
| chr10 | 95256917 | 95256918 | stomach | cg25314624 | CEP55 | loss |

(continued)

| chrom osome | start | end | Cancer type | illumina ProbID | gene | gain /los s |
|---|---|---|---|---|---|---|
| chr10 | 130185342 | 130185343 | ESCC | cg07834841 | - | loss |
| chr10 | 134588040 | 134588041 | EAC | cg09893465 | INPP5A | loss |
| chr10 | 134732891 | 134732892 | ESCC | cg06919865 | CFAP46 | loss |
| chr10 | 134734612 | 134734613 | ESCC | cg00647046 | CFAP46 | loss |
| chr10 | 134834817 | 134834818 | ESCC | cg11803843 | - | loss |
| chr10 | 135030554 | 135030555 | EAC | cg23709617 | KNDC1 | loss |
| chr11 | 655579 | 655580 | EAC | cg17005319 | DEAF1 | loss |
| chr11 | 1422468 | 1422469 | ESCC | cg25931491 | BRSK2 | loss |
| chr11 | 5009311 | 5009312 | stomach | cg12493906 | MMP26 | loss |
| chr11 | 12188996 | 12188997 | EAC,stom ach | cg01963702 | MICAL2 | loss |
| chr11 | 17563125 | 17563126 | stomach | cg00570628 | USH1C | loss |
| chr11 | 64658726 | 64658727 | EAC | cg27083891 | MIR192,MIR19 4-2 | loss |
| chr11 | 69218633 | 69218634 | EAC | cg05582286 | - | loss |
| chr11 | 111154826 | 111154827 | EAC | cg21127079 | C11orf53 | loss |
| chr12 | 53319217 | 53319218 | stomach | cg08094614 | KRT8 | loss |
| chr13 | 40690368 | 40690369 | EAC | cg26108621 | - | loss |
| chr13 | 113426250 | 113426251 | EAC | cg12194594 | ATP11A | loss |
| chr13 | 113426270 | 113426271 | EAC | cg13995599 | ATP11A | loss |
| chr13 | 113648882 | 113648883 | stomach | cg00086488 | MCF2L | loss |
| chr13 | 114065008 | 114065009 | EAC | cg19088635 | - | loss |
| chr13 | 114065897 | 114065898 | ESCC | cg26081974 | - | loss |
| chr13 | 114770074 | 114770075 | EAC | cg15187223 | RASA3 | loss |
| chr13 | 114917527 | 114917528 | EAC,stom ach | cg00386586 | - | loss |
| chr13 | 114917586 | 114917587 | stomach | cg13697968 | - | loss |
| chr13 | 114917640 | 114917641 | stomach | cg24618387 | - | loss |
| chr14 | 49102467 | 49102468 | ESCC | . | - | loss |
| chr14 | 58599692 | 58599693 | EAC | cg16499677 | ARMH4 | loss |
| chr14 | 65405931 | 65405932 | stomach | cg14947787 | GPX2,CHURC1 | loss |
| | | | | | -FNTB | |
| chr14 | 104600550 | 104600551 | ESCC | cg26120093 | KIF26A | loss |
| chr14 | 105195816 | 105195817 | stomach | cg02263377 | ADSS1 | loss |
| chr15 | 22798986 | 22798987 | stomach | cg20855160 | - | loss |
| chr15 | 90456924 | 90456925 | stomach | cg06184785 | ARPIN-AP3S2,ARPIN | loss |
| chr16 | 552754 | 552755 | stomach | cg00651016 | RAB11FIP3 | loss |
| chr16 | 7640452 | 7640453 | ESCC | . | RBFOX1 | loss |
| chr16 | 21245151 | 21245152 | EAC,stom ach | cg26937434 | ANKS4B | loss |

(continued)

| chrom osome | start | end | Cancer type | illumina ProbID | gene | gain /los s |
|---|---|---|---|---|---|---|
| chr16 | 30456325 | 30456326 | EAC,stom ach | cg01371233 | SEPHS2 | loss |
| chr16 | 33572404 | 33572405 | ESCC | . | - | loss |
| chr16 | 33572413 | 33572414 | ESCC | . | - | loss |
| chr16 | 73266679 | 73266680 | stomach | cg06446408 | - | loss |
| chr16 | 86653215 | 86653216 | EAC | cg06255006 | - | loss |
| chr16 | 87823925 | 87823926 | ESCC | cg16372825 | - | loss |
| chr17 | 30488883 | 30488884 | EAC | cg06674117 | RHOT1 | loss |
| chr17 | 34328592 | 34328593 | stomach | cg12251111 | CCL15,CCL15-CCL14 | loss |
| chr17 | 39059398 | 39059399 | stomach | cg27655168 | - | loss |
| chr17 | 74911520 | 74911521 | EAC | cg04215055 | MGAT5B | loss |
| chr17 | 77174120 | 77174121 | ESCC | cg14602220 | RBFOX3 | loss |
| chr19 | 2278618 | 2278619 | EAC,stom ach | cg27341866 | PEAK3,SPPL2B | loss |
| chr19 | 42408458 | 42408459 | EAC | cg24296761 | ARHGEF1 | loss |
| chr19 | 51128640 | 51128641 | EAC | cg18118147 | SYT3 | loss |
| chr20 | 21589093 | 21589094 | EAC | cg04543516 | - | loss |
| chr20 | 29960982 | 29960983 | stomach | cg14143580 | DEFB118 | loss |
| chr20 | 31592147 | 31592148 | EAC | cg02510802 | BPIFB2,SUN5 | loss |
| chr20 | 43029992 | 43029993 | stomach | cg11542165 | HNF4A | loss |
| chr20 | 43029994 | 43029995 | stomach | cg07064544 | HNF4A | loss |
| chr20 | 44876346 | 44876347 | EAC | cg09814034 | CDH22 | loss |
| chr20 | 44881449 | 44881450 | EAC | cg11152825 | CDH22 | loss |
| chr20 | 56134648 | 56134649 | stomach | cg13655303 | PCK1 | loss |
| chr20 | 56272862 | 56272863 | EAC | cg00177431 | PMEPA1 | loss |
| chr20 | 56273259 | 56273260 | stomach | cg10100887 | PMEPA1 | loss |
| chr20 | 57616191 | 57616192 | stomach | cg20726575 | SLMO2-ATP5E,PRELID 3B | loss |
| chr20 | 59047488 | 59047489 | stomach | cg26765743 | MIR646HG | loss |
| chr20 | 59832924 | 59832925 | EAC | cg06502570 | CDH4 | loss |
| chr20 | 60445998 | 60445999 | EAC | cg15873474 | CDH4 | loss |
| chr20 | 60470267 | 60470268 | ESCC | cg26429674 | CDH4 | loss |
| chr20 | 62048869 | 62048870 | ESCC | cg04649215 | KCNQ2 | loss |
| chr20 | 62121918 | 62121919 | EAC | cg10786876 | EEF1A2 | loss |

The 646 selected methylation markers are summarized by region as shown in Table 11 below.

[Table 11]

| chromosome | start | end | Cancer.type | gene | gain/loss |
|---|---|---|---|---|---|
| chr1 | 1475525 | 1475892 | EAC | TMEM240 | gain |
| chr1 | 32238510 | 32238810 | ESCC | - | gain |
| chr1 | 33391630 | 33391930 | ESCC | - | gain |
| chr1 | 50893501 | 50893800 | ESCC | DMRTA2 | gain |
| chr1 | 62660474 | 62660774 | EAC,stomach | L1TD1 | gain |
| chr1 | 65991026 | 65991326 | EAC,stomach | LEPR | gain |
| chr1 | 77332988 | 77333288 | stomach | ST6GALNAC5 | gain |
| chr1 | 91192201 | 91192500 | ESCC | - | gain |
| chr1 | 107683625 | 107683925 | EAC,stomach | NTNG1 | gain |
| chr1 | 111217044 | 111217865 | EAC,stomach | KCNA3 | gain |
| chr1 | 119528280 | 119528580 | ESCC | TBX15 | gain |
| chr1 | 147790398 | 147790608 | stomach | NBPF8 | gain |
| chr1 | 165323295 | 165323595 | ESCC | LMX1A | gain |
| chr1 | 169396501 | 169397100 | ESCC | CCDC181 | gain |
| chr1 | 170630401 | 170630700 | ESCC | PRRX1 | gain |
| chr1 | 170634220 | 170634520 | ESCC | PRRX1 | gain |
| chr1 | 175568409 | 175568709 | stomach | TNR | gain |
| chr1 | 190447140 | 190447440 | stomach | BRINP3 | gain |
| chr1 | 197890594 | 197890894 | ESCC | LHX9 | gain |
| chr1 | 207843017 | 207843317 | ESCC | CR1L | gain |
| chr1 | 209393543 | 209393843 | ESCC | - | gain |
| chr1 | 229569484 | 229569784 | ESCC | ACTA1 | gain |
| chr1 | 240255336 | 240255636 | EAC | FMN2 | gain |
| chr1 | 246952212 | 246952512 | stomach | LINC01341 | gain |
| chr2 | 27988635 | 27988935 | ESCC | - | gain |
| chr2 | 38301606 | 38301906 | stomach | CYP1B1 | gain |

38

| chromosome | start | end | Cancer.type | gene | gain/loss |
|---|---|---|---|---|---|
| chr2 | 45155841 | 45156141 | EAC | - | gain |
| chr2 | 45160295 | 45160595 | stomach | - | gain |
| chr2 | 45172019 | 45172319 | stomach | SIX3 | gain |
| chr2 | 47797813 | 47798113 | stomach | KCNK12,MSH2 | gain |
| chr2 | 61371988 | 61372406 | stomach | C2orf74,LOC339803 | gain |
| chr2 | 63285504 | 63286100 | ESCC | - | gain |
| chr2 | 70995309 | 70995609 | EAC | ADD2 | gain |
| chr2 | 91634481 | 91634781 | ESCC | - | gain |
| chr2 | 105471810 | 105472110 | stomach | POU3F3,PANTR1 | gain |
| chr2 | 119599501 | 119599800 | ESCC | EN1 | gain |
| chr2 | 119609442 | 119609742 | stomach | EN1 | gain |
| chr2 | 119615841 | 119616141 | ESCC | - | gain |
| chr2 | 127413681 | 127413981 | stomach | GYPC | gain |
| chr2 | 145281792 | 145282092 | EAC | ZEB2 | gain |
| chr2 | 158300325 | 158300635 | stomach | CYTIP | gain |
| chr2 | 162280501 | 162280800 | ESCC | TBR1 | gain |
| chr2 | 172948817 | 172949117 | ESCC | DLX1 | gain |
| chr2 | 172952136 | 172952436 | ESCC | DLX1 | gain |
| chr2 | 172953242 | 172953542 | ESCC | DLX1 | gain |
| chr2 | 176936286 | 176936586 | EAC,stomach | - | gain |
| chr2 | 176948093 | 176948393 | ESCC | EVX2 | gain |
| chr2 | 176956556 | 176956991 | ESCC | HOXD13 | gain |
| chr2 | 176979454 | 176979754 | ESCC | HOXD10 | gain |
| chr2 | 176981504 | 176981804 | ESCC | HOXD10 | gain |
| chr2 | 176985539 | 176985839 | ESCC | HOXD9 | gain |
| chr2 | 176985923 | 176986223 | ESCC | HOXD9 | gain |

| chrom osome | start | end | Cancer.type | gene | gain/ loss |
|---|---|---|---|---|---|
| chr2 | 176989208 | 176989508 | ESCC | HOXD9 | gain |
| chr2 | 176993401 | 176993700 | ESCC | HOXD8 | gain |
| chr2 | 177003960 | 177004260 | ESCC | HOXD-AS2 | gain |
| chr2 | 177036438 | 177037106 | ESCC | HOXD3 | gain |
| chr2 | 177054423 | 177054723 | EAC,stomach | HAGLR,HOXD1 | gain |
| chr2 | 198651001 | 198651497 | EAC,ESCC | BOLL | gain |
| chr2 | 200327086 | 200327386 | ESCC | SATB2 | gain |
| chr2 | 208989098 | 208989398 | stomach | CRYGD,LOC100507443 | gain |
| chr2 | 229044901 | 229045200 | ESCC | SPHKAP | gain |
| chr3 | 2140549 | 2140849 | EAC | CNTN4 | gain |
| chr3 | 42947540 | 42947840 | stomach | ZNF662 | gain |
| chr3 | 119528806 | 119529106 | EAC,stomach | NR1I2 | gain |
| chr3 | 137488469 | 137488769 | ESCC | - | gain |
| chr3 | 138655677 | 138655977 | ESCC | - | gain |
| chr3 | 142839841 | 142840141 | stomach | CHST2 | gain |
| chr3 | 147073801 | 147074100 | ESCC | - | gain |
| chr3 | 147108362 | 147108662 | stomach | ZIC4 | gain |
| chr3 | 147112080 | 147112200 | ESCC | ZIC4 | gain |
| chr3 | 147124023 | 147124567 | ESCC | ZIC1,ZIC4 | gain |
| chr3 | 147126068 | 147126278 | ESCC | ZIC1,ZIC4 | gain |
| chr3 | 147127429 | 147127729 | ESCC | ZIC4,ZIC1 | gain |
| chr3 | 147128306 | 147128606 | ESCC | ZIC4,ZIC1 | gain |
| chr3 | 147140764 | 147141064 | ESCC | - | gain |
| chr3 | 157812613 | 157812913 | ESCC | - | gain |
| chr3 | 157813172 | 157813500 | ESCC | - | gain |
| chr3 | 157821112 | 157821613 | ESCC | SHOX2 | gain |

| chrom osome | start | end | Cancer.type | gene | gain/ loss |
|---|---|---|---|---|---|
| chr3 | 157821656 | 157821956 | ESCC | SHOX2 | gain |
| chr3 | 181441414 | 181441714 | ESCC | SOX2-OT | gain |
| chr3 | 184301089 | 184301618 | EAC | - | gain |
| chr3 | 194018008 | 194018308 | EAC,stomach | LINC00887 | gain |
| chr4 | 1399085 | 1399385 | stomach | - | gain |
| chr4 | 3371726 | 3372026 | EAC | RGS12 | gain |
| chr4 | 4859071 | 4859281 | ESCC | MSX1 | gain |
| chr4 | 5710253 | 5710553 | EAC | EVC2,EVC | gain |
| chr4 | 13525572 | 13525872 | ESCC | - | gain |
| chr4 | 13530901 | 13531200 | ESCC | LINC01097 | gain |
| chr4 | 81185078 | 81185378 | ESCC | FGF5 | gain |
| chr4 | 111533196 | 111533650 | ESCC | - | gain |
| chr4 | 111535378 | 111535678 | stomach | - | gain |
| chr4 | 111539311 | 111539611 | ESCC | PITX2 | gain |
| chr4 | 111554497 | 111554797 | ESCC | PITX2 | gain |
| chr4 | 111560778 | 111561078 | ESCC | PITX2 | gain |
| chr4 | 111561787 | 111562112 | ESCC | PITX2 | gain |
| chr4 | 111562363 | 111562663 | ESCC | PITX2 | gain |
| chr4 | 174449490 | 174449790 | ESCC | HAND2-AS1,HAND2 | gain |
| chr4 | 174452311 | 174452611 | ESCC | HAND2,HAND2-AS1 | gain |
| chr5 | 1883804 | 1884104 | EAC | IRX4 | gain |
| chr5 | 2744272 | 2744572 | ESCC | - | gain |
| chr5 | 5139851 | 5140151 | EAC,stomach | ADAMTS16 | gain |
| chr5 | 42424375 | 42424675 | EAC | GHR | gain |
| chr5 | 42995219 | 42995677 | stomach | - | gain |
| chr5 | 87955633 | 87955933 | ESCC | LINC00461 | gain |

| chrom osome | start | end | Cancer.type | gene | gain/ loss |
|---|---|---|---|---|---|
| chr5 | 118609317 | 118609617 | stomach | TNFAIP8 | gain |
| chr5 | 122430284 | 122430584 | stomach | PRDM6 | gain |
| chr5 | 136834314 | 136834642 | EAC | SPOCK1 | gain |
| chr5 | 140793318 | 140793618 | ESCC | PCDHGB7,PCDHGA10,PC DHGA1,PCDHGA2,PCDH GA3,PCDHGB1,PCDHGA4 ,PCDHGB2,PCDHGA5,PC DHGB3,PCDHGA6,PCDH | gain |
| | | | | GA7,PCDHGB4,PCDHGA8 ,PCDHGB5,PCDHGA9,PC DHGB6 | |
| chr5 | 140864757 | 140865057 | ESCC | PCDHGC5,PCDHGC4,PCD HGA1,PCDHGA2,PCDHG A3,PCDHGB1,PCDHGA4, PCDHGB2,PCDHGA5,PCD HGB3,PCDHGA6,PCDHG A7,PCDHGB4,PCDHGA8, PCDHGB5,PCDHGA9,PCD HGB6,PCDHGA10,PCDHG B7,PCDHGA11,PCDHGA1 2,PCDHGC3 | gain |
| chr5 | 153853518 | 153853818 | ESCC | - | gain |
| chr5 | 153862521 | 153862821 | ESCC | HAND1 | gain |
| chr5 | 178003941 | 178004354 | EAC,stomach | COL23A1 | gain |
| chr5 | 178367677 | 178367977 | stomach | ZNF454 | gain |
| chr5 | 178421978 | 178422278 | EAC,stomach | GRM6 | gain |
| chr6 | 391593 | 392086 | stomach | IRF4 | gain |
| chr6 | 393089 | 393389 | EAC,stomach | IRF4 | gain |
| chr6 | 1385345 | 1385645 | ESCC | FOXF2 | gain |
| chr6 | 1393425 | 1393725 | ESCC | FOXF2 | gain |
| chr6 | 10393848 | 10394148 | ESCC | - | gain |
| chr6 | 26614501 | 26614800 | ESCC | - | gain |
| chr6 | 27258856 | 27259156 | ESCC | - | gain |
| chr6 | 27526069 | 27526369 | ESCC | - | gain |
| chr6 | 28414801 | 28415100 | ESCC | ZSCAN23 | gain |
| chr6 | 28510156 | 28510456 | stomach | - | gain |
| chr6 | 29521002 | 29521302 | stomach | - | gain |
| chr6 | 29521448 | 29521931 | stomach | - | gain |

| chrom osome | start | end | Cancer.type | gene | gain/ loss |
|---|---|---|---|---|---|
| chr6 | 30139682 | 30140168 | EAC,stomach | TRIM15 | gain |
| chr6 | 32116503 | 32117229 | EAC,stomach | PPT2,PPT2-EGFL8,PRRT1 | gain |
| chr6 | 58142486 | 58142786 | ESCC | - | gain |
| chr6 | 58147218 | 58147750 | ESCC | - | gain |
| chr6 | 58148830 | 58149133 | ESCC | - | gain |
| chr6 | 73330964 | 73331264 | stomach | KCNQ5 | gain |
| chr6 | 88876591 | 88876891 | EAC,stomach | CNR1 | gain |
| chr6 | 100901902 | 100902202 | ESCC | SIM1 | gain |
| chr6 | 100911594 | 100911894 | ESCC | SIM1 | gain |
| chr6 | 100915705 | 100916005 | ESCC | SIM1 | gain |
| chr6 | 110679416 | 110679716 | EAC | M ETTL24 | gain |
| chr6 | 117869374 | 117869674 | ESCC | DCBLD1,GOPC | gain |
| chr6 | 133562342 | 133562642 | stomach | EYA4 | gain |
| chr6 | 161100092 | 161100392 | stomach | - | gain |
| chr6 | 166218369 | 166218669 | stomach | - | gain |
| chr7 | 15727291 | 15727591 | stomach | MEOX2 | gain |
| chr7 | 19146001 | 19146300 | ESCC | - | gain |
| chr7 | 19157043 | 19157413 | stomach | TWIST1 | gain |
| chr7 | 24323790 | 24324089 | stomach | NPY | gain |
| chr7 | 24324301 | 24324600 | ESCC | NPY | gain |
| chr7 | 24324826 | 24325126 | EAC,stomach | NPY | gain |
| chr7 | 27225169 | 27225469 | ESCC | HOXA11,HOXA11-AS | gain |
| chr7 | 27284490 | 27284923 | ESCC | EVX1 | gain |
| chr7 | 35298241 | 35298541 | stomach | TBX20 | gain |
| chr7 | 42267948 | 42268259 | ESCC | GLI3 | gain |
| chr7 | 49812883 | 49813261 | EAC | VWC2 | gain |

(continued)

| chromosome | start | end | Cancer.type | gene | gain/loss |
|---|---|---|---|---|---|
| chr7 | 84814533 | 84814833 | EAC,stomach | SEMA3D | gain |
| chr7 | 96642301 | 96642600 | ESCC | DLX6-AS1 | gain |
| chr7 | 96646967 | 96647438 | ESCC,stomach | DLX6-AS1 | gain |
| chr7 | 96655294 | 96655594 | stomach | DLX5 | gain |
| chr7 | 114562697 | 114562997 | stomach | MDFIC | gain |
| chr7 | 121946273 | 121946573 | ESCC | FEZF1,FEZF1-AS1 | gain |
| chr7 | 127672308 | 127672714 | EAC | LRRC4,SND1 | gain |
| chr7 | 127841686 | 127841986 | ESCC | - | gain |
| chr7 | 142494835 | 142495135 | EAC,stomach | - | gain |
| chr7 | 149389779 | 149389989 | stomach | - | gain |
| chr7 | 149917113 | 149917413 | EAC | - | gain |
| chr7 | 151107107 | 151107435 | EAC,stomach | WDR86,WDR86-AS1 | gain |
| chr7 | 158937819 | 158938201 | EAC,stomach | VIPR2 | gain |
| chr8 | 495586 | 495967 | EAC,stomach | TDRP | gain |
| chr8 | 688267 | 688567 | EAC | - | gain |
| chr8 | 23562547 | 23562847 | ESCC | NKX2-6 | gain |
| chr8 | 23562974 | 23563274 | ESCC | NKX2-6 | gain |
| chr8 | 38964864 | 38965402 | EAC | ADAM32 | gain |
| chr8 | 41166957 | 41167257 | EAC | SFRP1 | gain |
| chr8 | 41753910 | 41754210 | EAC,stomach | ANK1 | gain |
| chr8 | 53851557 | 53851857 | EAC | NPBWR1 | gain |
| chr8 | 53851880 | 53852811 | EAC,stomach | NPBWR1 | gain |
| chr8 | 57069863 | 57070163 | EAC | - | gain |
| chr8 | 59058506 | 59058806 | EAC | FAM110B | gain |
| chr8 | 65281346 | 65281646 | stomach | MIR124-2HG | gain |
| chr8 | 65294493 | 65294793 | ESCC | MIR124-2HG | gain |

| chromosome | start | end | Cancer.type | gene | gain/loss |
|---|---|---|---|---|---|
| chr8 | 67090394 | 67090604 | ESCC | CRH | gain |
| chr8 | 67344786 | 67345156 | EAC,stomach | RRS1-AS1,ADHFE1 | gain |
| chr8 | 72470701 | 72471000 | ESCC | - | gain |
| chr8 | 72754803 | 72755202 | EAC,stomach | MSC-AS1,MSC | gain |
| chr8 | 72756005 | 72756491 | stomach | MSC-AS1,MSC | gain |
| chr8 | 80523263 | 80523563 | ESCC | STMN2 | gain |
| chr8 | 99951601 | 99952500 | ESCC | OSR2,STK3 | gain |
| chr8 | 132054237 | 132054537 | ESCC | ADCY8 | gain |
| chr8 | 143592225 | 143592525 | EAC,stomach | ADGRB1 | gain |
| chr8 | 145106432 | 145106732 | EAC | OPLAH | gain |
| chr9 | 841700 | 842000 | stomach | DMRT1 | gain |
| chr9 | 16869582 | 16869882 | ESCC | BNC2 | gain |
| chr9 | 96714448 | 96714748 | ESCC | BARX1 | gain |
| chr9 | 112810252 | 112810552 | stomach | PALM2AKAP2 | gain |
| chr9 | 126772285 | 126772585 | ESCC | LHX2 | gain |
| chr9 | 126784789 | 126785089 | ESCC | LHX2 | gain |
| chr10 | 7451672 | 7451972 | EAC,stomach | SFMBT2 | gain |
| chr10 | 44788662 | 44788962 | EAC | LINC02881 | gain |
| chr10 | 48438574 | 48438874 | EAC | GDF10 | gain |
| chr10 | 63212357 | 63212657 | ESCC | TMEM26 | gain |
| chr10 | 81002068 | 81002630 | EAC | ZMIZ1 | gain |
| chr10 | 90342588 | 90343049 | EAC | LIPJ,RNLS | gain |
| chr10 | 94822758 | 94823058 | EAC,stomach | CYP26C1 | gain |
| chr10 | 118030820 | 118031120 | stomach | GFRA1 | gain |
| chr10 | 118899257 | 118899557 | EAC | VAX1 | gain |
| chr10 | 129535743 | 129536043 | EAC,stomach | FOXI2 | gain |

EP 4 628 597 A1

| chromosome | start | end | Cancer.type | gene | gain/loss |
|---|---|---|---|---|---|
| chr10 | 131758181 | 131758481 | stomach | EBF3 | gain |
| chr10 | 131767376 | 131767676 | stomach | - | gain |
| chr10 | 133109717 | 133110017 | EAC,stomach | TCERG1L | gain |
| chr10 | 133110094 | 133110394 | EAC,stomach | TCERG1L | gain |
| chr11 | 2161742 | 2162333 | EAC,stomach | INS-IGF2,IGF2,IGF2-AS | gain |
| chr11 | 7272998 | 7273298 | EAC,stomach | SYT9 | gain |
| chr11 | 31826401 | 31826700 | ESCC | PAX6 | gain |
| chr11 | 31826934 | 31827234 | ESCC | PAX6 | gain |
| chr11 | 35105049 | 35105349 | EAC | - | gain |
| chr11 | 43602601 | 43602900 | ESCC | MIR129-2 | gain |
| chr11 | 57250319 | 57250619 | EAC | - | gain |
| chr11 | 70508260 | 70508560 | EAC | SHANK2 | gain |
| chr11 | 105481133 | 105481539 | stomach | GRIA4 | gain |
| chr11 | 120434906 | 120435206 | stomach | GRIK4 | gain |
| chr11 | 123301021 | 123301321 | EAC,stomach | - | gain |
| chr11 | 128564030 | 128564330 | stomach | FLI1,SENCR | gain |
| chr11 | 128564724 | 128565024 | stomach | FLI1,SENCR | gain |
| chr12 | 4918988 | 4919288 | stomach | KCNA6 | gain |
| chr12 | 22487697 | 22487997 | EAC | ST8SIA1 | gain |
| chr12 | 43944582 | 43944882 | ESCC | ADAMTS20 | gain |
| chr12 | 54345385 | 54345685 | ESCC | HOXC12 | gain |
| chr12 | 54359647 | 54359947 | EAC | HOTAIR | gain |
| chr12 | 54370332 | 54370632 | ESCC | HOTAIR | gain |
| chr12 | 54371108 | 54371408 | ESCC | HOTAIR | gain |
| chr12 | 54385154 | 54385454 | ESCC | MIR196A2 | gain |
| chr12 | 65217919 | 65218219 | stomach | TBC1D30 | gain |

EP 4 628 597 A1

46

| chrom osome | start | end | Cancer.type | gene | gain/ loss |
|---|---|---|---|---|---|
| chr12 | 79258257 | 79258557 | stomach | SYT1 | gain |
| chr12 | 85306678 | 85306978 | EAC | SLC6A15 | gain |
| chr12 | 103218443 | 103218743 | ESCC | LINC00485 | gain |
| chr12 | 114878284 | 114878584 | ESCC | - | gain |
| chr12 | 114882696 | 114882996 | ESCC | - | gain |
| chr12 | 115102563 | 115102863 | stomach | - | gain |
| chr12 | 115103810 | 115104110 | stomach | - | gain |
| chr12 | 115109784 | 115110084 | stomach | TBX3 | gain |
| chr12 | 115134895 | 115135195 | ESCC | - | gain |
| chr12 | 130388066 | 130388366 | EAC | TMEM132D | gain |
| chr13 | 28498011 | 28498534 | EAC | PDX1 | gain |
| chr13 | 28498806 | 28499106 | EAC | PDX1 | gain |
| chr13 | 28501216 | 28501516 | EAC | - | gain |
| chr13 | 28503223 | 28503523 | EAC | - | gain |
| chr13 | 28674301 | 28674601 | stomach | FLT3 | gain |
| chr13 | 37005339 | 37005720 | EAC,stomach | CCNA1 | gain |
| chr13 | 37005913 | 37006490 | EAC,stomach | CCNA1 | gain |
| chr13 | 46425716 | 46426024 | stomach | SIAH3 | gain |
| chr13 | 58204074 | 58204374 | EAC,stomach | PCDH17 | gain |
| chr13 | 78493155 | 78493455 | EAC,stomach | OBI1-AS1,EDNRB | gain |
| chr13 | 79176422 | 79176722 | stomach | POU4F1,OBI1-AS1 | gain |
| chr13 | 84454161 | 84454461 | ESCC | SLITRK1 | gain |
| chr13 | 95355001 | 95355300 | ESCC | - | gain |
| chr13 | 100607973 | 100608273 | ESCC | - | gain |
| chr13 | 100624789 | 100625089 | ESCC | ZIC5 | gain |
| chr13 | 100625702 | 100626002 | ESCC | ZIC5 | gain |

EP 4 628 597 A1

| chrom osome | start | end | Cancer.type | gene | gain/ loss |
|---|---|---|---|---|---|
| chr13 | 100640227 | 100640527 | ESCC | - | gain |
| chr13 | 102068846 | 102069146 | stomach | NALCN | gain |
| chr13 | 112720071 | 112720382 | ESCC | SOX1 | gain |
| chr14 | 29229689 | 29229989 | ESCC | - | gain |
| chr14 | 29236263 | 29236473 | EAC | FOXG1 | gain |
| chr14 | 36982295 | 36982595 | ESCC | SFTA3 | gain |
| chr14 | 37128275 | 37128575 | ESCC | PAX9 | gain |
| chr14 | 37129201 | 37129500 | ESCC | PAX9 | gain |
| chr14 | 38067055 | 38067445 | ESCC | FOXA1 | gain |
| chr14 | 38679482 | 38679782 | ESCC | SSTR1 | gain |
| chr14 | 51561143 | 51561443 | stomach | TRIM9 | gain |
| chr14 | 52735271 | 52735571 | EAC | PTGDR | gain |
| chr14 | 57276918 | 57277218 | ESCC | OTX2-AS1,OTX2 | gain |
| chr14 | 60951901 | 60952500 | ESCC | C14orf39 | gain |
| chr14 | 60973367 | 60973667 | stomach | SIX6 | gain |
| chr14 | 60976201 | 60976500 | ESCC | SIX6 | gain |
| chr14 | 61109705 | 61110106 | ESCC | - | gain |
| chr15 | 28351948 | 28352248 | EAC,stomach | - | gain |
| chr15 | 41793731 | 41794031 | EAC | ITPKA | gain |
| chr15 | 45409169 | 45409469 | stomach | DUOX2,DUOXA2 | gain |
| chr15 | 60287437 | 60287737 | ESCC | - | gain |
| chr15 | 65689002 | 65689302 | EAC | IGDCC4 | gain |
| chr15 | 86233064 | 86233370 | stomach | AKAP13 | gain |
| chr15 | 89922301 | 89922600 | ESCC | MIR9-3HG | gain |
| chr15 | 96887311 | 96887710 | stomach | - | gain |
| chr15 | 96887769 | 96888069 | stomach | - | gain |

(continued)

| chromosome | start | end | Cancer.type | gene | gain/loss |
|---|---|---|---|---|---|
| chr15 | 101514062 | 101514362 | ESCC | LRRK1 | gain |
| chr16 | 9107049 | 9107349 | stomach | - | gain |
| chr16 | 28074234 | 28074526 | EAC | GSG1L | gain |
| chr16 | 56696938 | 56697238 | EAC | - | gain |
| chr16 | 67196767 | 67197336 | EAC,ESCC | HSF4,TRADD,FBXL8 | gain |
| chr16 | 77822269 | 77822569 | stomach | VAT1L | gain |
| chr16 | 86541330 | 86541630 | stomach | FOXF1,FENDRR | gain |
| chr17 | 1880932 | 1881232 | ESCC | RTN4RL1 | gain |
| chr17 | 12877171 | 12877696 | EAC,stomach | ARHGAP44 | gain |
| chr17 | 36734761 | 36735061 | EAC | SRCIN1 | gain |
| chr17 | 43339347 | 43339647 | stomach | MAP3K14-AS1,SPATA32 | gain |
| chr17 | 46799870 | 46800170 | EAC,stomach | PRAC2,PRAC1 | gain |
| chr17 | 46919030 | 46919330 | ESCC | CALCOCO2 | gain |
| chr17 | 59534848 | 59535148 | EAC | TBX4 | gain |
| chr18 | 500829 | 501129 | EAC | COLEC12 | gain |
| chr18 | 4455187 | 4455487 | EAC | DLGAP1 | gain |
| chr18 | 5543398 | 5543698 | EAC,stomach | EPB41L3 | gain |
| chr18 | 11149285 | 11149620 | EAC,stomach | PIEZO2 | gain |
| chr18 | 14748135 | 14748435 | stomach | ANKRD30B | gain |
| chr18 | 19756552 | 19756852 | ESCC | GATA6 | gain |
| chr18 | 32847416 | 32847716 | EAC | ZSCAN30 | gain |
| chr18 | 55095027 | 55095327 | stomach | - | gain |
| chr18 | 74290778 | 74291078 | ESCC | - | gain |
| chr18 | 74962644 | 74962944 | EAC | GALR1 | gain |
| chr18 | 76739076 | 76739376 | EAC,stomach | SALL3 | gain |
| chr18 | 76739409 | 76739709 | stomach | SALL3 | gain |

| chrom osome | start | end | Cancer.type | gene | gain/ loss |
|---|---|---|---|---|---|
| chr19 | 9473524 | 9473834 | stomach | ZNF177,ZNF559-ZNF177 | gain |
| chr19 | 13124934 | 13125234 | EAC | NFIX | gain |
| chr19 | 15580550 | 15580850 | stomach | PGLYRP2 | gain |
| chr19 | 17439058 | 17439358 | EAC | ANO8 | gain |
| chr19 | 17439727 | 17440027 | EAC | ANO8 | gain |
| chr19 | 21646179 | 21646479 | EAC | - | gain |
| chr19 | 22018808 | 22019108 | EAC | ZNF43 | gain |
| chr19 | 22444443 | 22444743 | stomach | - | gain |
| chr19 | 22817221 | 22817521 | EAC | ZNF492 | gain |
| chr19 | 31841405 | 31841705 | EAC | TSHZ3 | gain |
| chr19 | 31842723 | 31843023 | stomach | TSHZ3 | gain |
| chr19 | 36909263 | 36909568 | EAC,stomach | LOC644189,ZFP82 | gain |
| chr19 | 37096173 | 37096479 | stomach | ZNF529,ZNF382 | gain |
| chr19 | 37957845 | 37958147 | EAC | ZNF570,ZNF569 | gain |
| chr19 | 37958196 | 37958496 | EAC | ZNF570,ZNF569 | gain |
| chr19 | 48983792 | 48984221 | EAC,stomach | CYTH2 | gain |
| chr19 | 51111527 | 51111827 | stomach | - | gain |
| chr19 | 53038822 | 53039122 | EAC | ZNF808 | gain |
| chr19 | 56879421 | 56879795 | EAC | ZNF542P,ZSCAN5A | gain |
| chr19 | 56904751 | 56905182 | EAC | ZNF582-AS1,ZNF582 | gain |
| chr19 | 57018919 | 57019219 | EAC,stomach | ZNF471 | gain |
| chr19 | 57049545 | 57049927 | EAC,stomach | ZFP28 | gain |
| chr19 | 57050470 | 57050770 | EAC | ZFP28 | gain |
| chr19 | 57078615 | 57078915 | EAC | ZNF470 | gain |
| chr19 | 58220101 | 58220700 | ESCC | ZNF154,ZNF776 | gain |
| chr19 | 58570269 | 58570569 | EAC,stomach | ZNF135 | gain |

(continued)

| chromosome | start | end | Cancer.type | gene | gain/loss |
|---|---|---|---|---|---|
| chr20 | 21694558 | 21694858 | ESCC | PAX1 | gain |
| chr20 | 25062104 | 25062597 | EAC | VSX1 | gain |
| chr20 | 34894498 | 34894798 | EAC,stomach | DLGAP4 | gain |
| chr20 | 37434079 | 37434379 | stomach | PPP1R16B | gain |
| chr20 | 41818206 | 41818508 | EAC | PTPRT | gain |
| chr20 | 61808738 | 61809038 | EAC | MIR124-3 | gain |
| chr21 | 34442210 | 34442527 | EAC | OLIG1 | gain |
| chr21 | 38075449 | 38075749 | ESCC | SIM2 | gain |
| chr21 | 38079541 | 38079841 | ESCC | SIM2 | gain |
| chr21 | 38083183 | 38083483 | ESCC | SIM2 | gain |
| chr21 | 45336523 | 45336823 | stomach | AGPAT3 | gain |
| chr1 | 2425710 | 2426010 | stomach | PLCH2 | loss |
| chr1 | 3139807 | 3140017 | EAC | PRDM16 | loss |
| chr1 | 3210267 | 3210567 | stomach | PRDM16 | loss |
| chr1 | 5661736 | 5662036 | EAC | - | loss |
| chr1 | 19599366 | 19599666 | EAC | AKR7L | loss |
| chr1 | 21877426 | 21877726 | EAC | ALPL | loss |
| chr1 | 157401687 | 157401987 | ESCC | - | loss |
| chr1 | 162319425 | 162319725 | stomach | NOS1AP | loss |
| chr1 | 199326770 | 199327070 | ESCC | - | loss |
| chr1 | 248366249 | 248366549 | stomach | OR2M3 | loss |
| chr2 | 1879801 | 1880100 | ESCC | MYT1L | loss |
| chr2 | 30496175 | 30496475 | stomach | - | loss |
| chr2 | 100327219 | 100327519 | stomach | AFF3 | loss |
| chr2 | 100370873 | 100371173 | stomach | AFF3 | loss |
| chr2 | 169658731 | 169659031 | EAC | NOSTRIN | loss |

| chrom osome | start | end | Cancer.type | gene | gain/ loss |
|---|---|---|---|---|---|
| chr2 | 189064407 | 189064707 | stomach | - | loss |
| chr2 | 201612794 | 201613094 | EAC | AOX2P | loss |
| chr2 | 238188557 | 238188857 | EAC | - | loss |
| chr2 | 238578341 | 238578641 | EAC | LRRFIP1 | loss |
| chr2 | 238972874 | 238973174 | stomach | UBE2F-SCLY,SCLY | loss |
| chr2 | 240169011 | 240169311 | EAC | HDAC4 | loss |
| chr3 | 11178443 | 11178948 | stomach | HRH1 | loss |
| chr3 | 19987378 | 19987965 | EAC,stomach | RAB5A,EFHB | loss |
| chr3 | 61623149 | 61623449 | EAC | PTPRG | loss |
| chr3 | 159386842 | 159387142 | EAC | IQCJ-SCHIP1,SCHIP1 | loss |
| chr3 | 160409874 | 160410095 | ESCC | - | loss |
| chr3 | 169374960 | 169375260 | EAC | MECOM | loss |
| chr3 | 180003041 | 180003341 | ESCC | - | loss |
| chr3 | 180076871 | 180077171 | ESCC | - | loss |
| chr3 | 180366205 | 180366505 | ESCC | CCDC39 | loss |
| chr3 | 180977784 | 180978084 | ESCC | SOX2-OT | loss |
| chr3 | 193840031 | 193840331 | EAC | - | loss |
| chr3 | 197247601 | 197247901 | EAC | BDH1 | loss |
| chr4 | 7483501 | 7483800 | ESCC | SORCS2 | loss |
| chr4 | 54374099 | 54374399 | EAC | LNX1,PDGFRA,LNX1-AS1 | loss |
| chr5 | 15521632 | 15521932 | stomach | FBXL7 | loss |
| chr5 | 34687415 | 34687715 | EAC | RAI14 | loss |
| chr6 | 30131133 | 30131433 | EAC | TRIM10,TRIM15 | loss |
| chr6 | 30131569 | 30131905 | EAC,stomach | TRIM10,TRIM15 | loss |
| chr6 | 44698584 | 44698884 | EAC | - | loss |
| chr6 | 166259862 | 166260162 | stomach | - | loss |

EP 4 628 597 A1

| chrom osome | start | end | Cancer.type | gene | gain/ loss |
|---|---|---|---|---|---|
| chr7 | 544375 | 544675 | EAC | PDGFA | loss |
| chr7 | 1303201 | 1303501 | EAC | - | loss |
| chr7 | 3221078 | 3221523 | stomach | - | loss |
| chr7 | 3234547 | 3234847 | EAC | - | loss |
| chr7 | 23749383 | 23749683 | EAC | STK31 | loss |
| chr7 | 27154502 | 27154712 | stomach | HOXA3 | loss |
| chr7 | 38726444 | 38726744 | ESCC | FAM183BP | loss |
| chr7 | 40566371 | 40566671 | stomach | SUGCT | loss |
| chr7 | 41026047 | 41026347 | EAC | - | loss |
| chr7 | 44083458 | 44083668 | EAC | DBNL,RASA4CP,LINC009 57 | loss |
| chr7 | 55145577 | 55145877 | EAC | EGFR | loss |
| chr7 | 69434046 | 69434346 | EAC | AUTS2 | loss |
| chr7 | 73247463 | 73247763 | EAC,stomach | - | loss |
| chr7 | 73392547 | 73392847 | stomach | - | loss |
| chr7 | 97568604 | 97568814 | stomach | CCZ1P-OR7E38P | loss |
| chr7 | 99136949 | 99137159 | stomach | - | loss |
| chr7 | 107735534 | 107735834 | EAC | LAMB4 | loss |
| chr7 | 140227123 | 140227423 | EAC | DENND2A | loss |
| chr7 | 157467901 | 157468200 | ESCC | PTPRN2 | loss |
| chr7 | 157568101 | 157568400 | ESCC | PTPRN2 | loss |
| chr8 | 2024099 | 2024399 | EAC | MYOM2 | loss |
| chr8 | 11839694 | 11839994 | stomach | DEFB135 | loss |
| chr8 | 13294962 | 13295262 | stomach | DLC1 | loss |
| chr8 | 23083321 | 23083680 | ESCC,stomac h | TNFRSF10A,LOC389641 | loss |
| chr8 | 37217918 | 37218218 | EAC | - | loss |
| chr8 | 53517050 | 53517350 | ESCC | - | loss |

EP 4 628 597 A1

(continued)

| chrom osome | start | end | Cancer.type | gene | gain/ loss |
|---|---|---|---|---|---|
| chr8 | 64588078 | 64588378 | ESCC | - | loss |
| chr8 | 90623977 | 90624277 | stomach | - | loss |
| chr8 | 105988133 | 105988433 | stomach | - | loss |
| chr8 | 110984688 | 110984988 | ESCC | KCNV1 | loss |
| chr8 | 117586928 | 117587228 | stomach | - | loss |
| chr8 | 118628850 | 118629150 | EAC | - | loss |
| chr8 | 120686835 | 120687135 | stomach | - | loss |
| chr8 | 124729780 | 124730080 | ESCC | ANXA13 | loss |
| chr8 | 124730147 | 124730447 | ESCC | ANXA13 | loss |
| chr8 | 139344592 | 139344892 | ESCC | FAM135B | loss |
| chr8 | 140630030 | 140630330 | stomach | KCNK9 | loss |
| chr8 | 144346612 | 144346912 | stomach | GLI4,ZFP41 | loss |
| chr8 | 144347047 | 144347257 | stomach | GLI4,ZFP41 | loss |
| chr9 | 79625119 | 79625419 | stomach | - | loss |
| chr9 | 137097130 | 137097430 | stomach | - | loss |
| chr9 | 139634101 | 139634400 | ESCC | LCN10 | loss |
| chr10 | 695694 | 695994 | EAC | DIP2C-AS1,DIP2C | loss |
| chr10 | 696171 | 696482 | EAC | DIP2C-AS1,DIP2C | loss |
| chr10 | 778559 | 778859 | EAC,stomach | - | loss |
| chr10 | 3329816 | 3330116 | EAC,stomach | - | loss |
| chr10 | 31388486 | 31388786 | stomach | - | loss |
| chr10 | 44285701 | 44286000 | ESCC | HNRNPA3P1 | loss |
| chr10 | 77021717 | 77022017 | EAC | - | loss |
| chr10 | 95256767 | 95256977 | stomach | CEP55 | loss |
| chr10 | 130185301 | 130185600 | ESCC | - | loss |
| chr10 | 134587890 | 134588190 | EAC | INPP5A | loss |

(continued)

| chromosome | start | end | Cancer.type | gene | gain/loss |
|---|---|---|---|---|---|
| chr10 | 134732701 | 134733000 | ESCC | CFAP46 | loss |
| chr10 | 134734501 | 134734800 | ESCC | CFAP46 | loss |
| chr10 | 134834701 | 134835000 | ESCC | - | loss |
| chr10 | 135030404 | 135030704 | EAC | KNDC1 | loss |
| chr11 | 655429 | 655729 | EAC | DEAF1 | loss |
| chr11 | 1422301 | 1422600 | ESCC | BRSK2 | loss |
| chr11 | 5009161 | 5009461 | stomach | MMP26 | loss |
| chr11 | 12188846 | 12189146 | EAC,stomach | MICAL2 | loss |
| chr11 | 17562975 | 17563275 | stomach | USH1C | loss |
| chr11 | 64658576 | 64658876 | EAC | MIR192,MIR194-2 | loss |
| chr11 | 69218483 | 69218783 | EAC | - | loss |
| chr11 | 111154676 | 111154976 | EAC | C11orf53 | loss |
| chr12 | 53319067 | 53319367 | stomach | KRT8 | loss |
| chr13 | 40690218 | 40690518 | EAC | - | loss |
| chr13 | 113426100 | 113426420 | EAC | ATP11A | loss |
| chr13 | 113648732 | 113649032 | stomach | MCF2L | loss |
| chr13 | 114064858 | 114065158 | EAC | - | loss |
| chr13 | 114065701 | 114066000 | ESCC | - | loss |
| chr13 | 114769924 | 114770224 | EAC | RASA3 | loss |
| chr13 | 114917377 | 114917790 | EAC,stomach | - | loss |
| chr14 | 49102317 | 49102617 | ESCC | - | loss |
| chr14 | 58599632 | 58599842 | EAC | ARMH4 | loss |
| chr14 | 65405781 | 65406081 | stomach | GPX2,CHURC1-FNTB | loss |
| chr14 | 104600401 | 104600700 | ESCC | KIF26A | loss |
| chr14 | 105195666 | 105195966 | stomach | ADSS1 | loss |
| chr15 | 22798836 | 22799136 | stomach | - | loss |

| chrom osome | start | end | Cancer.type | gene | gain/ loss |
|---|---|---|---|---|---|
| chr15 | 90456774 | 90457074 | stomach | ARPIN-AP3S2,ARPIN | loss |
| chr16 | 552604 | 552904 | stomach | RAB11FIP3 | loss |
| chr16 | 7640302 | 7640512 | ESCC | RBFOX1 | loss |
| chr16 | 21245001 | 21245301 | EAC,stomach | ANKS4B | loss |
| chr16 | 30456175 | 30456475 | EAC,stomach | SEPHS2 | loss |
| chr16 | 33572254 | 33572563 | ESCC | - | loss |
| chr16 | 73266529 | 73266829 | stomach | - | loss |
| chr16 | 86653065 | 86653365 | EAC | - | loss |
| chr16 | 87823801 | 87824100 | ESCC | - | loss |
| chr17 | 30488733 | 30489033 | EAC | RHOT1 | loss |
| chr17 | 34328442 | 34328742 | stomach | CCL15,CCL15-CCL14 | loss |
| chr17 | 39059248 | 39059548 | stomach | - | loss |
| chr17 | 74911370 | 74911670 | EAC | MGAT5B | loss |
| chr17 | 77174101 | 77174400 | ESCC | RBFOX3 | loss |
| chr19 | 2278468 | 2278768 | EAC,stomach | PEAK3,SPPL2B | loss |
| chr19 | 42408308 | 42408608 | EAC | ARHGEF1 | loss |
| chr19 | 51128490 | 51128790 | EAC | SYT3 | loss |
| chr20 | 21588943 | 21589243 | EAC | - | loss |
| chr20 | 29960832 | 29961132 | stomach | DEFB118 | loss |
| chr20 | 31591997 | 31592297 | EAC | BPIFB2,SUN5 | loss |
| chr20 | 43029842 | 43030144 | stomach | HNF4A | loss |
| chr20 | 44876196 | 44876496 | EAC | CDH22 | loss |
| chr20 | 44881299 | 44881599 | EAC | CDH22 | loss |
| chr20 | 56134498 | 56134798 | stomach | PCK1 | loss |
| chr20 | 56272712 | 56273012 | EAC | PMEPA1 | loss |
| chr20 | 56273109 | 56273409 | stomach | PMEPA1 | loss |

| chrom osome | start | end | Cancer.type | gene | gain/ loss |
|---|---|---|---|---|---|
| chr20 | 57616041 | 57616341 | stomach | SLMO2-ATP5E,PRELID3B | loss |
| chr20 | 59047338 | 59047638 | stomach | MIR646HG | loss |
| chr20 | 59832774 | 59833074 | EAC | CDH4 | loss |
| chr20 | 60445848 | 60446058 | EAC | CDH4 | loss |
| chr20 | 60470101 | 60470400 | ESCC | CDH4 | loss |
| chr20 | 62048701 | 62049000 | ESCC | KCNQ2 | loss |
| chr20 | 62121768 | 62122068 | EAC | EEF1A2 | loss |

Example 8. Final marker selection and performance verification

8-1. A. cfDNA Enzymatic Methylation sequencing (cfEM-seq)

Blood was collected from 48 esophageal cancer patients and 32 normal subjects, and only the plasma portion was primarily centrifuged under the conditions of 3,000 rpm and 25°C for 10 minutes. The plasma from the first centrifugation was secondarily centrifuged under the conditions of 16,000g and 25°C for 10 minutes to separate the plasma. Cell-free DNA was extracted using the Mag-bind cfDNA kit (Omega).

The concentration of the extracted cfDNA was measured using the Qubit DS DNA HS assay Kit (Thermo Fisher Scientific, USA), and the cfDNA size was measured using the Tapestation 4200 (Agilent, USA).

The maximum amount of extracted cfDNA was used and the library was produced by performing methylation conversion through substitution of unmethylated cytosine with uracil using ten-eleven translocation dioxygenase 2 (TET2) and APOBEC using enzymatic methyl-seq (NEB Kit), and the concentration and size of the produced DNA library were measured using Qubit DS DNA HS assay Kit (Thermo Fisher Scientific, USA) and Tapestation 4200 (Agilent, USA), respectively.

The 250 ng library was pooled into 8 samples, hybridization was performed, the concentration of the captured sample was measured using Qubit DS DNA HS assay Kit (Thermo Fisher Scientific, USA), and the captured DNA size was measured using high sensitivity D1000 screen tape & Reagent (Agilent, USA) with Tapestation 4200 (Agilent, USA).

Sequencing was performed using Miseq Dx (Illumina) equipment in 150 paired-end mode with a final concentration of 11pM. 500X depth was produced per sample.

8-2. Final marker selection and performance verification

48 ESCC patients and 32 normal subjects were used, the discovery set and external set were divided, markers were selected using the discovery set, and performance was measured using the external set. The data is shown in Table 12 below.

[Table 12]

|  | **Discovery** | **External** |
|---|---|---|
| **ESCC** | 16 | 32 |
| **Normal** | 24 | 8 |

Among the 497 regions, the regions with normal methylation beta values of 20 or less or 80 or more, SD of 5 or less and a median coverage of 100 or more in the discovery set were selected as the final marker set (region 474: / CpG sites: 8423), and the list is shown in Table 13 below.

[Table 13]

| chromosome | start | end | Cancer.type | gene | gain/loss |
|---|---|---|---|---|---|
| chr1 | 1475525 | 1475892 | EAC | TMEM240 | gain |
| chr1 | 32238510 | 32238810 | ESCC | - | gain |
| chr1 | 33391630 | 33391930 | ESCC | - | gain |
| chr1 | 50893501 | 50893800 | ESCC | DMRTA2 | gain |
| chr1 | 62660474 | 62660774 | EAC,stomach | L1TD1 | gain |
| chr1 | 65991026 | 65991326 | EAC,stomach | LEPR | gain |
| chr1 | 77332988 | 77333288 | stomach | ST6GALNAC5 | gain |
| chr1 | 91192201 | 91192500 | ESCC | - | gain |
| chr1 | 10768362 5 | 10768392 5 | EAC,stomach | NTNG1 | gain |
| chr1 | 11121704 4 | 11121786 5 | EAC,stomach | KCNA3 | gain |
| chr1 | 11952828 0 | 11952858 0 | ESCC | TBX15 | gain |
| chr1 | 14779039 8 | 14779060 8 | stomach | NBPF8 | gain |

(continued)

| chromosome | start | end | Cancer.type | gene | gain/loss |
|---|---|---|---|---|---|
| chr1 | 16532329 5 | 16532359 5 | ESCC | LMX1A | gain |
| chr1 | 16939650 1 | 16939710 0 | ESCC | CCDC181 | gain |
| chr1 | 17063040 1 | 17063070 0 | ESCC | PRRX1 | gain |
| chr1 | 17063422 0 | 17063452 0 | ESCC | PRRX1 | gain |
| chr1 | 17556840 9 | 17556870 9 | stomach | TNR | gain |
| chr1 | 19044714 0 | 19044744 0 | stomach | BRINP3 | gain |
| chr1 | 19789059 4 | 19789089 4 | ESCC | LHX9 | gain |
| chr1 | 20784301 7 | 20784331 7 | ESCC | CR1L | gain |
| chr1 | 22956948 4 | 22956978 4 | ESCC | ACTA1 | gain |
| chr1 | 24025533 6 | 24025563 6 | EAC | FMN2 | gain |
| chr1 | 24695221 2 | 24695251 2 | stomach | LINC01341 | gain |
| chr2 | 38301606 | 38301906 | stomach | CYP1B1 | gain |
| chr2 | 45155841 | 45156141 | EAC | - | gain |
| chr2 | 45160295 | 45160595 | stomach | - | gain |
| chr2 | 45172019 | 45172319 | stomach | SIX3 | gain |
| chr2 | 47797813 | 47798113 | stomach | KCNK12,MSH2 | gain |
| chr2 | 61371988 | 61372406 | stomach | C2orf74,LOC339 803 | gain |
| chr2 | 63285504 | 63286100 | ESCC | - | gain |
| chr2 | 70995309 | 70995609 | EAC | ADD2 | gain |
| chr2 | 91634481 | 91634781 | ESCC | - | gain |
| chr2 | 105471810 | 105472110 | stomach | POU3F3,PANTR1 | gain |
| chr2 | 11959950 1 | 11959980 0 | ESCC | EN1 | gain |
| chr2 | 11960944 2 | 11960974 2 | stomach | EN1 | gain |
| chr2 | 11961584 1 | 11961614 1 | ESCC | - | gain |
| chr2 | 12741368 1 | 12741398 1 | stomach | GYPC | gain |
| chr2 | 14528179 2 | 14528209 2 | EAC | ZEB2 | gain |
| chr2 | 15830032 5 | 15830063 5 | stomach | CYTIP | gain |
| chr2 | 16228050 1 | 16228080 0 | ESCC | TBR1 | gain |
| chr2 | 17294881 7 | 17294911 7 | ESCC | DLX1 | gain |
| chr2 | 17295213 6 | 17295243 6 | ESCC | DLX1 | gain |
| chr2 | 17295324 2 | 17295354 2 | ESCC | DLX1 | gain |
| chr2 | 17693628 6 | 17693658 6 | EAC,stomach | - | gain |
| chr2 | 17694809 3 | 17694839 3 | ESCC | EVX2 | gain |
| chr2 | 17695655 6 | 17695699 1 | ESCC | HOXD13 | gain |
| chr2 | 17697945 4 | 17697975 4 | ESCC | HOXD10 | gain |
| chr2 | 17698553 9 | 17698583 9 | ESCC | HOXD9 | gain |
| chr2 | 17698592 3 | 17698622 3 | ESCC | HOXD9 | gain |
| chr2 | 176989208 | 176989508 | ESCC | HOXD9 | gain |
| chr2 | 17699340 1 | 17699370 0 | ESCC | HOXD8 | gain |

(continued)

| chromosome | start | end | Cancer.type | gene | gain/loss |
|---|---|---|---|---|---|
| chr2 | 17700396 0 | 17700426 0 | ESCC | HOXD-AS2 | gain |
| chr2 | 17703643 8 | 17703710 6 | ESCC | HOXD3 | gain |
| chr2 | 17705442 3 | 17705472 3 | EAC,stomach | HAGLR,HOXD1 | gain |
| chr2 | 19865100 1 | 19865149 7 | ESCC,EAC | BOLL | gain |
| chr2 | 20032708 6 | 20032738 6 | ESCC | SATB2 | gain |
| chr2 | 20898909 8 | 20898939 8 | stomach | CRYGD,LOC1005 07443 | gain |
| chr2 | 22904490 1 | 22904520 0 | ESCC | SPHKAP | gain |
| chr3 | 2140549 | 2140849 | EAC | CNTN4 | gain |
| chr3 | 42947540 | 42947840 | stomach | ZN F662 | gain |
| chr3 | 11952880 6 | 11952910 6 | EAC,stomach | NR112 | gain |
| chr3 | 13748846 9 | 13748876 9 | ESCC | - | gain |
| chr3 | 13865567 7 | 13865597 7 | ESCC | - | gain |
| chr3 | 14283984 1 | 14284014 1 | stomach | CHST2 | gain |
| chr3 | 14707380 1 | 14707410 0 | ESCC | - | gain |
| chr3 | 14710836 2 | 14710866 2 | stomach | ZIC4 | gain |
| chr3 | 14711208 0 | 14711220 0 | ESCC | ZIC4 | gain |
| chr3 | 147124023 | 147124567 | ESCC | ZIC1,ZIC4 | gain |
| chr3 | 14712606 8 | 14712627 8 | ESCC | ZIC1,ZIC4 | gain |
| chr3 | 14712742 9 | 14712772 9 | ESCC | ZIC4,ZIC1 | gain |
| chr3 | 14712830 6 | 14712860 6 | ESCC | ZIC4,ZIC1 | gain |
| chr3 | 14714076 4 | 14714106 4 | ESCC | - | gain |
| chr3 | 15781261 3 | 15781291 3 | ESCC | - | gain |
| chr3 | 15781317 2 | 15781350 0 | ESCC | - | gain |
| chr3 | 15782111 2 | 15782161 3 | ESCC | SHOX2 | gain |
| chr3 | 15782165 6 | 15782195 6 | ESCC | SHOX2 | gain |
| chr3 | 18144141 4 | 18144171 4 | ESCC | SOX2-OT | gain |
| chr3 | 18430108 9 | 18430161 8 | EAC | - | gain |
| chr4 | 1399085 | 1399385 | stomach | - | gain |
| chr4 | 3371726 | 3372026 | EAC | RGS12 | gain |
| chr4 | 4859071 | 4859281 | ESCC | MSX1 | gain |
| chr4 | 5710253 | 5710553 | EAC | EVC2,EVC | gain |
| chr4 | 13525572 | 13525872 | ESCC | - | gain |
| chr4 | 13530901 | 13531200 | ESCC | LINC01097 | gain |
| chr4 | 81185078 | 81185378 | ESCC | FGF5 | gain |
| chr4 | 11153319 6 | 11153365 0 | ESCC | - | gain |
| chr4 | 11153537 8 | 11153567 8 | stomach | - | gain |
| chr4 | 111539311 | 111539611 | ESCC | PITX2 | gain |
| chr4 | 11155449 7 | 11155479 7 | ESCC | PITX2 | gain |
| chr4 | 11156077 8 | 11156107 8 | ESCC | PITX2 | gain |

(continued)

| chromosome | start | end | Cancer.type | gene | gain/loss |
|---|---|---|---|---|---|
| chr4 | 11156178 7 | 11156211 2 | ESCC | PITX2 | gain |
| chr4 | 11156236 3 | 11156266 3 | ESCC | PITX2 | gain |
| chr4 | 17444949 0 | 17444979 0 | ESCC | HAND2-AS1,HAND2 | gain |
| chr4 | 17445231 1 | 17445261 1 | ESCC | HAND2,HAND2-AS1 | gain |
| chr5 | 1883804 | 1884104 | EAC | IRX4 | gain |
| chr5 | 5139851 | 5140151 | EAC,stomach | ADAMTS16 | gain |
| chr5 | 42424375 | 42424675 | EAC | GHR | gain |
| chr5 | 42995219 | 42995677 | stomach | - | gain |
| chr5 | 87955633 | 87955933 | ESCC | LINC00461 | gain |
| chr5 | 11860931 7 | 11860961 7 | stomach | TNFAIP8 | gain |
| chr5 | 12243028 4 | 12243058 4 | stomach | PRDM6 | gain |
| chr5 | 13683431 4 | 13683464 2 | EAC | SPOCK1 | gain |
| chr5 | 14079331 8 | 14079361 8 | ESCC | PCDHGB7,PCDH GA10,PCDHGA1, PCDHGA2,PCDH GA3,PCDHGB1,P CDHGA4,PCDHG B2,PCDHGA5,PC DHGB3,PCDHGA 6,PCDHGA7,PCD HGB4,PCDHGA8, PCDHGB5,PCDH GA9,PCDHGB6 | gain |
| chr5 | 14086475 7 | 14086505 7 | ESCC | PCDHGC5,PCDH GC4,PCDHGA1,P CDHGA2,PCDHG A3,PCDHGB1,PC DHGA4,PCDHGB 2,PCDHGA5,PCD HGB3,PCDHGA6, PCDHGA7,PCDH GB4,PCDHGA8,P CDHGB5,PCDHG A9,PCDHGB6,PC DHGA10,PCDHG B7,PCDHGA11,P CDHGA12,PCDH GC3 | gain |
| chr5 | 15385351 8 | 15385381 8 | ESCC | - | gain |
| chr5 | 15386252 1 | 15386282 1 | ESCC | HAND1 | gain |
| chr5 | 17800394 1 | 17800435 4 | EAC,stomach | COL23A1 | gain |
| chr5 | 17836767 7 | 17836797 7 | stomach | ZN F454 | gain |
| chr5 | 17842197 8 | 17842227 8 | EAC,stomach | GRM6 | gain |
| chr6 | 391593 | 392086 | stomach | IRF4 | gain |
| chr6 | 393089 | 393389 | EAC,stomach | IRF4 | gain |
| chr6 | 1385345 | 1385645 | ESCC | FOXF2 | gain |
| chr6 | 1393425 | 1393725 | ESCC | FOXF2 | gain |

(continued)

| chromosome | start | end | Cancer.type | gene | gain/loss |
|---|---|---|---|---|---|
| chr6 | 10393848 | 10394148 | ESCC | - | gain |
| chr6 | 26614501 | 26614800 | ESCC | - | gain |
| chr6 | 27258856 | 27259156 | ESCC | - | gain |
| chr6 | 27526069 | 27526369 | ESCC | - | gain |
| chr6 | 28414801 | 28415100 | ESCC | ZSCAN23 | gain |
| chr6 | 58142486 | 58142786 | ESCC | - | gain |
| chr6 | 58147218 | 58147750 | ESCC | - | gain |
| chr6 | 58148830 | 58149133 | ESCC | - | gain |
| chr6 | 73330964 | 73331264 | stomach | KCNQ5 | gain |
| chr6 | 88876591 | 88876891 | EAC,stomach | CNR1 | gain |
| chr6 | 10090190 2 | 10090220 2 | ESCC | SIM1 | gain |
| chr6 | 10091159 4 | 10091189 4 | ESCC | SIM1 | gain |
| chr6 | 10091570 5 | 10091600 5 | ESCC | SIM1 | gain |
| chr6 | 11067941 6 | 11067971 6 | EAC | METTL24 | gain |
| chr6 | 13356234 2 | 13356264 2 | stomach | EYA4 | gain |
| chr6 | 16110009 2 | 16110039 2 | stomach | - | gain |
| chr6 | 166218369 | 166218669 | stomach | - | gain |
| chr7 | 15727291 | 15727591 | stomach | MEOX2 | gain |
| chr7 | 19146001 | 19146300 | ESCC | - | gain |
| chr7 | 19157043 | 19157413 | stomach | TWIST1 | gain |
| chr7 | 24323790 | 24324089 | stomach | NPY | gain |
| chr7 | 24324301 | 24324600 | ESCC | NPY | gain |
| chr7 | 24324826 | 24325126 | EAC,stomach | NPY | gain |
| chr7 | 27225169 | 27225469 | ESCC | HOXA11,HOXA1 1-AS | gain |
| chr7 | 27284490 | 27284923 | ESCC | EVX1 | gain |
| chr7 | 35298241 | 35298541 | stomach | TBX20 | gain |
| chr7 | 42267948 | 42268259 | ESCC | GLI3 | gain |
| chr7 | 49812883 | 49813261 | EAC | VWC2 | gain |
| chr7 | 84814533 | 84814833 | EAC,stomach | SEMA3D | gain |
| chr7 | 96642301 | 96642600 | ESCC | DLX6-AS1 | gain |
| chr7 | 96646967 | 96647438 | ESCC,stomach | DLX6-AS1 | gain |
| chr7 | 96655294 | 96655594 | stomach | DLX5 | gain |
| chr7 | 11456269 7 | 11456299 7 | stomach | MDFIC | gain |
| chr7 | 12194627 3 | 12194657 3 | ESCC | FEZF1,FEZF1-AS1 | gain |
| chr7 | 12767230 8 | 12767271 4 | EAC | LRRC4,SND1 | gain |
| chr7 | 12784168 6 | 12784198 6 | ESCC | - | gain |
| chr7 | 14249483 5 | 14249513 5 | EAC,stomach | - | gain |
| chr7 | 14938977 9 | 14938998 9 | stomach | - | gain |
| chr7 | 14991711 3 | 14991741 3 | EAC | - | gain |

(continued)

| chromosome | start | end | Cancer.type | gene | gain/loss |
|---|---|---|---|---|---|
| chr7 | 15110710 7 | 15110743 5 | EAC,stomach | WDR86,WDR86-AS1 | gain |
| chr7 | 15893781 9 | 15893820 1 | EAC,stomach | VIPR2 | gain |
| chr8 | 495586 | 495967 | EAC,stomach | TDRP | gain |
| chr8 | 688267 | 688567 | EAC | - | gain |
| chr8 | 23562547 | 23562847 | ESCC | NKX2-6 | gain |
| chr8 | 23562974 | 23563274 | ESCC | NKX2-6 | gain |
| chr8 | 38964864 | 38965402 | EAC | ADAM32 | gain |
| chr8 | 41166957 | 41167257 | EAC | SFRP1 | gain |
| chr8 | 41753910 | 41754210 | EAC,stomach | ANK1 | gain |
| chr8 | 53851557 | 53851857 | EAC | NPBWR1 | gain |
| chr8 | 53851880 | 53852811 | EAC,stomach | NPBWR1 | gain |
| chr8 | 57069863 | 57070163 | EAC | - | gain |
| chr8 | 59058506 | 59058806 | EAC | FAM110B | gain |
| chr8 | 65281346 | 65281646 | stomach | MIR124-2HG | gain |
| chr8 | 65294493 | 65294793 | ESCC | MIR124-2HG | gain |
| chr8 | 67090394 | 67090604 | ESCC | CRH | gain |
| chr8 | 67344786 | 67345156 | EAC,stomach | RRS1-AS1,ADHFE1 | gain |
| chr8 | 72470701 | 72471000 | ESCC | - | gain |
| chr8 | 72754803 | 72755202 | EAC,stomach | MSC-AS1,MSC | gain |
| chr8 | 72756005 | 72756491 | stomach | MSC-AS1,MSC | gain |
| chr8 | 80523263 | 80523563 | ESCC | STMN2 | gain |
| chr8 | 99951601 | 99952500 | ESCC | OSR2,STK3 | gain |
| chr8 | 13205423 7 | 13205453 7 | ESCC | ADCY8 | gain |
| chr8 | 14359222 5 | 14359252 5 | EAC,stomach | ADGRB1 | gain |
| chr8 | 14510643 2 | 14510673 2 | EAC | OPLAH | gain |
| chr9 | 841700 | 842000 | stomach | DMRT1 | gain |
| chr9 | 16869582 | 16869882 | ESCC | BNC2 | gain |
| chr9 | 96714448 | 96714748 | ESCC | BARX1 | gain |
| chr9 | 11281025 2 | 11281055 2 | stomach | PALM2AKAP2 | gain |
| chr9 | 12677228 5 | 12677258 5 | ESCC | LHX2 | gain |
| chr9 | 12678478 9 | 12678508 9 | ESCC | LHX2 | gain |
| chr10 | 7451672 | 7451972 | EAC,stomach | SFMBT2 | gain |
| chr10 | 44788662 | 44788962 | EAC | LINC02881 | gain |
| chr10 | 48438574 | 48438874 | EAC | GDF10 | gain |
| chr10 | 63212357 | 63212657 | ESCC | TMEM26 | gain |
| chr10 | 81002068 | 81002630 | EAC | ZMIZ1 | gain |
| chr10 | 90342588 | 90343049 | EAC | LIPJ,RNLS | gain |
| chr10 | 94822758 | 94823058 | EAC,stomach | CYP26C1 | gain |
| chr10 | 11803082 0 | 11803112 0 | stomach | GFRA1 | gain |

(continued)

| chromosome | start | end | Cancer.type | gene | gain/loss |
|---|---|---|---|---|---|
| chr10 | 11889925 7 | 11889955 7 | EAC | VAX1 | gain |
| chr10 | 12953574 3 | 12953604 3 | EAC,stomach | FOXI2 | gain |
| chr10 | 13175818 1 | 13175848 1 | stomach | EBF3 | gain |
| chr10 | 13176737 6 | 13176767 6 | stomach | - | gain |
| chr10 | 13310971 7 | 13311001 7 | EAC,stomach | TCERG1L | gain |
| chr10 | 13311009 4 | 13311039 4 | EAC,stomach | TCERG1L | gain |
| chr11 | 2161742 | 2162333 | EAC,stomach | INS-IGF2,IGF2,IGF2-AS | gain |
| chr11 | 7272998 | 7273298 | EAC,stomach | SYT9 | gain |
| chr11 | 31826401 | 31826700 | ESCC | PAX6 | gain |
| chr11 | 31826934 | 31827234 | ESCC | PAX6 | gain |
| chr11 | 35105049 | 35105349 | EAC | - | gain |
| chr11 | 43602601 | 43602900 | ESCC | MIR129-2 | gain |
| chr11 | 57250319 | 57250619 | EAC | - | gain |
| chr11 | 70508260 | 70508560 | EAC | SHANK2 | gain |
| chr11 | 10548113 3 | 10548153 9 | stomach | GRIA4 | gain |
| chr11 | 12043490 6 | 12043520 6 | stomach | GRIK4 | gain |
| chr11 | 12330102 1 | 12330132 1 | EAC,stomach | - | gain |
| chr11 | 12856403 0 | 12856433 0 | stomach | FLI1,SENCR | gain |
| chr11 | 12856472 4 | 12856502 4 | stomach | FLI1,SENCR | gain |
| chr12 | 4918988 | 4919288 | stomach | KCNA6 | gain |
| chr12 | 22487697 | 22487997 | EAC | ST8SIA1 | gain |
| chr12 | 43944582 | 43944882 | ESCC | ADAMTS20 | gain |
| chr12 | 54345385 | 54345685 | ESCC | HOXC12 | gain |
| chr12 | 54359647 | 54359947 | EAC | HOTAIR | gain |
| chr12 | 54370332 | 54370632 | ESCC | HOTAIR | gain |
| chr12 | 54371108 | 54371408 | ESCC | HOTAIR | gain |
| chr12 | 54385154 | 54385454 | ESCC | MIR196A2 | gain |
| chr12 | 65217919 | 65218219 | stomach | TBC1D30 | gain |
| chr12 | 79258257 | 79258557 | stomach | SYT1 | gain |
| chr12 | 85306678 | 85306978 | EAC | SLC6A15 | gain |
| chr12 | 10321844 3 | 10321874 3 | ESCC | LINC00485 | gain |
| chr12 | 11487828 4 | 11487858 4 | ESCC | - | gain |
| chr12 | 11488269 6 | 11488299 6 | ESCC | - | gain |
| chr12 | 11510256 3 | 11510286 3 | stomach | - | gain |
| chr12 | 11510381 0 | 11510411 0 | stomach | - | gain |
| chr12 | 11510978 4 | 11511008 4 | stomach | TBX3 | gain |
| chr12 | 11513489 5 | 11513519 5 | ESCC | - | gain |
| chr12 | 13038806 6 | 13038836 6 | EAC | TMEM132D | gain |
| chr13 | 28498011 | 28498534 | EAC | PDX1 | gain |

(continued)

| chromosome | start | end | Cancer.type | gene | gain/loss |
|---|---|---|---|---|---|
| chr13 | 28498806 | 28499106 | EAC | PDX1 | gain |
| chr13 | 28501216 | 28501516 | EAC | - | gain |
| chr13 | 28503223 | 28503523 | EAC | - | gain |
| chr13 | 28674301 | 28674601 | stomach | FLT3 | gain |
| chr13 | 37005339 | 37005720 | EAC,stomach | CCNA1 | gain |
| chr13 | 37005913 | 37006490 | EAC,stomach | CCNA1 | gain |
| chr13 | 46425716 | 46426024 | stomach | SIAH3 | gain |
| chr13 | 58204074 | 58204374 | EAC,stomach | PCDH17 | gain |
| chr13 | 78493155 | 78493455 | EAC,stomach | OBI1-AS1,EDNRB | gain |
| chr13 | 79176422 | 79176722 | stomach | POU4F1,OBI1-AS1 | gain |
| chr13 | 84454161 | 84454461 | ESCC | SLITRK1 | gain |
| chr13 | 95355001 | 95355300 | ESCC | - | gain |
| chr13 | 10060797 3 | 10060827 3 | ESCC | - | gain |
| chr13 | 10062478 9 | 10062508 9 | ESCC | ZIC5 | gain |
| chr13 | 10062570 2 | 10062600 2 | ESCC | ZIC5 | gain |
| chr13 | 10064022 7 | 10064052 7 | ESCC | - | gain |
| chr13 | 10206884 6 | 10206914 6 | stomach | NALCN | gain |
| chr13 | 112720071 | 112720382 | ESCC | SOX1 | gain |
| chr14 | 29229689 | 29229989 | ESCC | - | gain |
| chr14 | 29236263 | 29236473 | EAC | FOXG1 | gain |
| chr14 | 36982295 | 36982595 | ESCC | SFTA3 | gain |
| chr14 | 37128275 | 37128575 | ESCC | PAX9 | gain |
| chr14 | 37129201 | 37129500 | ESCC | PAX9 | gain |
| chr14 | 38067055 | 38067445 | ESCC | FOXA1 | gain |
| chr14 | 38679482 | 38679782 | ESCC | SSTR1 | gain |
| chr14 | 51561143 | 51561443 | stomach | TRIM9 | gain |
| chr14 | 52735271 | 52735571 | EAC | PTGDR | gain |
| chr14 | 57276918 | 57277218 | ESCC | OTX2-AS1,OTX2 | gain |
| chr14 | 60951901 | 60952500 | ESCC | C14orf39 | gain |
| chr14 | 60973367 | 60973667 | stomach | SIX6 | gain |
| chr14 | 60976201 | 60976500 | ESCC | SIX6 | gain |
| chr14 | 61109705 | 61110106 | ESCC | - | gain |
| chr15 | 28351948 | 28352248 | EAC,stomach | - | gain |
| chr15 | 41793731 | 41794031 | EAC | ITPKA | gain |
| chr15 | 45409169 | 45409469 | stomach | DUOX2,DUOXA2 | gain |
| chr15 | 60287437 | 60287737 | ESCC | - | gain |
| chr15 | 65689002 | 65689302 | EAC | IGDCC4 | gain |
| chr15 | 86233064 | 86233370 | stomach | AKAP13 | gain |
| chr15 | 89922301 | 89922600 | ESCC | MIR9-3HG | gain |

(continued)

| chromosome | start | end | Cancer.type | gene | gain/loss |
|---|---|---|---|---|---|
| chr15 | 96887311 | 96887710 | stomach | - | gain |
| chr15 | 96887769 | 96888069 | stomach | - | gain |
| chr15 | 10151406 2 | 10151436 2 | ESCC | LRRK1 | gain |
| chr16 | 9107049 | 9107349 | stomach | - | gain |
| chr16 | 28074234 | 28074526 | EAC | GSG1L | gain |
| chr16 | 56696938 | 56697238 | EAC | - | gain |
| chr16 | 67196767 | 67197336 | ESCC,EAC | HSF4,TRADD,FBX L8 | gain |
| chr16 | 77822269 | 77822569 | stomach | VAT1L | gain |
| chr16 | 86541330 | 86541630 | stomach | FOXF1,FENDRR | gain |
| chr17 | 1880932 | 1881232 | ESCC | RTN4RL1 | gain |
| chr17 | 12877171 | 12877696 | EAC,stomach | ARHGAP44 | gain |
| chr17 | 36734761 | 36735061 | EAC | SRCIN1 | gain |
| chr17 | 43339347 | 43339647 | stomach | MAP3K14-AS1,SPATA32 | gain |
| chr17 | 46799870 | 46800170 | EAC,stomach | PRAC2,PRAC1 | gain |
| chr17 | 46919030 | 46919330 | ESCC | CALCOCO2 | gain |
| chr17 | 59534848 | 59535148 | EAC | TBX4 | gain |
| chr18 | 500829 | 501129 | EAC | COLEC12 | gain |
| chr18 | 4455187 | 4455487 | EAC | DLGAP1 | gain |
| chr18 | 5543398 | 5543698 | EAC,stomach | EPB41L3 | gain |
| chr18 | 11149285 | 11149620 | EAC,stomach | PIEZO2 | gain |
| chr18 | 14748135 | 14748435 | stomach | ANKRD30B | gain |
| chr18 | 19756552 | 19756852 | ESCC | GATA6 | gain |
| chr18 | 32847416 | 32847716 | EAC | ZSCAN30 | gain |
| chr18 | 55095027 | 55095327 | stomach | - | gain |
| chr18 | 74290778 | 74291078 | ESCC | - | gain |
| chr18 | 74962644 | 74962944 | EAC | GALR1 | gain |
| chr18 | 76739076 | 76739376 | EAC,stomach | SALL3 | gain |
| chr18 | 76739409 | 76739709 | stomach | SALL3 | gain |
| chr19 | 9473524 | 9473834 | stomach | ZNF177,ZNF559-ZNF177 | gain |
| chr19 | 13124934 | 13125234 | EAC | NFIX | gain |
| chr19 | 15580550 | 15580850 | stomach | PGLYRP2 | gain |
| chr19 | 17439058 | 17439358 | EAC | ANO8 | gain |
| chr19 | 17439727 | 17440027 | EAC | ANO8 | gain |
| chr19 | 21646179 | 21646479 | EAC | - | gain |
| chr19 | 22018808 | 22019108 | EAC | ZNF43 | gain |
| chr19 | 22444443 | 22444743 | stomach | - | gain |
| chr19 | 22817221 | 22817521 | EAC | ZNF492 | gain |
| chr19 | 31841405 | 31841705 | EAC | TSHZ3 | gain |
| chr19 | 31842723 | 31843023 | stomach | TSHZ3 | gain |

(continued)

| chromosome | start | end | Cancer.type | gene | gain/loss |
|---|---|---|---|---|---|
| chr19 | 36909263 | 36909568 | EAC,stomach | LOC644189,ZFP8 2 | gain |
| chr19 | 37096173 | 37096479 | stomach | ZNF529,ZNF382 | gain |
| chr19 | 37957845 | 37958147 | EAC | ZNF570,ZNF569 | gain |
| chr19 | 37958196 | 37958496 | EAC | ZNF570,ZNF569 | gain |
| chr19 | 48983792 | 48984221 | EAC,stomach | CYTH2 | gain |
| chr19 | 51111527 | 51111827 | stomach | - | gain |
| chr19 | 53038822 | 53039122 | EAC | ZNF808 | gain |
| chr19 | 56879421 | 56879795 | EAC | ZNF542P,ZSCAN 5A | gain |
| chr19 | 56904751 | 56905182 | EAC | ZNF582-AS1,ZNF582 | gain |
| chr19 | 57018919 | 57019219 | EAC,stomach | ZNF471 | gain |
| chr19 | 57049545 | 57049927 | EAC,stomach | ZFP28 | gain |
| chr19 | 57050470 | 57050770 | EAC | ZFP28 | gain |
| chr19 | 57078615 | 57078915 | EAC | ZNF470 | gain |
| chr19 | 58220101 | 58220700 | ESCC | ZNF154,ZNF776 | gain |
| chr19 | 58570269 | 58570569 | EAC,stomach | ZNF135 | gain |
| chr20 | 21694558 | 21694858 | ESCC | PAX1 | gain |
| chr20 | 25062104 | 25062597 | EAC | VSX1 | gain |
| chr20 | 34894498 | 34894798 | EAC,stomach | DLGAP4 | gain |
| chr20 | 37434079 | 37434379 | stomach | PPP1R16B | gain |
| chr20 | 41818206 | 41818508 | EAC | PTPRT | gain |
| chr20 | 61808738 | 61809038 | EAC | MIR124-3 | gain |
| chr21 | 34442210 | 34442527 | EAC | OLIG1 | gain |
| chr21 | 38075449 | 38075749 | ESCC | SIM2 | gain |
| chr21 | 38079541 | 38079841 | ESCC | SIM2 | gain |
| chr21 | 45336523 | 45336823 | stomach | AGPAT3 | gain |
| chr1 | 2425710 | 2426010 | stomach | PLCH2 | loss |
| chr1 | 3139807 | 3140017 | EAC | PRDM16 | loss |
| chr1 | 3210267 | 3210567 | stomach | PRDM16 | loss |
| chr1 | 5661736 | 5662036 | EAC | - | loss |
| chr1 | 19599366 | 19599666 | EAC | AKR7L | loss |
| chr1 | 21877426 | 21877726 | EAC | ALPL | loss |
| chr1 | 15740168 7 | 15740198 7 | ESCC | - | loss |
| chr1 | 16231942 5 | 16231972 5 | stomach | NOS1AP | loss |
| chr1 | 19932677 0 | 19932707 0 | ESCC | - | loss |
| chr1 | 24836624 9 | 24836654 9 | stomach | OR2M3 | loss |
| chr2 | 1879801 | 1880100 | ESCC | MYT1 L | loss |
| chr2 | 30496175 | 30496475 | stomach | - | loss |
| chr2 | 10032721 9 | 10032751 9 | stomach | AFF3 | loss |
| chr2 | 16965873 1 | 16965903 1 | EAC | NOSTRIN | loss |

(continued)

| chromosome | start | end | Cancer.type | gene | gain/loss |
|---|---|---|---|---|---|
| chr2 | 18906440 7 | 18906470 7 | stomach | - | loss |
| chr2 | 20161279 4 | 20161309 4 | EAC | AOX2P | loss |
| chr2 | 23818855 7 | 23818885 7 | EAC | - | loss |
| chr2 | 23857834 1 | 23857864 1 | EAC | LRRFIP1 | loss |
| chr2 | 23897287 4 | 23897317 4 | stomach | UBE2F-SCLY,SCLY | loss |
| chr2 | 240169011 | 240169311 | EAC | HDAC4 | loss |
| chr3 | 11178443 | 11178948 | stomach | HRH1 | loss |
| chr3 | 19987378 | 19987965 | EAC,stomach | RAB5A,EFHB | loss |
| chr3 | 61623149 | 61623449 | EAC | PTPRG | loss |
| chr3 | 15938684 2 | 15938714 2 | EAC | IQCJ-SCHIP1,SCHIP1 | loss |
| chr3 | 16040987 4 | 16041009 5 | ESCC | - | loss |
| chr3 | 16937496 0 | 16937526 0 | EAC | MECOM | loss |
| chr3 | 18000304 1 | 18000334 1 | ESCC | - | loss |
| chr3 | 18007687 1 | 18007717 1 | ESCC | - | loss |
| chr3 | 18036620 5 | 18036650 5 | ESCC | CCDC39 | loss |
| chr3 | 18097778 4 | 18097808 4 | ESCC | SOX2-OT | loss |
| chr3 | 19384003 1 | 19384033 1 | EAC | - | loss |
| chr3 | 19724760 1 | 19724790 1 | EAC | BDH1 | loss |
| chr4 | 7483501 | 7483800 | ESCC | SORCS2 | loss |
| chr4 | 54374099 | 54374399 | EAC | LNX1,PDGFRA,LN X1-AS1 | loss |
| chr5 | 15521632 | 15521932 | stomach | FBXL7 | loss |
| chr6 | 44698584 | 44698884 | EAC | - | loss |
| chr6 | 16625986 2 | 16626016 2 | stomach | - | loss |
| chr7 | 544375 | 544675 | EAC | PDGFA | loss |
| chr7 | 1303201 | 1303501 | EAC | - | loss |
| chr7 | 3221078 | 3221523 | stomach | - | loss |
| chr7 | 3234547 | 3234847 | EAC | - | loss |
| chr7 | 23749383 | 23749683 | EAC | STK31 | loss |
| chr7 | 27154502 | 27154712 | stomach | HOXA3 | loss |
| chr7 | 38726444 | 38726744 | ESCC | FAM183BP | loss |
| chr7 | 40566371 | 40566671 | stomach | SUGCT | loss |
| chr7 | 41026047 | 41026347 | EAC | - | loss |
| chr7 | 55145577 | 55145877 | EAC | EGFR | loss |
| chr7 | 73247463 | 73247763 | EAC,stomach | - | loss |
| chr7 | 73392547 | 73392847 | stomach | - | loss |
| chr7 | 97568604 | 97568814 | stomach | CCZ1P-OR7E38P | loss |
| chr7 | 99136949 | 99137159 | stomach | - | loss |
| chr7 | 14022712 3 | 14022742 3 | EAC | DENND2A | loss |
| chr7 | 15746790 1 | 15746820 0 | ESCC | PTPRN2 | loss |

(continued)

| chromosome | start | end | Cancer.type | gene | gain/loss |
|---|---|---|---|---|---|
| chr7 | 15756810 1 | 15756840 0 | ESCC | PTPRN2 | loss |
| chr8 | 2024099 | 2024399 | EAC | MYOM2 | loss |
| chr8 | 11839694 | 11839994 | stomach | DEFB135 | loss |
| chr8 | 13294962 | 13295262 | stomach | DLC1 | loss |
| chr8 | 23083321 | 23083680 | ESCC,stomach | TNFRSF10A,LOC3 89641 | loss |
| chr8 | 37217918 | 37218218 | EAC | - | loss |
| chr8 | 53517050 | 53517350 | ESCC | - | loss |
| chr8 | 64588078 | 64588378 | ESCC | - | loss |
| chr8 | 10598813 3 | 10598843 3 | stomach | - | loss |
| chr8 | 11098468 8 | 11098498 8 | ESCC | KCNV1 | loss |
| chr8 | 11758692 8 | 11758722 8 | stomach | - | loss |
| chr8 | 11862885 0 | 11862915 0 | EAC | - | loss |
| chr8 | 12068683 5 | 12068713 5 | stomach | - | loss |
| chr8 | 124729780 | 124730080 | ESCC | ANXA13 | loss |
| chr8 | 12473014 7 | 12473044 7 | ESCC | ANXA13 | loss |
| chr8 | 13934459 2 | 13934489 2 | ESCC | FAM135B | loss |
| chr8 | 14063003 0 | 14063033 0 | stomach | KCNK9 | loss |
| chr8 | 14434661 2 | 14434691 2 | stomach | GLI4,ZFP41 | loss |
| chr8 | 14434704 7 | 14434725 7 | stomach | GLI4,ZFP41 | loss |
| chr9 | 79625119 | 79625419 | stomach | - | loss |
| chr9 | 13709713 0 | 13709743 0 | stomach | - | loss |
| chr9 | 13963410 1 | 13963440 0 | ESCC | LCN10 | loss |
| chr10 | 696171 | 696482 | EAC | DIP2C-AS1,DIP2C | loss |
| chr10 | 778559 | 778859 | EAC,stomach | - | loss |
| chr10 | 3329816 | 3330116 | EAC,stomach | - | loss |
| chr10 | 31388486 | 31388786 | stomach | - | loss |
| chr10 | 44285701 | 44286000 | ESCC | HNRNPA3P1 | loss |
| chr10 | 77021717 | 77022017 | EAC | - | loss |
| chr10 | 95256767 | 95256977 | stomach | CEP55 | loss |
| chr10 | 13018530 1 | 13018560 0 | ESCC | - | loss |
| chr10 | 13458789 0 | 13458819 0 | EAC | INPP5A | loss |
| chr10 | 13473270 1 | 13473300 0 | ESCC | CFAP46 | loss |
| chr10 | 13473450 1 | 13473480 0 | ESCC | CFAP46 | loss |
| chr10 | 134834701 | 134835000 | ESCC | - | loss |
| chr10 | 13503040 4 | 13503070 4 | EAC | KNDC1 | loss |
| chr11 | 655429 | 655729 | EAC | DEAF1 | loss |
| chr11 | 1422301 | 1422600 | ESCC | BRSK2 | loss |
| chr11 | 5009161 | 5009461 | stomach | MMP26 | loss |
| chr11 | 12188846 | 12189146 | EAC,stomach | MICAL2 | loss |

(continued)

| chromosome | start | end | Cancer.type | gene | gain/loss |
|---|---|---|---|---|---|
| chr11 | 17562975 | 17563275 | stomach | USH1C | loss |
| chr11 | 64658576 | 64658876 | EAC | MIR192,MIR194-2 | loss |
| chr11 | 69218483 | 69218783 | EAC | - | loss |
| chr11 | 11115467 6 | 11115497 6 | EAC | C11orf53 | loss |
| chr12 | 53319067 | 53319367 | stomach | KRT8 | loss |
| chr13 | 11342610 0 | 11342642 0 | EAC | ATP11A | loss |
| chr13 | 11364873 2 | 11364903 2 | stomach | MCF2L | loss |
| chr13 | 11406485 8 | 11406515 8 | EAC | - | loss |
| chr13 | 11406570 1 | 11406600 0 | ESCC | - | loss |
| chr13 | 11476992 4 | 11477022 4 | EAC | RASA3 | loss |
| chr13 | 11491737 7 | 11491779 0 | EAC,stomach | - | loss |
| chr14 | 58599632 | 58599842 | EAC | ARMH4 | loss |
| chr14 | 65405781 | 65406081 | stomach | GPX2,CHURC1-FNTB | loss |
| chr14 | 10460040 1 | 10460070 0 | ESCC | KIF26A | loss |
| chr14 | 10519566 6 | 10519596 6 | stomach | ADSS1 | loss |
| chr15 | 22798836 | 22799136 | stomach | - | loss |
| chr15 | 90456774 | 90457074 | stomach | ARPIN-AP3S2,ARPIN | loss |
| chr16 | 552604 | 552904 | stomach | RAB11FIP3 | loss |
| chr16 | 7640302 | 7640512 | ESCC | RBFOX1 | loss |
| chr16 | 21245001 | 21245301 | EAC,stomach | ANKS4B | loss |
| chr16 | 30456175 | 30456475 | EAC,stomach | SEPHS2 | loss |
| chr16 | 33572254 | 33572563 | ESCC | - | loss |
| chr16 | 73266529 | 73266829 | stomach | - | loss |
| chr16 | 86653065 | 86653365 | EAC | - | loss |
| chr16 | 87823801 | 87824100 | ESCC | - | loss |
| chr17 | 30488733 | 30489033 | EAC | RHOT1 | loss |
| chr17 | 34328442 | 34328742 | stomach | CCL15,CCL15-CCL14 | loss |
| chr17 | 39059248 | 39059548 | stomach | - | loss |
| chr17 | 74911370 | 74911670 | EAC | MGAT5B | loss |
| chr17 | 77174101 | 77174400 | ESCC | RBFOX3 | loss |
| chr19 | 2278468 | 2278768 | EAC,stomach | PEAK3,SPPL2B | loss |
| chr19 | 42408308 | 42408608 | EAC | ARHGEF1 | loss |
| chr19 | 51128490 | 51128790 | EAC | SYT3 | loss |
| chr20 | 21588943 | 21589243 | EAC | - | loss |
| chr20 | 29960832 | 29961132 | stomach | DEFB118 | loss |
| chr20 | 31591997 | 31592297 | EAC | BPIFB2,SUN5 | loss |
| chr20 | 43029842 | 43030144 | stomach | HNF4A | loss |
| chr20 | 44876196 | 44876496 | EAC | CDH22 | loss |
| chr20 | 44881299 | 44881599 | EAC | CDH22 | loss |

(continued)

| chromosome | start | end | Cancer.type | gene | gain/loss |
|---|---|---|---|---|---|
| chr20 | 56134498 | 56134798 | stomach | PCK1 | loss |
| chr20 | 56272712 | 56273012 | EAC | PMEPA1 | loss |
| chr20 | 56273109 | 56273409 | stomach | PMEPA1 | loss |
| chr20 | 57616041 | 57616341 | stomach | SLMO2-ATP5E,PRELID3B | loss |
| chr20 | 59047338 | 59047638 | stomach | MIR646HG | loss |
| chr20 | 59832774 | 59833074 | EAC | CDH4 | loss |
| chr20 | 60445848 | 60446058 | EAC | CDH4 | loss |
| chr20 | 60470101 | 60470400 | ESCC | CDH4 | loss |
| chr20 | 62048701 | 62049000 | ESCC | KCNQ2 | loss |
| chr20 | 62121768 | 62122068 | EAC | EEF1A2 | loss |

The methylation score was calculated using the methylation value of the selected region and the performance was measured using the score. The methylation score was defined using the alpha value of the DNA fragment unit with the reads present in the hypermethylated and hypomethylated regions of esophageal and gastric cancer.

That is, the alpha value of the fragment is calculated as the methylation ratio of the CpG site present in the fragment. For example, when 9 out of 10 CpG sites present in a fragment are methylated, the alpha value of the fragment is 90%. The score was calculated using fragments present in the entire panel, and the relative frequency of fragments having an alpha value of 80% or higher in the hypermethylated region and 20% or lower in the hypomethylated region was calculated.

That is, the methylation score was calculated using the following Equation 1.

$$\text{Equation 1:}$$

$$\text{Methylation score} =$$

$$\frac{\begin{array}{c}\textit{Number of nucleic acid fragments with alpha value of hypermethylation marker of 80\% or more} + \\ \textit{Number of nucleic acid fragments with alpha value of hypomethylation marker of 20\% or less}\end{array}}{\textit{Total number of nucleic acid fragments}}$$

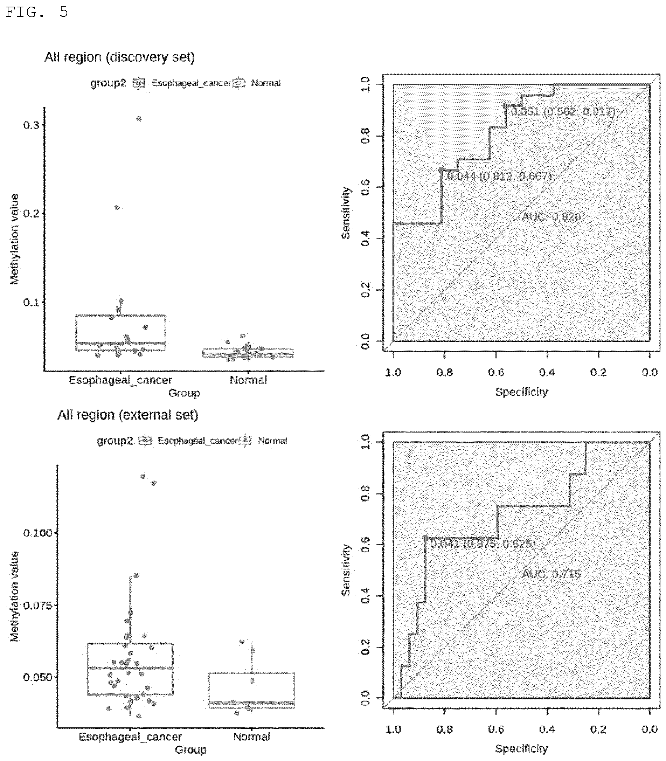

The cut-off value of the discovery set was determined as 0.044 and the cut-off value of the external set was determined as 0.041. Then, the methylation score distribution to distinguish between cancer and normal samples was determined. As a result, the performance of discovery set AUC 0.82 and external set AUC 0.715 was determined, as shown in FIG. 5.

8-3. Minimum marker selection and performance verification

Out of 497 regions, there were 345 hypermethylation regions, and among them, regions with a methylation beta value of 20 or less, an SD of 5 or less in normal and a median coverage of 100 or more in the discovery set were selected (region: 333/CpG sites: 7,427).

The list of the 333 hypermethylation regions selected is shown in Table 14 below.

[Table 14]

| chromosome | start | end | Cancer.type | gene | gain/loss |
|---|---|---|---|---|---|
| chr1 | 1475525 | 1475892 | EAC | TMEM240 | gain |
| chr1 | 32238510 | 32238810 | ESCC | - | gain |

(continued)

| chromosome | start | end | Cancer.type | gene | gain/ loss |
|---|---|---|---|---|---|
| chr1 | 33391630 | 33391930 | ESCC | - | gain |
| chr1 | 50893501 | 50893800 | ESCC | DMRTA2 | gain |
| chr1 | 62660474 | 62660774 | EAC,stomach | L1TD1 | gain |
| chr1 | 65991026 | 65991326 | EAC,stomach | LEPR | gain |
| chr1 | 77332988 | 77333288 | stomach | ST6GALNAC5 | gain |
| chr1 | 91192201 | 91192500 | ESCC | - | gain |
| chr1 | 107683625 | 107683925 | EAC,stomach | NTNG1 | gain |
| chr1 | 111217044 | 111217865 | EAC,stomach | KCNA3 | gain |
| chr1 | 119528280 | 119528580 | ESCC | TBX15 | gain |
| chr1 | 147790398 | 147790608 | stomach | NBPF8 | gain |
| chr1 | 165323295 | 165323595 | ESCC | LMX1A | gain |
| chr1 | 169396501 | 169397100 | ESCC | CCDC181 | gain |
| chr1 | 170630401 | 170630700 | ESCC | PRRX1 | gain |
| chr1 | 170634220 | 170634520 | ESCC | PRRX1 | gain |
| chr1 | 175568409 | 175568709 | stomach | TNR | gain |
| chr1 | 190447140 | 190447440 | stomach | BRINP3 | gain |
| chr1 | 197890594 | 197890894 | ESCC | LHX9 | gain |
| chr1 | 207843017 | 207843317 | ESCC | CR1L | gain |
| chr1 | 229569484 | 229569784 | ESCC | ACTA1 | gain |
| chr1 | 240255336 | 240255636 | EAC | FMN2 | gain |
| chr1 | 246952212 | 246952512 | stomach | LINC01 341 | gain |
| chr2 | 38301606 | 38301906 | stomach | CYP1B1 | gain |
| chr2 | 45155841 | 45156141 | EAC | - | gain |
| chr2 | 45160295 | 45160595 | stomach | - | gain |
| chr2 | 45172019 | 45172319 | stomach | SIX3 | gain |
| chr2 | 47797813 | 47798113 | stomach | KCNK12,MSH2 | gain |
| chr2 | 61371988 | 61372406 | stomach | C2orf74,LOC33980 3 | gain |
| chr2 | 63285504 | 63286100 | ESCC | - | gain |
| chr2 | 70995309 | 70995609 | EAC | ADD2 | gain |
| chr2 | 91634481 | 91634781 | ESCC | - | gain |
| chr2 | 105471810 | 105472110 | stomach | POU3F3,PANTR1 | gain |
| chr2 | 119599501 | 119599800 | ESCC | EN1 | gain |
| chr2 | 119609442 | 119609742 | stomach | EN1 | gain |
| chr2 | 119615841 | 119616141 | ESCC | - | gain |
| chr2 | 127413681 | 127413981 | stomach | GYPC | gain |
| chr2 | 145281792 | 145282092 | EAC | ZEB2 | gain |
| chr2 | 158300325 | 158300635 | stomach | CYTIP | gain |
| chr2 | 162280501 | 162280800 | ESCC | TBR1 | gain |
| chr2 | 172948817 | 172949117 | ESCC | DLX1 | gain |

(continued)

| chromosome | start | end | Cancer.type | gene | gain/ loss |
|---|---|---|---|---|---|
| chr2 | 172952136 | 172952436 | ESCC | DLX1 | gain |
| chr2 | 172953242 | 172953542 | ESCC | DLX1 | gain |
| chr2 | 176936286 | 176936586 | EAC,stomach | - | gain |
| chr2 | 176948093 | 176948393 | ESCC | EVX2 | gain |
| chr2 | 176956556 | 176956991 | ESCC | HOXD13 | gain |
| chr2 | 176979454 | 176979754 | ESCC | HOXD10 | gain |
| chr2 | 176985539 | 176985839 | ESCC | HOXD9 | gain |
| chr2 | 176985923 | 176986223 | ESCC | HOXD9 | gain |
| chr2 | 176989208 | 176989508 | ESCC | HOXD9 | gain |
| chr2 | 176993401 | 176993700 | ESCC | HOXD8 | gain |
| chr2 | 177003960 | 177004260 | ESCC | HOXD-AS2 | gain |
| chr2 | 177036438 | 177037106 | ESCC | HOXD3 | gain |
| chr2 | 177054423 | 177054723 | EAC,stomach | HAGLR,HOXD1 | gain |
| chr2 | 198651001 | 198651497 | ESCC,EAC | BOLL | gain |
| chr2 | 200327086 | 200327386 | ESCC | SATB2 | gain |
| chr2 | 208989098 | 208989398 | stomach | CRYGD,LOC100507 443 | gain |
| chr2 | 229044901 | 229045200 | ESCC | SPHKAP | gain |
| chr3 | 2140549 | 2140849 | EAC | CNTN4 | gain |
| chr3 | 42947540 | 42947840 | stomach | ZNF662 | gain |
| chr3 | 119528806 | 119529106 | EAC,stomach | NR1I2 | gain |
| chr3 | 137488469 | 137488769 | ESCC | - | gain |
| chr3 | 138655677 | 138655977 | ESCC | - | gain |
| chr3 | 142839841 | 142840141 | stomach | CHST2 | gain |
| chr3 | 147073801 | 147074100 | ESCC | - | gain |
| chr3 | 147108362 | 147108662 | stomach | ZIC4 | gain |
| chr3 | 147112080 | 147112200 | ESCC | ZIC4 | gain |
| chr3 | 147124023 | 147124567 | ESCC | ZIC1,ZIC4 | gain |
| chr3 | 147126068 | 147126278 | ESCC | ZIC1,ZIC4 | gain |
| chr3 | 147127429 | 147127729 | ESCC | ZIC4,ZIC1 | gain |
| chr3 | 147128306 | 147128606 | ESCC | ZIC4,ZIC1 | gain |
| chr3 | 147140764 | 147141064 | ESCC | - | gain |
| chr3 | 157812613 | 157812913 | ESCC | - | gain |
| chr3 | 157813172 | 157813500 | ESCC | - | gain |
| chr3 | 157821112 | 157821613 | ESCC | SHOX2 | gain |
| chr3 | 157821656 | 157821956 | ESCC | SHOX2 | gain |
| chr3 | 181441414 | 181441714 | ESCC | SOX2-OT | gain |
| chr3 | 184301089 | 184301618 | EAC | - | gain |
| chr4 | 1399085 | 1399385 | stomach | - | gain |
| chr4 | 3371726 | 3372026 | EAC | RGS12 | gain |

(continued)

| chromosome | start | end | Cancer.type | gene | gain/loss |
|---|---|---|---|---|---|
| chr4 | 4859071 | 4859281 | ESCC | MSX1 | gain |
| chr4 | 5710253 | 5710553 | EAC | EVC2,EVC | gain |
| chr4 | 13525572 | 13525872 | ESCC | - | gain |
| chr4 | 13530901 | 13531200 | ESCC | LINC01097 | gain |
| chr4 | 81185078 | 81185378 | ESCC | FGF5 | gain |
| chr4 | 111533196 | 111533650 | ESCC | - | gain |
| chr4 | 111535378 | 111535678 | stomach | - | gain |
| chr4 | 111539311 | 111539611 | ESCC | PITX2 | gain |
| chr4 | 111554497 | 111554797 | ESCC | PITX2 | gain |
| chr4 | 111560778 | 111561078 | ESCC | PITX2 | gain |
| chr4 | 111561787 | 111562112 | ESCC | PITX2 | gain |
| chr4 | 111562363 | 111562663 | ESCC | PITX2 | gain |
| chr4 | 174449490 | 174449790 | ESCC | HAND2-AS1,HAND2 | gain |
| chr4 | 174452311 | 174452611 | ESCC | HAND2,HAND2-AS1 | gain |
| chr5 | 1883804 | 1884104 | EAC | IRX4 | gain |
| chr5 | 5139851 | 5140151 | EAC,stomach | ADAMTS16 | gain |
| chr5 | 42424375 | 42424675 | EAC | GHR | gain |
| chr5 | 42995219 | 42995677 | stomach | - | gain |
| chr5 | 87955633 | 87955933 | ESCC | LINC00461 | gain |
| chr5 | 118609317 | 118609617 | stomach | TNFAIP8 | gain |
| chr5 | 122430284 | 122430584 | stomach | PRDM6 | gain |
| chr5 | 136834314 | 136834642 | EAC | SPOCK1 | gain |
| chr5 | 140793318 | 140793618 | ESCC | PCDHGB7,PCDHGA 10,PCDHGA1,PCDH GA2,PCDHGA3,PCD HGB1,PCDHGA4,PC DHGB2,PCDHGA5,P CDHGB3,PCDHGA6, PCDHGA7,PCDHGB 4,PCDHGA8,PCDHG B5,PCDHGA9,PCDH GB6 | gain |
| chr5 | 140864757 | 140865057 | ESCC | PCDHGC5,PCDHGC 4,PCDHGA1,PCDHG A2,PCDHGA3,PCDH GB1,PCDHGA4,PCD HGB2,PCDHGA5,PC DHGB3,PCDHGA6,P CDHGA7,PCDHGB4, PCDHGA8,PCDHGB 5,PCDHGA9,PCDHG B6,PCDHGA10,PCD HGB7,PCDHGA11,P CDHGA12,PCDHGC 3 | gain |
| chr5 | 153853518 | 153853818 | ESCC | - | gain |
| chr5 | 153862521 | 153862821 | ESCC | HAND1 | gain |

(continued)

| chromosome | start | end | Cancer.type | gene | gain/loss |
|---|---|---|---|---|---|
| chr5 | 178003941 | 178004354 | EAC,stomach | COL23A1 | gain |
| chr5 | 178367677 | 178367977 | stomach | ZNF454 | gain |
| chr5 | 178421978 | 178422278 | EAC,stomach | GRM6 | gain |
| chr6 | 391593 | 392086 | stomach | IRF4 | gain |
| chr6 | 393089 | 393389 | EAC,stomach | IRF4 | gain |
| chr6 | 1385345 | 1385645 | ESCC | FOXF2 | gain |
| chr6 | 1393425 | 1393725 | ESCC | FOXF2 | gain |
| chr6 | 10393848 | 10394148 | ESCC | - | gain |
| chr6 | 26614501 | 26614800 | ESCC | - | gain |
| chr6 | 27258856 | 27259156 | ESCC | - | gain |
| chr6 | 27526069 | 27526369 | ESCC | - | gain |
| chr6 | 28414801 | 28415100 | ESCC | ZSCAN23 | gain |
| chr6 | 58142486 | 58142786 | ESCC | - | gain |
| chr6 | 58147218 | 58147750 | ESCC | - | gain |
| chr6 | 58148830 | 58149133 | ESCC | - | gain |
| chr6 | 73330964 | 73331264 | stomach | KCNQ5 | gain |
| chr6 | 88876591 | 88876891 | EAC,stomach | CNR1 | gain |
| chr6 | 100901902 | 100902202 | ESCC | SIM1 | gain |
| chr6 | 100911594 | 100911894 | ESCC | SIM1 | gain |
| chr6 | 100915705 | 100916005 | ESCC | SIM1 | gain |
| chr6 | 110679416 | 110679716 | EAC | M ETTL24 | gain |
| chr6 | 133562342 | 133562642 | stomach | EYA4 | gain |
| chr6 | 161100092 | 161100392 | stomach | - | gain |
| chr6 | 166218369 | 166218669 | stomach | - | gain |
| chr7 | 15727291 | 15727591 | stomach | MEOX2 | gain |
| chr7 | 19146001 | 19146300 | ESCC | - | gain |
| chr7 | 19157043 | 19157413 | stomach | TWIST1 | gain |
| chr7 | 24323790 | 24324089 | stomach | NPY | gain |
| chr7 | 24324301 | 24324600 | ESCC | NPY | gain |
| chr7 | 24324826 | 24325126 | EAC,stomach | NPY | gain |
| chr7 | 27225169 | 27225469 | ESCC | HOXA11,HOXA11-AS | gain |
| chr7 | 27284490 | 27284923 | ESCC | EVX1 | gain |
| chr7 | 35298241 | 35298541 | stomach | TBX20 | gain |
| chr7 | 42267948 | 42268259 | ESCC | GLI3 | gain |
| chr7 | 49812883 | 49813261 | EAC | VWC2 | gain |
| chr7 | 84814533 | 84814833 | EAC,stomach | SEMA3D | gain |
| chr7 | 96642301 | 96642600 | ESCC | DLX6-AS1 | gain |
| chr7 | 96646967 | 96647438 | ESCC,stomach | DLX6-AS1 | gain |

(continued)

| chromosome | start | end | Cancer.type | gene | gain/loss |
|---|---|---|---|---|---|
| chr7 | 96655294 | 96655594 | stomach | DLX5 | gain |
| chr7 | 114562697 | 114562997 | stomach | MDFIC | gain |
| chr7 | 121946273 | 121946573 | ESCC | FEZF1,FEZF1-AS1 | gain |
| chr7 | 127672308 | 127672714 | EAC | LRRC4,SND1 | gain |
| chr7 | 127841686 | 127841986 | ESCC | - | gain |
| chr7 | 142494835 | 142495135 | EAC,stomach | - | gain |
| chr7 | 149389779 | 149389989 | stomach | - | gain |
| chr7 | 149917113 | 149917413 | EAC | - | gain |
| chr7 | 151107107 | 151107435 | EAC,stomach | WDR86,WDR86-AS1 | gain |
| chr7 | 158937819 | 158938201 | EAC,stomach | VIPR2 | gain |
| chr8 | 495586 | 495967 | EAC,stomach | TDRP | gain |
| chr8 | 688267 | 688567 | EAC | - | gain |
| chr8 | 23562547 | 23562847 | ESCC | NKX2-6 | gain |
| chr8 | 23562974 | 23563274 | ESCC | NKX2-6 | gain |
| chr8 | 38964864 | 38965402 | EAC | ADAM32 | gain |
| chr8 | 41166957 | 41167257 | EAC | SFRP1 | gain |
| chr8 | 41753910 | 41754210 | EAC,stomach | ANK1 | gain |
| chr8 | 53851557 | 53851857 | EAC | NPBWR1 | gain |
| chr8 | 53851880 | 53852811 | EAC,stomach | NPBWR1 | gain |
| chr8 | 57069863 | 57070163 | EAC | - | gain |
| chr8 | 59058506 | 59058806 | EAC | FAM110B | gain |
| chr8 | 65281346 | 65281646 | stomach | MIR124-2HG | gain |
| chr8 | 65294493 | 65294793 | ESCC | MIR124-2HG | gain |
| chr8 | 67090394 | 67090604 | ESCC | CRH | gain |
| chr8 | 67344786 | 67345156 | EAC,stomach | RRS1-AS1,ADHFE1 | gain |
| chr8 | 72470701 | 72471000 | ESCC | - | gain |
| chr8 | 72754803 | 72755202 | EAC,stomach | MSC-AS1,MSC | gain |
| chr8 | 72756005 | 72756491 | stomach | MSC-AS1,MSC | gain |
| chr8 | 80523263 | 80523563 | ESCC | STMN2 | gain |
| chr8 | 99951601 | 99952500 | ESCC | OSR2,STK3 | gain |
| chr8 | 132054237 | 132054537 | ESCC | ADCY8 | gain |
| chr8 | 143592225 | 143592525 | EAC,stomach | ADGRB1 | gain |
| chr8 | 145106432 | 145106732 | EAC | OPLAH | gain |
| chr9 | 841700 | 842000 | stomach | DMRT1 | gain |
| chr9 | 16869582 | 16869882 | ESCC | BNC2 | gain |
| chr9 | 96714448 | 96714748 | ESCC | BARX1 | gain |
| chr9 | 112810252 | 112810552 | stomach | PALM2AKAP2 | gain |
| chr9 | 126772285 | 126772585 | ESCC | LHX2 | gain |
| chr9 | 126784789 | 126785089 | ESCC | LHX2 | gain |

(continued)

| chromosome | start | end | Cancer.type | gene | gain/loss |
|---|---|---|---|---|---|
| chr10 | 7451672 | 7451972 | EAC,stomach | SFMBT2 | gain |
| chr10 | 44788662 | 44788962 | EAC | LINC02881 | gain |
| chr10 | 48438574 | 48438874 | EAC | GDF10 | gain |
| chr10 | 63212357 | 63212657 | ESCC | TMEM26 | gain |
| chr10 | 81002068 | 81002630 | EAC | ZMIZ1 | gain |
| chr10 | 90342588 | 90343049 | EAC | LIPJ,RNLS | gain |
| chr10 | 94822758 | 94823058 | EAC,stomach | CYP26C1 | gain |
| chr10 | 118030820 | 118031120 | stomach | GFRA1 | gain |
| chr10 | 118899257 | 118899557 | EAC | VAX1 | gain |
| chr10 | 129535743 | 129536043 | EAC,stomach | FOXI2 | gain |
| chr10 | 131758181 | 131758481 | stomach | EBF3 | gain |
| chr10 | 131767376 | 131767676 | stomach | - | gain |
| chr10 | 133109717 | 133110017 | EAC,stomach | TCERG1L | gain |
| chr10 | 133110094 | 133110394 | EAC,stomach | TCERG1L | gain |
| chr11 | 2161742 | 2162333 | EAC,stomach | INS-IGF2,IGF2,IGF2-AS | gain |
| chr11 | 7272998 | 7273298 | EAC,stomach | SYT9 | gain |
| chr11 | 31826401 | 31826700 | ESCC | PAX6 | gain |
| chr11 | 31826934 | 31827234 | ESCC | PAX6 | gain |
| chr11 | 35105049 | 35105349 | EAC | - | gain |
| chr11 | 43602601 | 43602900 | ESCC | MIR129-2 | gain |
| chr11 | 57250319 | 57250619 | EAC | - | gain |
| chr11 | 70508260 | 70508560 | EAC | SHANK2 | gain |
| chr11 | 105481133 | 105481539 | stomach | GRIA4 | gain |
| chr11 | 120434906 | 120435206 | stomach | GRIK4 | gain |
| chr11 | 123301021 | 123301321 | EAC,stomach | - | gain |
| chr11 | 128564030 | 128564330 | stomach | FLI1,SENCR | gain |
| chr11 | 128564724 | 128565024 | stomach | FLI1,SENCR | gain |
| chr12 | 4918988 | 4919288 | stomach | KCNA6 | gain |
| chr12 | 22487697 | 22487997 | EAC | ST8SIA1 | gain |
| chr12 | 43944582 | 43944882 | ESCC | ADAMTS20 | gain |
| chr12 | 54345385 | 54345685 | ESCC | HOXC12 | gain |
| chr12 | 54359647 | 54359947 | EAC | HOTAIR | gain |
| chr12 | 54370332 | 54370632 | ESCC | HOTAIR | gain |
| chr12 | 54371108 | 54371408 | ESCC | HOTAIR | gain |
| chr12 | 54385154 | 54385454 | ESCC | MIR196A2 | gain |
| chr12 | 65217919 | 65218219 | stomach | TBC1D30 | gain |
| chr12 | 79258257 | 79258557 | stomach | SYT1 | gain |
| chr12 | 85306678 | 85306978 | EAC | SLC6A15 | gain |
| chr12 | 103218443 | 103218743 | ESCC | LINC00485 | gain |

(continued)

| chromosome | start | end | Cancer.type | gene | gain/ loss |
|---|---|---|---|---|---|
| chr12 | 114878284 | 114878584 | ESCC | - | gain |
| chr12 | 114882696 | 114882996 | ESCC | - | gain |
| chr12 | 115102563 | 115102863 | stomach | - | gain |
| chr12 | 115103810 | 115104110 | stomach | - | gain |
| chr12 | 115109784 | 115110084 | stomach | TBX3 | gain |
| chr12 | 115134895 | 115135195 | ESCC | - | gain |
| chr12 | 130388066 | 130388366 | EAC | TMEM132D | gain |
| chr13 | 28498011 | 28498534 | EAC | PDX1 | gain |
| chr13 | 28498806 | 28499106 | EAC | PDX1 | gain |
| chr13 | 28501216 | 28501516 | EAC | - | gain |
| chr13 | 28503223 | 28503523 | EAC | - | gain |
| chr13 | 28674301 | 28674601 | stomach | FLT3 | gain |
| chr13 | 37005339 | 37005720 | EAC,stomach | CCNA1 | gain |
| chr13 | 37005913 | 37006490 | EAC,stomach | CCNA1 | gain |
| chr13 | 46425716 | 46426024 | stomach | SIAH3 | gain |
| chr13 | 58204074 | 58204374 | EAC,stomach | PCDH17 | gain |
| chr13 | 78493155 | 78493455 | EAC,stomach | OBI1-AS1,EDNRB | gain |
| chr13 | 79176422 | 79176722 | stomach | POU4F1,OBI1-AS1 | gain |
| chr13 | 84454161 | 84454461 | ESCC | SLITRK1 | gain |
| chr13 | 95355001 | 95355300 | ESCC | - | gain |
| chr13 | 100607973 | 100608273 | ESCC | - | gain |
| chr13 | 100624789 | 100625089 | ESCC | ZIC5 | gain |
| chr13 | 100625702 | 100626002 | ESCC | ZIC5 | gain |
| chr13 | 100640227 | 100640527 | ESCC | - | gain |
| chr13 | 102068846 | 102069146 | stomach | NALCN | gain |
| chr13 | 112720071 | 112720382 | ESCC | SOX1 | gain |
| chr14 | 29229689 | 29229989 | ESCC | - | gain |
| chr14 | 29236263 | 29236473 | EAC | FOXG1 | gain |
| chr14 | 36982295 | 36982595 | ESCC | SFTA3 | gain |
| chr14 | 37128275 | 37128575 | ESCC | PAX9 | gain |
| chr14 | 37129201 | 37129500 | ESCC | PAX9 | gain |
| chr14 | 38067055 | 38067445 | ESCC | FOXA1 | gain |
| chr14 | 38679482 | 38679782 | ESCC | SSTR1 | gain |
| chr14 | 51561143 | 51561443 | stomach | TRIM9 | gain |
| chr14 | 52735271 | 52735571 | EAC | PTGDR | gain |
| chr14 | 57276918 | 57277218 | ESCC | OTX2-AS1,OTX2 | gain |
| chr14 | 60951901 | 60952500 | ESCC | C14orf39 | gain |
| chr14 | 60973367 | 60973667 | stomach | SIX6 | gain |
| chr14 | 60976201 | 60976500 | ESCC | SIX6 | gain |

(continued)

| chromosome | start | end | Cancer.type | gene | gain/loss |
|---|---|---|---|---|---|
| chr14 | 61109705 | 61110106 | ESCC | - | gain |
| chr15 | 28351948 | 28352248 | EAC,stomach | - | gain |
| chr15 | 41793731 | 41794031 | EAC | ITPKA | gain |
| chr15 | 45409169 | 45409469 | stomach | DUOX2,DUOXA2 | gain |
| chr15 | 60287437 | 60287737 | ESCC | - | gain |
| chr15 | 65689002 | 65689302 | EAC | IGDCC4 | gain |
| chr15 | 86233064 | 86233370 | stomach | AKAP13 | gain |
| chr15 | 89922301 | 89922600 | ESCC | MIR9-3HG | gain |
| chr15 | 96887311 | 96887710 | stomach | - | gain |
| chr15 | 96887769 | 96888069 | stomach | - | gain |
| chr15 | 101514062 | 101514362 | ESCC | LRRK1 | gain |
| chr16 | 9107049 | 9107349 | stomach | - | gain |
| chr16 | 28074234 | 28074526 | EAC | GSG1L | gain |
| chr16 | 56696938 | 56697238 | EAC | - | gain |
| chr16 | 67196767 | 67197336 | ESCC,EAC | HSF4,TRADD,FBXL8 | gain |
| chr16 | 77822269 | 77822569 | stomach | VAT1 L | gain |
| chr16 | 86541330 | 86541630 | stomach | FOXF1,FENDRR | gain |
| chr17 | 1880932 | 1881232 | ESCC | RTN4RL1 | gain |
| chr17 | 12877171 | 12877696 | EAC,stomach | ARHGAP44 | gain |
| chr17 | 36734761 | 36735061 | EAC | SRCIN1 | gain |
| chr17 | 43339347 | 43339647 | stomach | MAP3K14-AS1,SPATA32 | gain |
| chr17 | 46799870 | 46800170 | EAC,stomach | PRAC2,PRAC1 | gain |
| chr17 | 46919030 | 46919330 | ESCC | CALCOCO2 | gain |
| chr17 | 59534848 | 59535148 | EAC | TBX4 | gain |
| chr18 | 500829 | 501129 | EAC | COLEC12 | gain |
| chr18 | 4455187 | 4455487 | EAC | DLGAP1 | gain |
| chr18 | 5543398 | 5543698 | EAC,stomach | EPB41L3 | gain |
| chr18 | 11149285 | 11149620 | EAC,stomach | PIEZO2 | gain |
| chr18 | 14748135 | 14748435 | stomach | ANKRD30B | gain |
| chr18 | 19756552 | 19756852 | ESCC | GATA6 | gain |
| chr18 | 32847416 | 32847716 | EAC | ZSCAN30 | gain |
| chr18 | 55095027 | 55095327 | stomach | - | gain |
| chr18 | 74290778 | 74291078 | ESCC | - | gain |
| chr18 | 74962644 | 74962944 | EAC | GALR1 | gain |
| chr18 | 76739076 | 76739376 | EAC,stomach | SALL3 | gain |
| chr18 | 76739409 | 76739709 | stomach | SALL3 | gain |
| chr19 | 9473524 | 9473834 | stomach | ZNF177,ZNF559-ZNF177 | gain |
| chr19 | 13124934 | 13125234 | EAC | NFIX | gain |
| chr19 | 15580550 | 15580850 | stomach | PGLYRP2 | gain |

(continued)

| chromosome | start | end | Cancer.type | gene | gain/loss |
|---|---|---|---|---|---|
| chr19 | 17439058 | 17439358 | EAC | ANO8 | gain |
| chr19 | 17439727 | 17440027 | EAC | ANO8 | gain |
| chr19 | 21646179 | 21646479 | EAC | - | gain |
| chr19 | 22018808 | 22019108 | EAC | ZNF43 | gain |
| chr19 | 22444443 | 22444743 | stomach | - | gain |
| chr19 | 22817221 | 22817521 | EAC | ZN F492 | gain |
| chr19 | 31841405 | 31841705 | EAC | TSHZ3 | gain |
| chr19 | 31842723 | 31843023 | stomach | TSHZ3 | gain |
| chr19 | 36909263 | 36909568 | EAC,stomach | LOC644189,ZFP82 | gain |
| chr19 | 37096173 | 37096479 | stomach | ZNF529,ZNF382 | gain |
| chr19 | 37957845 | 37958147 | EAC | ZNF570,ZNF569 | gain |
| chr19 | 37958196 | 37958496 | EAC | ZNF570,ZNF569 | gain |
| chr19 | 48983792 | 48984221 | EAC,stomach | CYTH2 | gain |
| chr19 | 51111527 | 51111827 | stomach | - | gain |
| chr19 | 53038822 | 53039122 | EAC | ZNF808 | gain |
| chr19 | 56879421 | 56879795 | EAC | ZNF542P,ZSCAN5A | gain |
| chr19 | 56904751 | 56905182 | EAC | ZNF582-AS1,ZNF582 | gain |
| chr19 | 57018919 | 57019219 | EAC,stomach | ZNF471 | gain |
| chr19 | 57049545 | 57049927 | EAC,stomach | ZFP28 | gain |
| chr19 | 57050470 | 57050770 | EAC | ZFP28 | gain |
| chr19 | 57078615 | 57078915 | EAC | ZNF470 | gain |
| chr19 | 58220101 | 58220700 | ESCC | ZNF154,ZNF776 | gain |
| chr19 | 58570269 | 58570569 | EAC,stomach | ZNF135 | gain |
| chr20 | 21694558 | 21694858 | ESCC | PAX1 | gain |
| chr20 | 25062104 | 25062597 | EAC | VSX1 | gain |
| chr20 | 34894498 | 34894798 | EAC,stomach | DLGAP4 | gain |
| chr20 | 37434079 | 37434379 | stomach | PPP1R16B | gain |
| chr20 | 41818206 | 41818508 | EAC | PTPRT | gain |
| chr20 | 61808738 | 61809038 | EAC | MIR124-3 | gain |
| chr21 | 34442210 | 34442527 | EAC | OLIG1 | gain |
| chr21 | 38075449 | 38075749 | ESCC | SIM2 | gain |
| chr21 | 38079541 | 38079841 | ESCC | SIM2 | gain |
| chr21 | 45336523 | 45336823 | stomach | AGPAT3 | gain |

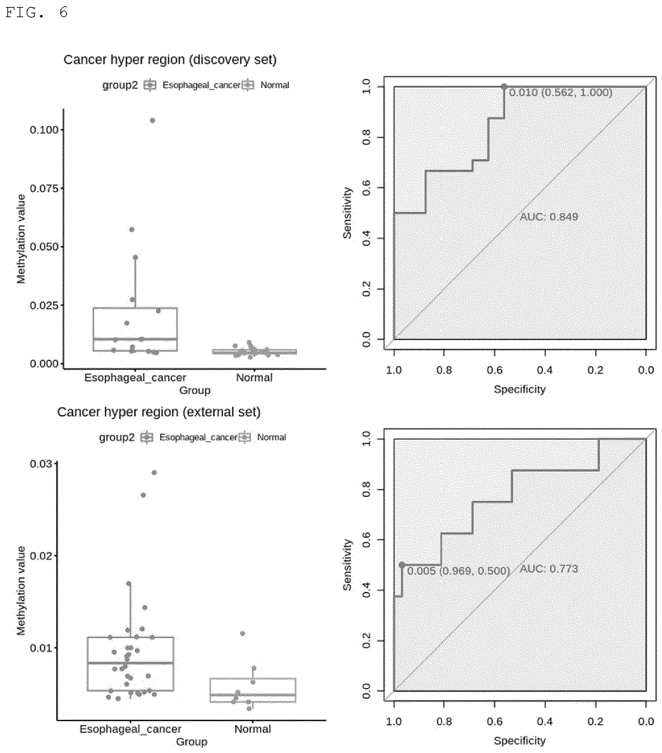

The alpha value was calculated from fragments present in the hypermethylated region, and the relative frequency of fragments with an alpha value of 80% or higher was calculated: number of fragments 80% or higher/total number of fragments. The area under the curve (AUC) value was calculated to determine how well the ESCC patients and normal groups were distinguished using the calculated score. As a result, as shown in FIG. 6, the performance of AUC 0.85 in the discovery set and of AUC 0.77 in the external set was determined. At this time, the cut-off value of the discovery set was 0.01 and the cut-off value of the external set was 0.005.

In addition, among the cancer hypermethylation CpG sites present in the panel, the CpG site having the highest difference between cancer and normal samples was selected using the discovery set. In other words, five areas that have an AUC to distinguish the cancer sample from normal sample, higher than 0.8, and a median difference value higher than 0.05 between the cancer and normal groups were selected as minimum markers using the beta value of each CpG site

The list is shown in Table 15 below.

[Table 15]

| chromosome | start | end | Cancer.type | gene | Gain/loss |
|---|---|---|---|---|---|
| chr2 | 200327093 | 200327094 | ESCC | SATB2 | gain |
| chr3 | 157812614 | 157812615 | ESCC | - | gain |
| chr3 | 157812620 | 157812621 | ESCC | - | gain |
| chr3 | 157812651 | 157812652 | ESCC | - | gain |
| chr14 | 38679763 | 38679764 | ESCC | SSTR1 | gain |

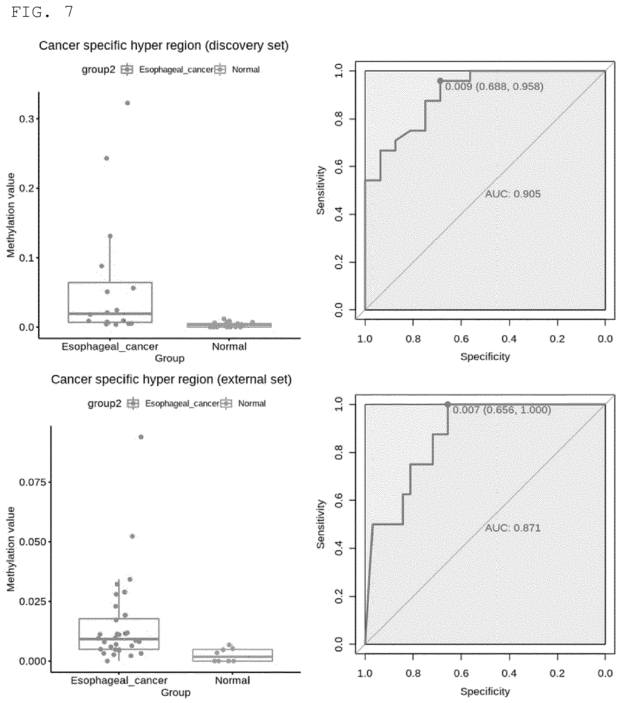

The methylation score was calculated with fragments present in the five hypermethylated regions. That is, the alpha value was calculated with the fragments present in the hypermethylated regions, and the relative frequency of fragments having an alpha value of 80% or more was calculated: number of fragments 80% or more / total number of fragments. The area under the curve (AUC) value was calculated to determine how well the ESCC patients and normal groups were distinguished using the calculated score. As a result, as shown in FIG. 7, the performance of AUC 0.91 in the discovery set and AUC 0.87 in the external set was determined. At this time, the cut-off value of the discovery set was 0.009 and the cut-off value of the external set was 0.007.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

[Industrial Applicability]

The DNA methylation marker for diagnosing esophageal cancer and gastric cancer according to the present invention is capable of diagnosing esophageal cancer and gastric cancer with high accuracy using only DNA methylation information of a blood sample without using esophageal cancer and gastric cancer tissue samples, and thus is useful for the early diagnosis of esophageal cancer and gastric cancer.

**Claims**

**1.**  A DNA methylation marker combination for diagnosing esophageal cancer and gastric cancer comprising DNA methylation markers shown in Table 1:

[Table 1]

| Chromosome | Start point | End point |
|---|---|---|
| chr2 | 200327093 | 200327094 |
| chr3 | 157812614 | 157812615 |
| chr3 | 157812620 | 157812621 |
| chr3 | 157812651 | 157812652 |
| chr14 | 38679763 | 38679764 |

**2.**  The DNA methylation marker according to claim 1, wherein the DNA methylation marker combination further comprises DNA markers shown in Table 2 below:

[Table 2]

| Chromosome | Start point | End point | Chromosome | Start point | End point |
|---|---|---|---|---|---|
| chr1 | 1475525 | 1475892 | chr8 | 67090394 | 67090604 |

(continued)

| Chromosome | Start point | End point | Chromosome | Start point | End point |
|---|---|---|---|---|---|
| chr1 | 32238510 | 32238810 | chr8 | 67344786 | 67345156 |
| chr1 | 33391630 | 33391930 | chr8 | 72470701 | 72471,000 |
| chr1 | 50893501 | 50893800 | chr8 | 72754803 | 72755202 |
| chr1 | 62660474 | 62660774 | chr8 | 72756005 | 72756491 |
| chr1 | 65991026 | 65991326 | chr8 | 80523263 | 80523563 |
| chr1 | 77332988 | 77333288 | chr8 | 99951601 | 99952500 |
| chr1 | 91192201 | 91192500 | chr8 | 132054237 | 132054537 |
| chr1 | 107683625 | 107683925 | chr8 | 143592225 | 143592525 |
| chr1 | 111217044 | 111217865 | chr8 | 145106432 | 145106732 |
| chr1 | 119528280 | 119528580 | chr9 | 841700 | 842000 |
| chr1 | 147790398 | 147790608 | chr9 | 16869582 | 16869882 |
| chr1 | 165323295 | 165323595 | chr9 | 96714448 | 96714748 |
| chr1 | 169396501 | 169397100 | chr9 | 112810252 | 112810552 |
| chr1 | 170630401 | 170630700 | chr9 | 126772285 | 126772585 |
| chr1 | 170634220 | 170634520 | chr9 | 126784789 | 126785089 |
| chr1 | 175568409 | 175568709 | chr10 | 7451672 | 7451972 |
| chr1 | 190447140 | 190447440 | chr10 | 44788662 | 44788962 |
| chr1 | 197890594 | 197890894 | chr10 | 48438574 | 48438874 |
| chr1 | 207843017 | 207843317 | chr10 | 63212357 | 63212657 |
| chr1 | 229569484 | 229569784 | chr10 | 81002068 | 81002630 |
| chr1 | 240255336 | 240255636 | chr10 | 90342588 | 90343049 |
| chr1 | 246952212 | 246952512 | chr10 | 94822758 | 94823058 |
| chr2 | 38301606 | 38301906 | chr10 | 118030820 | 118031120 |
| chr2 | 45155841 | 45156141 | chr10 | 118899257 | 118899557 |
| chr2 | 45160295 | 45160595 | chr10 | 129535743 | 129536043 |
| chr2 | 45172019 | 45172319 | chr10 | 131758181 | 131758481 |
| chr2 | 47797813 | 47798113 | chr10 | 131767376 | 131767676 |
| chr2 | 61371988 | 61372406 | chr10 | 133109717 | 133110017 |
| chr2 | 63285504 | 63286100 | chr10 | 133110094 | 133110394 |
| chr2 | 70995309 | 70995609 | chr11 | 2161742 | 2162333 |
| chr2 | 91634481 | 91634781 | chr11 | 7272998 | 7273298 |
| chr2 | 105471810 | 105472110 | chr11 | 31826401 | 31826700 |
| chr2 | 119599501 | 119599800 | chr11 | 31826934 | 31827234 |
| chr2 | 119609442 | 119609742 | chr11 | 35105049 | 35105349 |
| chr2 | 119615841 | 119616141 | chr11 | 43602601 | 43602900 |
| chr2 | 127413681 | 127413981 | chr11 | 57250319 | 57250619 |
| chr2 | 145281792 | 145282092 | chr11 | 70508260 | 70508560 |
| chr2 | 158300325 | 158300635 | chr11 | 105481133 | 105481539 |
| chr2 | 162280501 | 162280800 | chr11 | 120434906 | 120435206 |

(continued)

| Chromosome | Start point | End point | Chromosome | Start point | End point |
|---|---|---|---|---|---|
| chr2 | 172948817 | 172949117 | chr11 | 123301021 | 123301321 |
| chr2 | 172952136 | 172952436 | chr11 | 128564030 | 128564330 |
| chr2 | 172953242 | 172953542 | chr11 | 128564724 | 128565024 |
| chr2 | 176936286 | 176936586 | chr12 | 4918988 | 4919288 |
| chr2 | 176948093 | 176948393 | chr12 | 22487697 | 22487997 |
| chr2 | 176956556 | 176956991 | chr12 | 43944582 | 43944882 |
| chr2 | 176979454 | 176979754 | chr12 | 54345385 | 54345685 |
| chr2 | 176985539 | 176985839 | chr12 | 54359647 | 54359947 |
| chr2 | 176985923 | 176986223 | chr12 | 54370332 | 54370632 |
| chr2 | 176989208 | 176989508 | chr12 | 54371108 | 54371408 |
| chr2 | 176993401 | 176993700 | chr12 | 54385154 | 54385454 |
| chr2 | 177003960 | 177004260 | chr12 | 65217919 | 65218219 |
| chr2 | 177036438 | 177037106 | chr12 | 79258257 | 79258557 |
| chr2 | 177054423 | 177054723 | chr12 | 85306678 | 85306978 |
| chr2 | 198651001 | 198651497 | chr12 | 103218443 | 103218743 |
| chr2 | 208989098 | 208989398 | chr12 | 114878284 | 114878584 |
| chr2 | 229044901 | 229045200 | chr12 | 114882696 | 114882996 |
| chr3 | 2140549 | 2140849 | chr12 | 115102563 | 115102863 |
| chr3 | 42947540 | 42947840 | chr12 | 115103810 | 115104110 |
| chr3 | 119528806 | 119529106 | chr12 | 115109784 | 115110084 |
| chr3 | 137488469 | 137488769 | chr12 | 115134895 | 115135195 |
| chr3 | 138655677 | 138655977 | chr12 | 130388066 | 130388366 |
| chr3 | 142839841 | 142840141 | chr13 | 28498011 | 28498534 |
| chr3 | 147073801 | 147074100 | chr13 | 28498806 | 28499106 |
| chr3 | 147108362 | 147108662 | chr13 | 28501216 | 28501516 |
| chr3 | 147112080 | 147112200 | chr13 | 28503223 | 28503523 |
| chr3 | 147124023 | 147124567 | chr13 | 28674301 | 28674601 |
| chr3 | 147126068 | 147126278 | chr13 | 37005339 | 37005720 |
| chr3 | 147127429 | 147127729 | chr13 | 37005913 | 37006490 |
| chr3 | 147128306 | 147128606 | chr13 | 46425716 | 46426024 |
| chr3 | 147140764 | 147141064 | chr13 | 58204074 | 58204374 |
| chr3 | 157813172 | 157813500 | chr13 | 78493155 | 78493455 |
| chr3 | 157821112 | 157821613 | chr13 | 79176422 | 79176722 |
| chr3 | 157821656 | 157821956 | chr13 | 84454161 | 84454461 |
| chr3 | 181441414 | 181441714 | chr13 | 95355001 | 95355300 |
| chr3 | 184301089 | 184301618 | chr13 | 100607973 | 100608273 |
| chr4 | 1399085 | 1399385 | chr13 | 100624789 | 100625089 |
| chr4 | 3371726 | 3372026 | chr13 | 100625702 | 100626002 |
| chr4 | 4859071 | 4859281 | chr13 | 100640227 | 100640527 |

(continued)

| Chromosome | Start point | End point | Chromosome | Start point | End point |
|---|---|---|---|---|---|
| chr4 | 5710253 | 5710553 | chr13 | 102068846 | 102069146 |
| chr4 | 13525572 | 13525872 | chr13 | 112720071 | 112720382 |
| chr4 | 13530901 | 13531200 | chr14 | 29229689 | 29229989 |
| chr4 | 81185078 | 81185378 | chr14 | 29236263 | 29236473 |
| chr4 | 111533196 | 111533650 | chr14 | 36982295 | 36982595 |
| chr4 | 111535378 | 111535678 | chr14 | 37128275 | 37128575 |
| chr4 | 111539311 | 111539611 | chr14 | 37129201 | 37129500 |
| chr4 | 111554497 | 111554797 | chr14 | 38067055 | 38067445 |
| chr4 | 111560778 | 111561078 | chr14 | 51561143 | 51561443 |
| chr4 | 111561787 | 111562112 | chr14 | 52735271 | 52735571 |
| chr4 | 111562363 | 111562663 | chr14 | 57276918 | 57277218 |
| chr4 | 174449490 | 174449790 | chr14 | 60951901 | 60952500 |
| chr4 | 174452311 | 174452611 | chr14 | 60973367 | 60973667 |
| chr5 | 1883804 | 1884104 | chr14 | 60976201 | 60976500 |
| chr5 | 5139851 | 5140151 | chr14 | 61109705 | 61110106 |
| chr5 | 42424375 | 42424675 | chr15 | 28351948 | 28352248 |
| chr5 | 42995219 | 42995677 | chr15 | 41793731 | 41794031 |
| chr5 | 87955633 | 87955933 | chr15 | 45409169 | 45409469 |
| chr5 | 118609317 | 118609617 | chr15 | 60287437 | 60287737 |
| chr5 | 122430284 | 122430584 | chr15 | 65689002 | 65689302 |
| chr5 | 136834314 | 136834642 | chr15 | 86233064 | 86233370 |
| chr5 | 140793318 | 140793618 | chr15 | 89922301 | 89922600 |
| chr5 | 140864757 | 140865057 | chr15 | 96887311 | 96887710 |
| chr5 | 153853518 | 153853818 | chr15 | 96887769 | 96888069 |
| chr5 | 153862521 | 153862821 | chr15 | 101514062 | 101514362 |
| chr5 | 178003941 | 178004354 | chr16 | 9107049 | 9107349 |
| chr5 | 178367677 | 178367977 | chr16 | 28074234 | 28074526 |
| chr5 | 178421978 | 178422278 | chr16 | 56696938 | 56697238 |
| chr6 | 391593 | 392086 | chr16 | 67196767 | 67197336 |
| chr6 | 393089 | 393389 | chr16 | 77822269 | 77822569 |
| chr6 | 1385345 | 1385645 | chr16 | 86541330 | 86541630 |
| chr6 | 1393425 | 1393725 | chr17 | 1880932 | 1881232 |
| chr6 | 10393848 | 10394148 | chr17 | 12877171 | 12877696 |
| chr6 | 26614501 | 26614800 | chr17 | 36734761 | 36735061 |
| chr6 | 27258856 | 27259156 | chr17 | 43339347 | 43339647 |
| chr6 | 27526069 | 27526369 | chr17 | 46799870 | 46800170 |
| chr6 | 28414801 | 28415100 | chr17 | 46919030 | 46919330 |
| chr6 | 58142486 | 58142786 | chr17 | 59534848 | 59535148 |
| chr6 | 58147218 | 58147750 | chr18 | 500829 | 501129 |

(continued)

| Chromosome | Start point | End point | Chromosome | Start point | End point |
|---|---|---|---|---|---|
| chr6 | 58148830 | 58149133 | chr18 | 4455187 | 4455487 |
| chr6 | 73330964 | 73331264 | chr18 | 5543398 | 5543698 |
| chr6 | 88876591 | 88876891 | chr18 | 11149285 | 11149620 |
| chr6 | 100901902 | 100902202 | chr18 | 14748135 | 14748435 |
| chr6 | 100911594 | 100911894 | chr18 | 19756552 | 19756852 |
| chr6 | 100915705 | 100916005 | chr18 | 32847416 | 32847716 |
| chr6 | 110679416 | 110679716 | chr18 | 55095027 | 55095327 |
| chr6 | 133562342 | 133562642 | chr18 | 74290778 | 74291078 |
| chr6 | 1611,00092 | 161100392 | chr18 | 74962644 | 74962944 |
| chr6 | 166218369 | 166218669 | chr18 | 76739076 | 76739376 |
| chr7 | 15727291 | 15727591 | chr18 | 76739409 | 76739709 |
| chr7 | 19146001 | 19146300 | chr19 | 9473524 | 9473834 |
| chr7 | 19157043 | 19157413 | chr19 | 13124934 | 13125234 |
| chr7 | 24323790 | 24324089 | chr19 | 15580550 | 15580850 |
| chr7 | 24324301 | 24324600 | chr19 | 17439058 | 17439358 |
| chr7 | 24324826 | 24325126 | chr19 | 17439727 | 17440027 |
| chr7 | 27225169 | 27225469 | chr19 | 21646179 | 21646479 |
| chr7 | 27284490 | 27284923 | chr19 | 22018808 | 22019108 |
| chr7 | 35298241 | 35298541 | chr19 | 22444443 | 22444743 |
| chr7 | 42267948 | 42268259 | chr19 | 22817221 | 22817521 |
| chr7 | 49812883 | 49813261 | chr19 | 31841405 | 31841705 |
| chr7 | 84814533 | 84814833 | chr19 | 31842723 | 31843023 |
| chr7 | 96642301 | 96642600 | chr19 | 36909263 | 36909568 |
| chr7 | 96646967 | 96647438 | chr19 | 37096173 | 37096479 |
| chr7 | 96655294 | 96655594 | chr19 | 37957845 | 37958147 |
| chr7 | 114562697 | 114562997 | chr19 | 37958196 | 37958496 |
| chr7 | 121946273 | 121946573 | chr19 | 48983792 | 48984221 |
| chr7 | 127672308 | 127672714 | chr19 | 51111527 | 51111827 |
| chr7 | 127841686 | 127841986 | chr19 | 53038822 | 53039122 |
| chr7 | 142494835 | 142495135 | chr19 | 56879421 | 56879795 |
| chr7 | 149389779 | 149389989 | chr19 | 56904751 | 56905182 |
| chr7 | 149917113 | 149917413 | chr19 | 57018919 | 57019219 |
| chr7 | 151107107 | 151107435 | chr19 | 57049545 | 57049927 |
| chr7 | 158937819 | 158938201 | chr19 | 57050470 | 57050770 |
| chr8 | 495586 | 495967 | chr19 | 57078615 | 57078915 |
| chr8 | 688267 | 688567 | chr19 | 58220101 | 58220700 |
| chr8 | 23562547 | 23562847 | chr19 | 58570269 | 58570569 |
| chr8 | 23562974 | 23563274 | chr20 | 21694558 | 21694858 |
| chr8 | 38964864 | 38965402 | chr20 | 25062104 | 25062597 |

(continued)

| Chromosome | Start point | End point | Chromosome | Start point | End point |
|---|---|---|---|---|---|
| chr8 | 41166957 | 41167257 | chr20 | 34894498 | 34894798 |
| chr8 | 41753910 | 41754210 | chr20 | 37434079 | 37434379 |
| chr8 | 53851557 | 53851857 | chr20 | 41818206 | 41818508 |
| chr8 | 53851880 | 53852811 | chr20 | 61808738 | 61809038 |
| chr8 | 57069863 | 57070163 | chr21 | 34442210 | 34442527 |
| chr8 | 59058506 | 59058806 | chr21 | 38075449 | 38075749 |
| chr8 | 65281346 | 65281646 | chr21 | 38079541 | 38079841 |
| chr8 | 65294493 | 65294793 | chr21 | 45336523 | 45336823 |

3. The DNA methylation marker according to claim 2, wherein the DNA methylation marker combination further comprises two or more DNA methylation markers selected from the group consisting of DNA markers shown in Table 3 below:

[Table 3]

| Chromosome | Start point | End point | Chromosome | Start point | End point |
|---|---|---|---|---|---|
| chr1 | 2425710 | 2426010 | chr8 | 144347047 | 144347257 |
| chr1 | 3139807 | 3140017 | chr9 | 79625119 | 79625419 |
| chr1 | 3210267 | 3210567 | chr9 | 137097130 | 137097430 |
| chr1 | 5661736 | 5662036 | chr9 | 139634101 | 139634400 |
| chr1 | 19599366 | 19599666 | chr10 | 696171 | 696482 |
| chr1 | 21877426 | 21877726 | chr10 | 778559 | 778859 |
| chr1 | 157401687 | 157401987 | chr10 | 3329816 | 3330116 |
| chr1 | 162319425 | 162319725 | chr10 | 31388486 | 31388786 |
| chr1 | 199326770 | 199327070 | chr10 | 44285701 | 44286000 |
| chr1 | 248366249 | 248366549 | chr10 | 77021717 | 77022017 |
| chr2 | 1879801 | 1880100 | chr10 | 95256767 | 95256977 |
| chr2 | 30496175 | 30496475 | chr10 | 130185301 | 130185600 |
| chr2 | 100327219 | 100327519 | chr10 | 134587890 | 134588190 |
| chr2 | 169658731 | 169659031 | chr10 | 134732701 | 134733000 |
| chr2 | 189064407 | 189064707 | chr10 | 134734501 | 134734800 |
| chr2 | 201612794 | 201613094 | chr10 | 134834701 | 134835000 |
| chr2 | 238188557 | 238188857 | chr10 | 135030404 | 135030704 |
| chr2 | 238578341 | 238578641 | chr11 | 655429 | 655729 |
| chr2 | 238972874 | 238973174 | chr11 | 1422301 | 1422600 |
| chr2 | 240169011 | 240169311 | chr11 | 5009161 | 5009461 |
| chr3 | 11178443 | 11178948 | chr11 | 12188846 | 12189146 |
| chr3 | 19987378 | 19987965 | chr11 | 17562975 | 17563275 |
| chr3 | 61623149 | 61623449 | chr11 | 64658576 | 64658876 |
| chr3 | 159386842 | 159387142 | chr11 | 69218483 | 69218783 |
| chr3 | 160409874 | 160410095 | chr11 | 111154676 | 111154976 |
| chr3 | 169374960 | 169375260 | chr12 | 53319067 | 53319367 |

(continued)

| Chromosome | Start point | End point | Chromosome | Start point | End point |
|---|---|---|---|---|---|
| chr3 | 180003041 | 180003341 | chr13 | 113426100 | 113426420 |
| chr3 | 180076871 | 180077171 | chr13 | 113648732 | 113649032 |
| chr3 | 180366205 | 180366505 | chr13 | 114064858 | 114065158 |
| chr3 | 180977784 | 180978084 | chr13 | 114065701 | 114066000 |
| chr3 | 193840031 | 193840331 | chr13 | 114769924 | 114770224 |
| chr3 | 197247601 | 197247901 | chr13 | 114917377 | 114917790 |
| chr4 | 7483501 | 7483800 | chr14 | 58599632 | 58599842 |
| chr4 | 54374099 | 54374399 | chr14 | 65405781 | 65406081 |
| chr5 | 15521632 | 15521932 | chr14 | 104600401 | 104600700 |
| chr6 | 44698584 | 44698884 | chr14 | 105195666 | 105195966 |
| chr6 | 166259862 | 166260162 | chr15 | 22798836 | 22799136 |
| chr7 | 544375 | 544675 | chr15 | 90456774 | 90457074 |
| chr7 | 1303201 | 1303501 | chr16 | 552604 | 552904 |
| chr7 | 3221078 | 3221523 | chr16 | 7640302 | 7640512 |
| chr7 | 3234547 | 3234847 | chr16 | 21245001 | 21245301 |
| chr7 | 23749383 | 23749683 | chr16 | 30456175 | 30456475 |
| chr7 | 27154502 | 27154712 | chr16 | 33572254 | 33572563 |
| chr7 | 38726444 | 38726744 | chr16 | 73266529 | 73266829 |
| chr7 | 40566371 | 40566671 | chr16 | 86653065 | 86653365 |
| chr7 | 41026047 | 41026347 | chr16 | 87823801 | 87824100 |
| chr7 | 55145577 | 55145877 | chr17 | 30488733 | 30489033 |
| chr7 | 73247463 | 73247763 | chr17 | 34328442 | 34328742 |
| chr7 | 73392547 | 73392847 | chr17 | 39059248 | 39059548 |
| chr7 | 97568604 | 97568814 | chr17 | 74911370 | 74911670 |
| chr7 | 99136949 | 99137159 | chr17 | 77174101 | 77174400 |
| chr7 | 140227123 | 140227423 | chr19 | 2278468 | 2278768 |
| chr7 | 157467901 | 157468200 | chr19 | 42408308 | 42408608 |
| chr7 | 157568101 | 157568400 | chr19 | 51128490 | 51128790 |
| chr8 | 2024099 | 2024399 | chr20 | 21588943 | 21589243 |
| chr8 | 11839694 | 11839994 | chr20 | 29960832 | 29961132 |
| chr8 | 13294962 | 13295262 | chr20 | 31591997 | 31592297 |
| chr8 | 23083321 | 23083680 | chr20 | 43029842 | 43030144 |
| chr8 | 37217918 | 37218218 | chr20 | 44876196 | 44876496 |
| chr8 | 53517050 | 53517350 | chr20 | 44881299 | 44881599 |
| chr8 | 64588078 | 64588378 | chr20 | 56134498 | 56134798 |
| chr8 | 105988133 | 105988433 | chr20 | 56272712 | 56273012 |
| chr8 | 110984688 | 110984988 | chr20 | 56273109 | 56273409 |
| chr8 | 117586928 | 117587228 | chr20 | 57616041 | 57616341 |
| chr8 | 118628850 | 118629150 | chr20 | 59047338 | 59047638 |

(continued)

| Chromosome | Start point | End point | Chromosome | Start point | End point |
|---|---|---|---|---|---|
| chr8 | 120686835 | 120687135 | chr20 | 59832774 | 59833074 |
| chr8 | 124729780 | 124730080 | chr20 | 60445848 | 60446058 |
| chr8 | 124730147 | 124730447 | chr20 | 60470101 | 60470400 |
| chr8 | 139344592 | 139344892 | chr20 | 62048701 | 62049000 |
| chr8 | 140630030 | 140630330 | chr20 | 62121768 | 62122068 |
| chr8 | 144346612 | 144346912 | | | |

4. A method of providing information for diagnosing esophageal cancer and gastric cancer, the method comprising:

   (a) isolating DNA from a biological sample;
   (b) detecting a methylation level of the DNA methylation marker combination; and
   (c) determining that esophageal cancer and gastric cancer develop when the detected DNA methylation marker level exceeds a cut-off value.

5. A method of diagnosing esophageal cancer and gastric cancer, the method comprising:

   (a) isolating DNA from a biological sample;
   (b) detecting a methylation level of the DNA methylation marker combination; and
   (c) determining that esophageal cancer and gastric cancer develop when the detected DNA methylation marker level exceeds a cut-off value.

6. The method according to claim 4 or 5, wherein the detection of the methylation level in step (b) is performed using any one method selected from the group consisting of PCR, methylation specific PCR, real time methylation specific PCR, PCR using methylated DNA-specific binding protein, quantitative PCR, PCR using methylation-specific PNA, melting curve analysis, DNA chip, pyrosequencing, bisulfite sequencing, and methylation next-generation base sequence sequencing.

7. The method according to claim 4 or 5, wherein the methylation level of step (b) is expressed as an alpha value.

8. The method according to claim 4 or 5, wherein step (c) comprises:

   (c-i) calculating a methylation score using the following Equation 1; and

   Equation 1:

   Methylation score =

   $$\frac{\textit{Number of nucleic acid fragments with alpha value of hypermethylation marker of 80\% or more } + \textit{Number of nucleic acid fragments with alpha value of hypomethylation marker of 20\% or less}}{\textit{Total number of nucleic acid fragments}}$$

   (c-ii) determining that esophageal cancer and stomach cancer develop when the calculated methylation score exceeds a cut-off value.

9. A primer composition for diagnosing esophageal cancer and gastric cancer comprising a primer combination capable of amplifying each of the DNA methylation markers of the DNA methylation marker combination according to any one of claims 1 to 3.

10. A probe composition for diagnosing esophageal cancer and gastric cancer comprising a probe combination specifically hybridizing with a polynucleotide comprising 10 or more consecutive bases containing the methylated bases of the DNA methylation marker of the DNA methylation marker combination according to any one of claims 1 to 3 or a complementary polynucleotide thereof.

**11.** A kit for diagnosing esophageal cancer and gastric cancer comprising the composition according to claim 9 or 10.

FIG. 1

```
┌─────────────────┐  ┌─────────────────┐  ┌─────────────────┐  ┌─────────────────┐
│ TCGA methylation│  │   cfMeDIP-Seq   │  │  Whole genome   │  │Illumina methylation│
│   450k array    │  │                 │  │ bisulfite-Seq   │  │   450k array    │
└────────┬────────┘  └────────┬────────┘  └────────┬────────┘  └────────┬────────┘
         │                    │                    │                    │
         ▼                    ▼                    ▼                    ▼
┌─────────────────┐  ┌─────────────────┐  ┌─────────────────┐  ┌─────────────────┐
│Discovery of     │  │Discovery of     │  │Discovery of     │  │Discovery of     │
│esophageal       │  │esophageal       │  │esophageal       │  │esophageal       │
│squamous cell    │  │squamous cell    │  │squamous cell    │  │stomach adenocarcinoma-│
│carcinoma-       │  │carcinoma-       │  │carcinoma-       │  │specific markers │
│specific markers │  │specific markers │  │specific markers │  │                 │
└────────┬────────┘  └────────┬────────┘  └────────┬────────┘  └────────┬────────┘
         └──────────┬─────────┘                    │                    │
                    ▼                               ▼                    ▼
         ┌─────────────────┐           ┌─────────────────┐  ┌─────────────────┐
         │Selection of     │           │Selection of ESCC-│  │Selection of     │
         │regions found    │           │specific key     │  │esophageal       │
         │identically in   │           │markers          │  │stomach adenocarcinoma-│
         │two datasets     │           │                 │  │specific key     │
         └────────┬────────┘           └────────┬────────┘  │markers          │
                  ▼                              │           └────────┬────────┘
         ┌─────────────────┐                     │                    │
         │Selection of ESCC-│                    └──────────┬─────────┘
         │specific key     │                               │
         │markers          │                               │
         └────────┬────────┘                               │
                  └────────────────┬──────────────────────┘
                                   ▼
                        ┌─────────────────┐
                        │Selection of     │
                        │regions including│
                        │3 or more CpG    │
                        │sites            │
                        └────────┬────────┘
                                 ▼
                        ┌─────────────────┐
                        │Determination of │
                        │performance of   │
                        │selected regions │
                        │in cfWGEM-seq    │
                        └─────────────────┘
```

FIG. 2

▢ : Training Set  ▢ : Test Set

FIG. 3

FIG. 4

| Production of esophageal and gastric cancer panels |
| :---: |

| Filter 1.<br>Region filtering using beta value mean, SD, and discovery set coverage of normal samples |
| :---: |

| Filter 2.<br>Selecting hyper 5 CpGs that differ significantly between cancer vs normal |
| :---: |

| Determination of performance in entire region (Filter 1), cancer hypermethylation region (Filter 1 hyper), and 5 CpG regions (Filter 2): |
| :---: |

FIG. 5

FIG. 6

Cancer hyper region (discovery set)

Cancer hyper region (external set)

FIG. 7

Cancer specific hyper region (discovery set)

Cancer specific hyper region (external set)

## INTERNATIONAL SEARCH REPORT

International application No.

**PCT/KR2023/019487**

### A. CLASSIFICATION OF SUBJECT MATTER

**C12Q 1/6886**(2018.01)i

According to International Patent Classification (IPC) or to both national classification and IPC

### B. FIELDS SEARCHED

Minimum documentation searched (classification system followed by classification symbols)

C12Q 1/6886(2018.01); C12N 15/11(2006.01); C12Q 1/68(2006.01); G01N 33/50(2006.01); G06F 19/20(2011.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: DNA 메틸화(DNA methylation), 마커(marker), chr2, chr3, chr14, 식도암 (esophageal cancer), 위암(stomach cancer), 진단(diagnosis), 프라이머(primer), 프로브(probe)

### C. DOCUMENTS CONSIDERED TO BE RELEVANT

| Category* | Citation of document, with indication, where appropriate, of the relevant passages | Relevant to claim No. |
|---|---|---|
| A | JP 2019-504642 A (EPIGENOMICS AG) 21 February 2019 (2019-02-21) <br> See abstract, claims 1, 3 and 6, and paragraphs [0006] and [0044]-[0045]. | 1-4,6-10 |
| A | JP 2021-532823 A (SHANGHAI PUBLIC HEALTH CLINICAL CENTER) 02 December 2021 (2021-12-02) <br> See entire document. | 1-4,6-10 |
| A | KR 10-2009-0120934 A (GENOMICTREE, INC.) 25 November 2009 (2009-11-25) <br> See entire document. | 1-4,6-10 |
| A | KR 10-2022-0020191 A (SEOUL NATIONAL UNIVERSITY HOSPITAL) 18 February 2022 (2022-02-18) <br> See entire document. | 1-4,6-10 |
| A | WO 2008-063655 A2 (THE JOHNS HOPKINS UNIVERSITY et al.) 29 May 2008 (2008-05-29) <br> See entire document. | 1-4,6-10 |

☐ Further documents are listed in the continuation of Box C.  ☑ See patent family annex.

| | |
|---|---|
| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |

| Date of the actual completion of the international search | Date of mailing of the international search report |
|---|---|
| **07 March 2024** | **08 March 2024** |

| Name and mailing address of the ISA/KR | Authorized officer |
|---|---|
| **Korean Intellectual Property Office** <br> **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/019487** |

| **Box No. II** | **Observations where certain claims were found unsearchable (Continuation of item 2 of first sheet)** |
| --- | --- |

This international search report has not been established in respect of certain claims under Article 17(2)(a) for the following reasons:

1. ☑ Claims Nos.: **5**
   because they relate to subject matter not required to be searched by this Authority, namely:

   Claim 5 pertains to a method for treatment of the human body by surgery or therapy, as well as a diagnostic method (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☑ Claims Nos.: **11**
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/KR2023/019487**

| Patent document cited in search report | | | Publication date (day/month/year) | Patent family member(s) | | | Publication date (day/month/year) |
|---|---|---|---|---|---|---|---|
| JP | 2019-504642 | A | 21 February 2019 | CN | 109312404 | A | 05 February 2019 |
| | | | | EP | 3408407 | A1 | 05 December 2018 |
| | | | | EP | 3408407 | B1 | 06 January 2021 |
| | | | | EP | 3828281 | A1 | 02 June 2021 |
| | | | | JP | 2023-052157 | A | 11 April 2023 |
| | | | | JP | 7261587 | B2 | 20 April 2023 |
| | | | | US | 2019-0032148 | A1 | 31 January 2019 |
| | | | | WO | 2017-129716 | A1 | 03 August 2017 |
| JP | 2021-532823 | A | 02 December 2021 | CN | 108913777 | A | 30 November 2018 |
| | | | | CN | 108913777 | B | 18 October 2022 |
| | | | | EP | 3839070 | A1 | 23 June 2021 |
| | | | | JP | 7391082 | B2 | 04 December 2023 |
| | | | | US | 2021-0340627 | A1 | 04 November 2021 |
| | | | | WO | 2020-034888 | A1 | 20 February 2020 |
| KR | 10-2009-0120934 | A | 25 November 2009 | KR | 10-1009062 | B1 | 17 January 2011 |
| KR | 10-2022-0020191 | A | 18 February 2022 | WO | 2022-035206 | A1 | 17 February 2022 |
| WO | 2008-063655 | A2 | 29 May 2008 | EP | 2102361 | A2 | 23 September 2009 |
| | | | | EP | 2102361 | B1 | 08 July 2015 |
| | | | | EP | 2508623 | A1 | 10 October 2012 |
| | | | | EP | 2508623 | B1 | 12 August 2015 |
| | | | | US | 10329621 | B2 | 25 June 2019 |
| | | | | US | 2010-0278772 | A1 | 04 November 2010 |
| | | | | US | 2015-0031022 | A1 | 29 January 2015 |
| | | | | WO | 2008-063655 | A3 | 27 November 2008 |

Form PCT/ISA/210 (patent family annex) (July 2022)

## REFERENCES CITED IN THE DESCRIPTION

**Patent documents cited in the description**

- KR 1557183
- KR 1191947
- WO 2019195268 A2
- WO 2019199696 A
- WO 2019199696 A1

**Non-patent literature cited in the description**

- **CROSS, S. et al.** *Curr. Opin. Gene Develop.*, 1995, vol. 5, 309
- **METZKER, M.** *Nature Biotechnology Reviews*, 2010, vol. 11, 31-46